(19) Europäisches Patentamt
European Patent Office
Office européen des brevets

(11) Publication number: **0 558 245 A1**

(12) # EUROPEAN PATENT APPLICATION

(21) Application number: **93301264.3**

(22) Date of filing: **22.02.93**

(51) Int. Cl.$^5$: **C07D 311/30,** C07D 407/12, C07D 405/12, C07D 405/14, C07D 311/22, C07D 407/04, C07D 409/04, C07D 335/06, C07D 405/04, C07D 307/79, C07F 9/655, A61K 31/35

(30) Priority: **25.02.92 IT MI920408**
**26.05.92 US 888775**

(43) Date of publication of application: **01.09.93 Bulletin 93/35**

(84) Designated Contracting States: **AT BE CH DE DK ES FR GB GR IE IT LI LU MC NL PT SE**

(71) Applicant: **RECORDATI S.A. CHEMICAL and PHARMACEUTICAL COMPANY**
**Corso S. Gottardo 54**
**CH-6830 Chiasso (CH)**
(84) **BE CH DE DK ES FR GB GR IE LI LU MC NL PT SE AT**

(71) Applicant: **RECORDATI INDUSTRIA CHIMICA E FARMACEUTICA S.p.a.**
**Via M. Civitali, 1**
**I-20148 Milano (IT)**
(84) **IT**

(72) Inventor: **Leonardi, Amedeo**
**Via Poliziano, 16**
**I-20154 Milano (IT)**
Inventor: **Motta, Gianni**
**Via Ungaretti, 8/2**
**I-20030 Barlassina (IT)**
Inventor: **Riva, Carlo**
**Via Walder 10**
**I-21100 Varese (IT)**
Inventor: **Testa, Rodolfo**
**Via Astesani, 64**
**I-20161 Milano (IT)**

(74) Representative: **SERJEANTS**
**25, The Crescent King Street**
**Leicester, LE1 6RX (GB)**

(54) **Heterobicyclic compounds as antagogists of alpha-1 adrenergic and SHT1A receptors.**

(57) There are disclosed compounds of the general formula

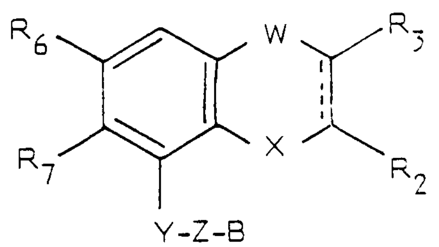

The heteroatom X is preferably oxygen, but may have other values. The group W is preferably a carbonyl group, but may have other values. The preferred heterocyclic ring is thus a 4-oxo-4H-1-benzopyran ring. This may have a wide range of $R_2$, $R_3$, $R_6$ and $R_7$ substituents. Y is a linking group, chosen from a wide range, but including -COO- , -CONH- , -O- , $-SO_2-$ and $-SO_2NH-$. Z is an alkylene chain, and B is a complex amine. These compounds and their prodrugs, enantiomers, diastereoisomers, N-oxides and pharmaceutically acceptable salts are useful for the treatment of hypertension and urinary tract troubles associated with benign prostatic hypertrophy, and for the treatment of other diseases.

EP 0 558 245 A1

## DESCRIPTION

The invention relates to heterobicyclic compounds and to pharmaceutical compositions containing them.

Flavoxate, which is 8-(2-piperidinoethoxycarbonyl)-3-methyl-4-oxo-2-phenyl-4H-1-benzopyran, and has the formula

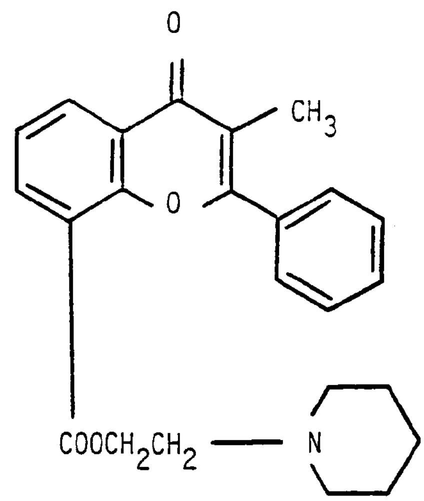

is used as a pharmaceutical agent for urinary tract disturbances as it possesses a smooth muscle relaxing activity attributable to its calcium antagonist activity. This activity is exerted on the bladder dome smooth muscles or can be related to the micturition centre in the central nervous system.

The compounds of the invention, described below, essentially include more complex amino moieties in place of the piperidino group. Further changes include alternatives to the ethoxycarbonyl group which spaces the amino moiety from the benzopyran ring, alternative 2-, 3-, 6- and 7-substitution patterns in the benzopyran ring, replacement of the ring heteroatom by a sulphur atom or by a sulphinyl, sulphonyl or imino group, and/or 2,3-dihydrogenation of the benzopyran ring. These structural variations give the new compounds a capability of interacting with different biological systems, as supported by the affinity of the new compounds for the $\alpha_1$-adrenergic and $5HT_{1A}$-serotoninergic receptors. Flavoxate is practically devoid of affinity for these receptors.

## COMPOUNDS OF THE INVENTION

The compounds of the invention have the general formula I

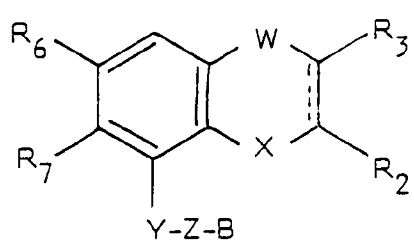

wherein

- ---- represents a single or double bond;
- X represents an oxygen or sulphur atom or an imino, alkylimino, sulphinyl or sulphonyl group;
- W represents a valence bond or a carbonyl, thiocarbonyl, methylene or hydroxymethylene group;
- $R_2$ represents a hydrogen atom or an alkyl, substituted alkyl, alkenyl, substituted alkenyl, alkynyl, substituted alkynyl, carbocyclyl, substituted carbocyclyl, heterocyclyl, substituted heterocyclyl or aroyl group; the substituents for the aforesaid substituted groups being one or more halogen atoms and/or one or more alkyl, cyano, hydroxy, alkoxy, phenyl, phenoxy, trifluoromethyl, nitro, amino, alkylamino, dialkylamino, acylamino, alkylsulphonylamino or benzoyl groups;
- $R_3$ represents a hydrogen atom or an alkyl, hydroxyalkyl, alkoxyalkyl, aralkoxyalkyl, phenyl, hydroxy, alkoxy or aralkoxy group;

$R_6$ represents a hydrogen or halogen atom or a nitro, amino, alkylamino, dialkylamino, acylamino, alkylsulphonylamino, cyano, hydroxy, alkoxy or alkyl group;

$R_7$ represents a hydrogen atom or an alkoxy group;

Y represents one of the following groups, each of which is depicted with its left hand end being the end which attaches to the heterobicyclic ring and its right hand end being the end which attaches to the group Z:

(Y1) -CO-,
(Y2) -COO-,
(Y3) -CONH-,
(Y4) -CON($CH_3$)-,
(Y5) -CON(OH)-,
(Y6) -CH(OH)-,
(Y7) -CH(OAlkyl)-,
(Y8) -CH=CH-,
(Y9) -CH=CH-COO-,
(Y10) -CH=CH-CONH-,
(Y11) -CH=NO-,
(Y12) -$CH_2$-,
(Y13) -$CH_2$COO-,
(Y14) -$CH_2$CONH-,
(Y15) -$CH_2$NH-,
(Y16) -$CH_2$N($CH_3$)-,
(Y17) -$CH_2$N($COCH_3$)-,
(Y18) -$CH_2$N($CONH_2$)-,
(Y19) -$CH_2$NHCO-,
(Y20) -$CH_2$N($CH_3$)CO-,
(Y21) -$CH_2$NH-CONH-,
(Y22) -$CH_2$NH$SO_2$-,
(Y23) -$CH_2$O-,
(Y24) -$CH_2$S-,
(Y25) -$CH_2$SO-,
(Y26) -$CH_2SO_2$-,
(Y27) -$CH_2SO_2$NH-,
(Y28) -$CH_2SO_2$N($CH_3$)-,
(Y29) -NH-,
(Y30) -N($CH_3$)-,
(Y31) -N($COCH_3$)-,
(Y32) -N($CONH_2$)-,
(Y33) -NHCO-,
(Y34) -N($CH_3$)CO-,
(Y35) -NH-CONH-,
(Y36) -NH$SO_2$-,
(Y37) -O-,
(Y38) -S-,
(Y39) -SO-,
(Y40) -$SO_2$-,
(Y41) -$SO_2$NH-,
(Y42) -$SO_2$N($CH_3$)-,
(Y43) -CONHO-,
(Y44) -CON($COCH_3$)-,
(Y45) -CSNH-,
(Y46) -CSN(CH3)-,
(Y47)

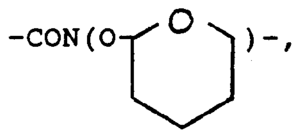

(Y48) -NHCOO-, and
(Y49) -COS-;

Z represents a linear or branched chain alkylene group having from 1 to 6 carbon atoms and optionally having one hydroxy substituent; and

B represents one of the following groups:

(B1)

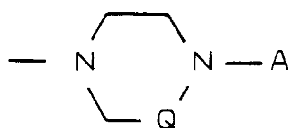

wherein Q represents a methylene or ethylene group and A represents one of the following groups:

(A1) a phenyl group substituted by one or more halogen atoms and/or one or more alkyl, alkoxy or hydroxy groups,
(A2) a 2-pyrimidinyl group, and
(A3) a group of the general formula

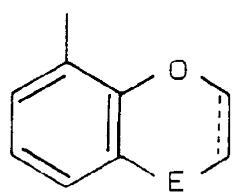

wherein ---- is as above defined and E represents an oxygen atom or a valence bond,

(B2)

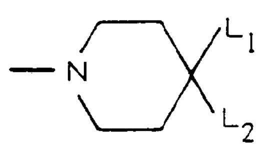

wherein each of $L_1$ and $L_2$ independently represents a hydrogen atom, a phenyl, 4-fluorobenzoyl or 2-oxo-1-benzimidazolinyl group, or a group of the general formula $(CH_2)_n$-O-A wherein n is 0, 1 or 2 and A is as above defined, with the proviso that $L_1$ and $L_2$ do not both represent hydrogen atoms,

(B3)

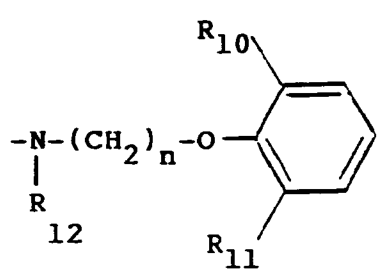

wherein each of $R_{10}$ and $R_{11}$ independently represents a hydrogen atom or an alkoxy or alkylthio group,

$R_{12}$ represents a hydrogen atom or an alkyl group, and n is 2 or 3,

(B4)

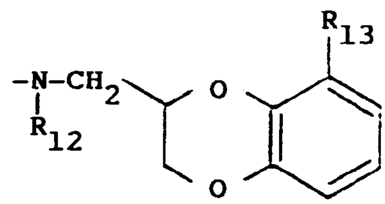

wherein $R_{12}$ is as above defined and $R_{13}$ represents a hydrogen atom or an alkoxy group, and

(B5)

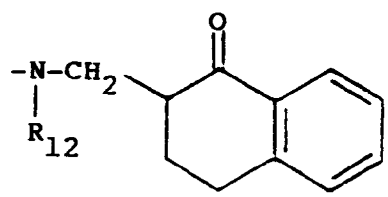

wherein $R_{12}$ is as above defined.

When ---- represents a double bond, W represents a carbonyl group and X represents an imino group, the ring may undergo tautomerization to give a 4-hydroxy-quinoline structure; such tautomeric compounds are included within the invention.

The invention also includes prodrugs, enantiomers, diastereoisomers, N-oxides and pharmaceutically acceptable salts of the compounds of the general formula I.

The term "prodrug" is used herein to mean a derivative of a compound of the general formula I in which reactive groups such as amino, imino or hydroxy groups (particularly as or within the W, $R_2$, $R_3$, $R_6$, Z, B1 and B2 moieties) or acidic imino groups (e.g. those present in Y3, Y10, Y19, Y27, Y36 and Y41) have been masked; the derivative releasing the compound itself within a living body and thus having the same pharmacological effect on the body as the compound itself. Prodrugs may be prepared so as to alter the pharmacokinetic properties of the compound, for instance so as to prepare a delayed release or sustained release form of the compound, or otherwise modify the metabolism, adsorbtion, distribution or plasmatic half-life of the compound. The compounds prepared in Examples 114 and 120 to 122 below are examples of prodrugs.

The group

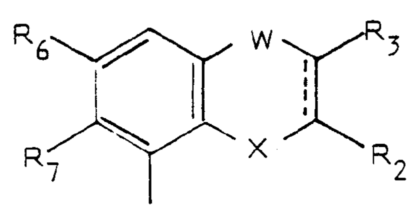

will be abbreviated hereinafter as Fl. The preferred values of the substituents in the group Fl are as follows:

----: a double bond,
X: an oxygen atom,
W: a carbonyl group,
$R_2$: a phenyl group,
$R_3$: a methyl group,
$R_6$: a hydrogen atom, and
$R_7$: a hydrogen atom.

The group having all these preferred substituents is the 3-methyl-4-oxo-2-phenyl-4H-1-benzopyran-8-yl group.

The preferred groups which Z may represent are trimethylene and tetramethylene. Y preferably represents

one of the groups Y2, Y3, Y37, Y40, Y41 or Y42. B preferably represents one of the groups B1 or B3, especially the 1-(2-methoxyphenyl)-piperazinyl group.

Conventional abbreviations used herein include Me for methyl, Et for ethyl, Ac for acetyl, Alk for alkyl, THF for tetrahydrofuran, DMF for dimethylformamide, and DMSO for dimethylsulphoxide.

The adrenergic antagonist activity of the compounds of the invention renders them useful as agents acting on body tissues particularly rich in $\alpha_1$-adrenergic receptors (such as blood vessels, prostate and urethra). Accordingly, anti-adrenergic compounds within the invention established as such on the basis of their receptor binding profile, may be useful therapeutic agents for the treatment, for example, of hypertension and micturition troubles associated with obstructive disorders of the lower urinary tract, including but not limited to those caused by benign prostatic hypertrophy.

The serotoninergic activity of the compounds of the invention renders them useful as agents acting on tissues, particularly in the central nervous system, where $5HT_{1A}$ receptors are functioning. $5HT_{1A}$ receptors are believed to regulate the action and release of serotonin as well as the release of other neuromediators and are found both pre- and post-synaptically. The compounds of the invention have biological activity in blocking binding between these receptors and their various specific ligands (e.g. serotonin). Accordingly, the compounds of the invention which interact with the $5HT_{1A}$ receptor (established as such on the basis of their receptor binding profile) may be useful therapeutic agents for the treatment of anxiety disorders and depression.

Surprisingly, the compounds of the invention (especially those displaying affinity for both the $\alpha_1$-adrenergic and the $5HT_{1A}$-serotoninergic receptors) show high selectivity for the mammalian lower urinary tract, i.e. they are substantially more active in antagonizing urethral contractions than in lowering blood pressure. On the contrary, known $\alpha_1$-antagonists, such as prazosin, which is 1-(4-amino-6,7-dimethoxy-2-quinazonlinyl)-4-(2-furoyl)-piperazine (GB 1156973), do not exhibit such selectivity (and in fact cause hypotension as a most common side effect) while flavone derivatives structurally similar to flavoxate, such as terflavoxate, which is 8-(1,1-dimethyl-2-piperidino-ethoxycarbonyl)-3-methyl-4-oxo-2-phenyl-4H-1-benzopyran hydrochloride (EP 0072620), have no effect on urethral contractions. Naturally, those compounds of the invention that are not selective for the lower urinary tract are preferred as antihypertensive agents, but even the selective compounds can often be used as antihypertensives because of their low toxicity.

Compounds of the invention have also shown a good antagonist effect against contractions of rat bladder strip induced by potassium chloride. This effect can be attributed to a calcium antagonistic activity, and renders the new compounds useful as spasmolytics of the lower urinary tract (i.e. useful in the treatment of urinary incontinence, urge syndrome and other similar disorders).

The majority of the compounds of the invention exhibit low toxicity. Thus they can be used in higher amounts, an advantage that often more than compensates for a relatively lower level of activity that some of these compounds have. Naturally, those compounds exhibiting both high activity and low toxicity are preferred.

The invention further provides a pharmaceutical composition comprising a compound according to the invention, or a prodrug, enantiomer, diastereoisomer, N-oxide or pharmaceutically acceptable salt of such a compound, in admixture with a pharmaceutically acceptable diluent or carrier.

## SYNTHESIS OF THE COMPOUNDS OF THE INVENTION

The compounds according to the invention may generally be prepared (except when the groups $R_6$ and the substituents at $R_2$ are OH, $NH_2$ or aminoalkyl and Y = Y15 or Y29) as follows:

Path a:

By condensing compounds Fl-Y-Z-L, wherein L represents a halogen atom or a leaving group such as a tosyloxy group, with a compound H-B. The condensation is preferably, but not necessarily, carried out at a temperature within the range of 20-140°C in a polar solvent such as dimethylformamide or methanol, usually in the presence of a base such as potassium carbonate. Such condensations are illustrated in Examples 1 to 3, 7 to 9, 11, 13 to 16, 21, 23 to 31, 38 to 42, 46 to 49, 54 to 59, 69, 73, 77, 78 and 84 below. See also Gibson's chapter in Patai, *The Chemistry of the Amino Group*, p.45 et seq., Wiley Interscience, New York, 1968.

An alternative method for the preparation of the compounds of the invention is condensation (under the same conditions described in the preceding paragraph) of a compound Fl-Y-H with a compound L-Z-B wherein L is as above defined. This condensation is illustrated in Examples 5, 6, 66, 79 and 81 below. By this route, compounds having Y = Y15 or Y29 can also be prepared (see Gibson's chapter in Patai, *supra*).

Compounds of formula (I) bearing a $NH_2$ group in $R_6$ or as substituent in $R_2$, may be prepared by reduction of the corresponding compounds (I) wherein $R_6$ or the substituent in $R_2$ are $NO_2$ groups. Such reduction can be carried out:

- with Ni-Raney catalyst in a protic solvent selected from methanol, ethanol, isopropanol, water and mixtures of them; or
- with $SnCl_2$, $H_2O$, optionally in presence of hydrochloric acid, either in a protic solvent such as methanol, ethanol, isopropanol, water, acetic acid and mixtures of them, or in an aprotic solvent such as ethyl acetate; or
- with Fe and aqueous hydrochloric acid in a protic solvent such as methanol, ethanol, isopropanol, water and mixtures of them.

The temperatures of the above reactions will be chosen in a range between 20°C and 100°C. (J. March, *Advanced Organic Chemistry*, III Ed., page 1103, Wiley Interscience, 1985).

Examples of this reduction are given in Examples 94 and 124. Compounds of formula (I) having a NHAlk group as a $R_6$ substituent can be prepared by monoalkylation, starting from the corresponding parent compounds (I) where $R_6$ = $NH_2$. For example, this may be done by first reacting the amino compound (I) with an excess of trifluoroacetic anhydride, then reacting the obtained trifluoroacetyl derivative with an alkyl-L reagent and finally unprotecting the thus-obtained trifluoroacetyl-alkylated derivative by treatment with potassium carbonate in methanol or with sodium borohydride in methanol or dimethylsulphoxide. These reactions are described in Examples 32 and 33, where they were carried out on Y groups.

Alternatively, compounds of formula I having a NHAlk or $N(Alk)_2$ groups as a $R_6$ substituent or as a substituent on the phenyl group in $R_2$ can be obtained by alkylation of the corresponding parent compounds (I) where $R_6$ = $NH_2$ with the appropriate alkanals in presence of a reducing agent, such as sodium cyanoborohydride. Descriptions of these reactions are given in Examples 96 and 97 below.

Compounds bearing a OH group as $R_6$ or as a substituent in $R_2$ may be prepared starting from the corresponding parent compounds (I) alkoxy-substituted at said positions. This can be accomplished by treating the parent compounds for example, with $BBr_3$ in dichloromethane at 0-40°C (T. W. Greene, *Protective Groups in Organic Synthesis*, page 87, Wiley Interscience, 1981) or according to other methods described in the same reference.

Compounds of the general formula I in which ---- represents a single bond can be obtained either by selective hydrogenation of the corresponding compounds in which ---- represents a double bond or by conversion of appropriate starting materials in which the 2,3-bond is already saturated, such starting materials being obtainable according to Reaction Schemes 4, 6 to 9, 11, 12 and 14 below. The latter route is illustrated in Example 87 below, and is particularly preferred when the compound concerned contains a nitro group, as hydrogenation could convert the nitro group to an amino group. The selective hydrogenations can be carried out using alternatively:

- hydrogen in presence of a metal or metal oxide catalyst (e.g. palladium on charcoal, or platinum dioxide) in a protic solvent at 20-120°C (E. H. Rodd, *Chemistry of Carbon Compounds*, Vol. IVB, page 903, Elsevier, 1959); or
- di(isobutyl)aluminium hydride in an aprotic solvent (e.g. tetrahydrofuran and/or dichloromethane) at -70 to 0°C (H. Sarges et al., *J. Med. Chem.*, 33, 1859, 1990).

Compounds in which W represents a hydroxymethylene group and the 2,3-bond is saturated can be obtained by reduction with sodium borohydride of corresponding compounds in which W represents a carbonyl group and the 2,3-bond is saturated, as reported in Example 123 below.

In some cases, the compounds of the general formula I may be prepared by conversion of other (parent) compounds of the invention. Such conversions include:

Path b: Fl-CO-Z-B → Fl-CH(OH)-Z-B
by reduction as illustrated in Examples 17 to 20 below,

Path c: Fl-CH(OH)-Z-B → Fl-CH(OAlkyl)-Z-B
by etherification as illustrated in Example 22 below,

Path d: $Fl\text{-}(CH_2)n\text{-}NH\text{-}Z\text{-}B \rightarrow Fl(CH_2)_n\text{-}N(CH_3)\text{-}Z\text{-}B$
where n = 0 or 1, by N-methylation as illustrated in Example 35 below,

Path e: $Fl\text{-}(CH_2)_n\text{-}NH\text{-}Z\text{-}B \rightarrow Fl\text{-}(CH_2)_n\text{-}N(COCH_3)\text{-}Z\text{-}B$
where n = 0 or 1, by N-acetylation as illustrated in Example 36 below,

Path f: $Fl\text{-}(CH_2)_n\text{-}NH\text{-}Z\text{-}B \rightarrow Fl\text{-}(CH_2)_n\text{-}N(CONH_2)\text{-}Z\text{-}B$
where n = 0 or 1, by reaction with potassium isocyanate as illustrated in Example 50 below,

Path g: Fl-CH(OH)-Z-B → Fl-CO-Z-B
by oxidation, as illustrated in Example 51 below,

Path h: Fl-Y-Z-B → Fl-Y-Z-B(N-oxide)
by oxidation as illustrated in Examples 43 and 122 below,

Path i: $H_2N\text{-}Fl\text{-}Y\text{-}Z\text{-}B \rightarrow CH_3CONH\text{-}Fl\text{-}Y\text{-}Z\text{-}B$
(wherein $H_2N$-Fl represents a Fl group in which $R_6$ is an amino group or $R_2$ includes an amino

group) using the N-acylation method described in Examples 36 and 95 below.

Path j: $Fl(R_6 = NH_2)\text{-}Y\text{-}Z\text{-}B \rightarrow Fl(R_6 = CH_3SO_2NH)\text{-}Y\text{-}Z\text{-}B$
by amidification using the method described in Example 112 below.

Path k:

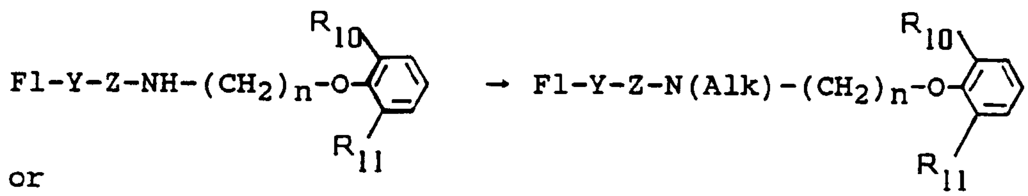

or

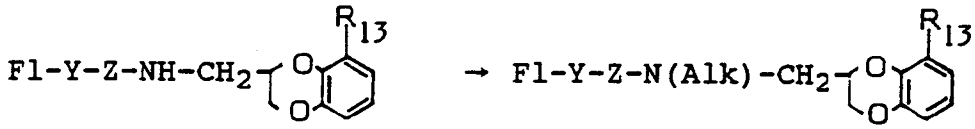

or

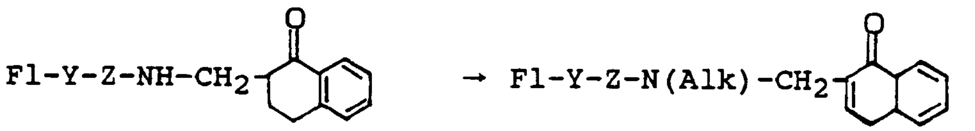

by N-alkylation using the procedure described in Examples 35 and 62 below.

Some compounds may be prepared by addition reactions. For example those in which Z contains a hydroxy substituent may be prepared by addition across an epoxy group.

Path l:

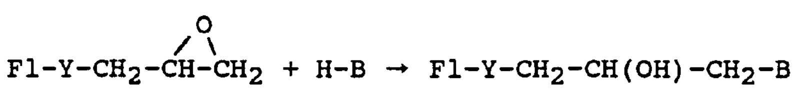

as illustrated in Example 45 hereinbelow.

Addition across a double bond is also possible, e.g.:

Path m: $Fl\text{-}Y\text{-}CH{=}CH_2 + H\text{-}B \rightarrow Fl\text{-}Y\text{-}CH_2\text{-}CH_2\text{-}B$
as illustrated in Examples 37, 63 and 82 below.

Other synthetic schemes include the formation of Y, Z or B during the reaction, for example:

Path n: Fl-(X)-(Q)-Cl + A-HN-Z-B → Fl-(X)-(Q)-N(A)-Z-B
(wherein X = bond, $CH_2$ or CH=CH, Q = CO or $SO_2$, and A = H, alkyl or $OP_r$ wherein $P_r$ is a protective group) as illustrated in Example 12 (particularly preferred) and in Examples 60, 61, 64, 67, 68, 72, 87, 88, 93, 98, 116, 129 and 130.

The same compounds may also be prepared by other routes including:

Fl-(X)-COOH + A-NH-Z-B in presence of a coupling agent (e.g. dicyclohexylcarbodiimide, N,N'-carbonyl-diimidazole or diethyl cyanophosphonate) optionally in the presence of a promoting agent (e.g. 4-dimethylaminopyridine or N-hydroxybenzotriazole) in an aprotic or a chlorinated solvent (e.g. dimethylformamide, chloroform) at -10/140°C (Albertson, *Org. React.*, 12, 205-218, 1962; Doherty et al., *J. Med. Chem.*, 35, 9, 1992; Staab et al., *Newer Methods Prep. Org. Chem.*, 5, 61, 1968; Ishihara, *Chem. Pharm. Bull.*, 39, 3236, 1991); as illustrated in Examples 80, 86, 89, 90, 92, 99 to 111, 113 to 115, 117 to 119 and 128.

Fl-(X)-COOH + A-NH-Z-B without a solvent at 150-220°C (Mitchell et al., *J. Am. Chem. Soc.*, 53, 1879, 1931) or in high-boiling ethereal solvents (e.g. diglyme);

Fl-(X)-COO-Alk + A-NH-Z-B optionally in the presence of a coupling agent (e.g. trimethylaluminium) in an aprotic and/or a chlorinated solvent (e.g. hexane, dichloromethane) at - 10/80°C, or without solvents at 80-180°C (S. M. Weinreb et al., Tetrahedron, 4171, 1977); M. F. Lipton et al., *Org. Synth.* 59, 49, 1979); Fl-(X)-COOH + alkylchloroformate in the presence of a tertiary amine (e.g. triethylamine) followed by addition of A-NH-Z-B at 0-80°C; optionally a promoting agent (e.g. 1-hydroxypiperidine) may be added before the amine addition (Albertson, *Org. React.*, 12, 157, 1962).

Path o: Fl-COCl + HS-Z-B → Fl-Y49-Z-B

Path p: Fl-COCl + HO-Z-B → Fl-Y2-Z-B
as illustrated in Example 10 below.

Path q: FlCHO + $H_2NO$-Z-B → Fl-YII-Z-B
as illustrated in Example 70 below.

Path r: Fl-CHO + A-HN-Z-B → Fl-CS-N(A)-Z-B
(where A = H or $CH_3$) in the presence of sulphur in an aprotic solvent, e.g. DMF or pyridine, at 60-120°C (M. Carmack et al., *Org. Reaction.*, 83, 1947; R. Benassi et al., *Org. Magn. Res.*, 15, 25, 1981), as illustrated in Example 83 below.

Path s: Fl-$NH_2$ + HCO-Z-B → Fl-Y29-Z-B
as illustrated in Example 34 below.

Path t: Fl-Y-$CH_3$ + HO-$CH_2$-B → Fl-Y-$CH_2$-$CH_2$-B
as illustrated in Example 4 below.

Path u: Fl-CH=CH-$CONH_2$ + $HOCH_2$-B → Fl-Y10-$CH_2$-B.

Path v:

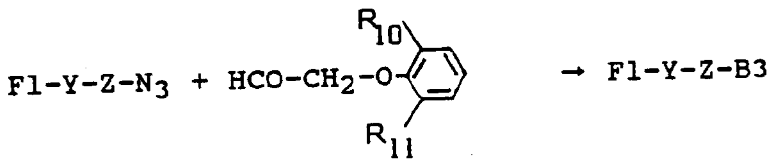

under reducing conditions as illustrated in Example 44 below.

Path w:

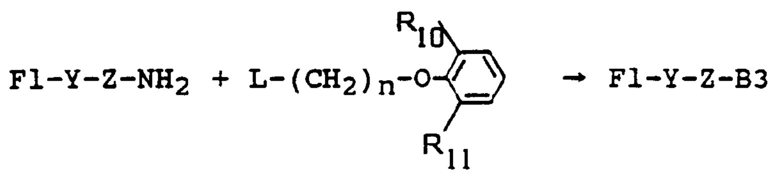

as illustrated in Examples 74, 75 and 76 below.

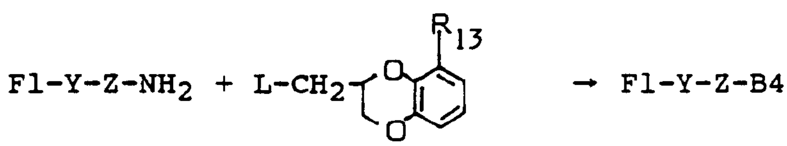

as illustrated in Example 52 below.

Path x:

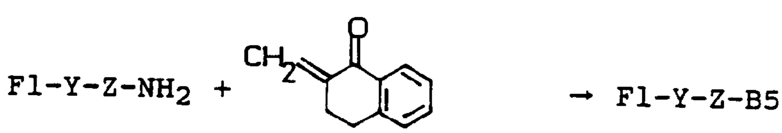

as illustrated in Example 65 below.

Path y: Fl-Y-Z-CHO + HB → Fl-Y-Z-B
as illustrated in Example 53 below.

Persons skilled in the art will be aware that all the above synthetic pathways b) to y) might be simplified provided that the reacting intermediate does not bear further groups sensitive to the same reactants (for example: CO, $NH_2$, NHAlk or OH groups). Compounds of formula I bearing such reactive groups can be prepared through paths b) to y) provided that the reactive groups present in the starting materials are protected beforehand and then deprotected after the reaction as illustrated in Example 71. Several examples of protection and deprotection for various reactive groups can be found in T.W. Green, *Protective Groups in Organic Synthesis*, Wiley Interscience, 1981 (2nd Edition 1991).

Alternatively, unreactive groups (e.g. $NO_2$) can be left unconverted during the first reaction and then converted to reactive ones (e.g. $NH_2$) as a final step of the pathway. See for example path a).

Which synthetic technique will be preferred depends on the compound desired to be synthesized, but path n) is generally preferred for the compounds that can be made by it. Additional synthetic methods will be apparent to those skilled in the art.

## STARTING MATERIALS

The starting materials (Fl-Y-Z-L and Fl-Y-H and others) used in the preparations described above, may themselves be prepared from simple compounds such as Fl-COOH, Fl-CHO, Fl-COCl, Fl-$NH_2$ and Fl-OH by transformations known to those skilled in the art. Numerous such transformations are described in detail hereinbelow. Many of these simple compounds (Fl-COOH, Fl-CHO, Fl-COCl, Fl-$NH_2$ and Fl-OH) are commercially available or their synthesis has been reported in the literature. Those which are not available may be synthesized according to one or more of the following Reaction Schemes 1 to 16.

Reaction Scheme 1 leads to compounds in which W represents a carbonyl group and X represents an oxygen atom.

## SCHEME 1

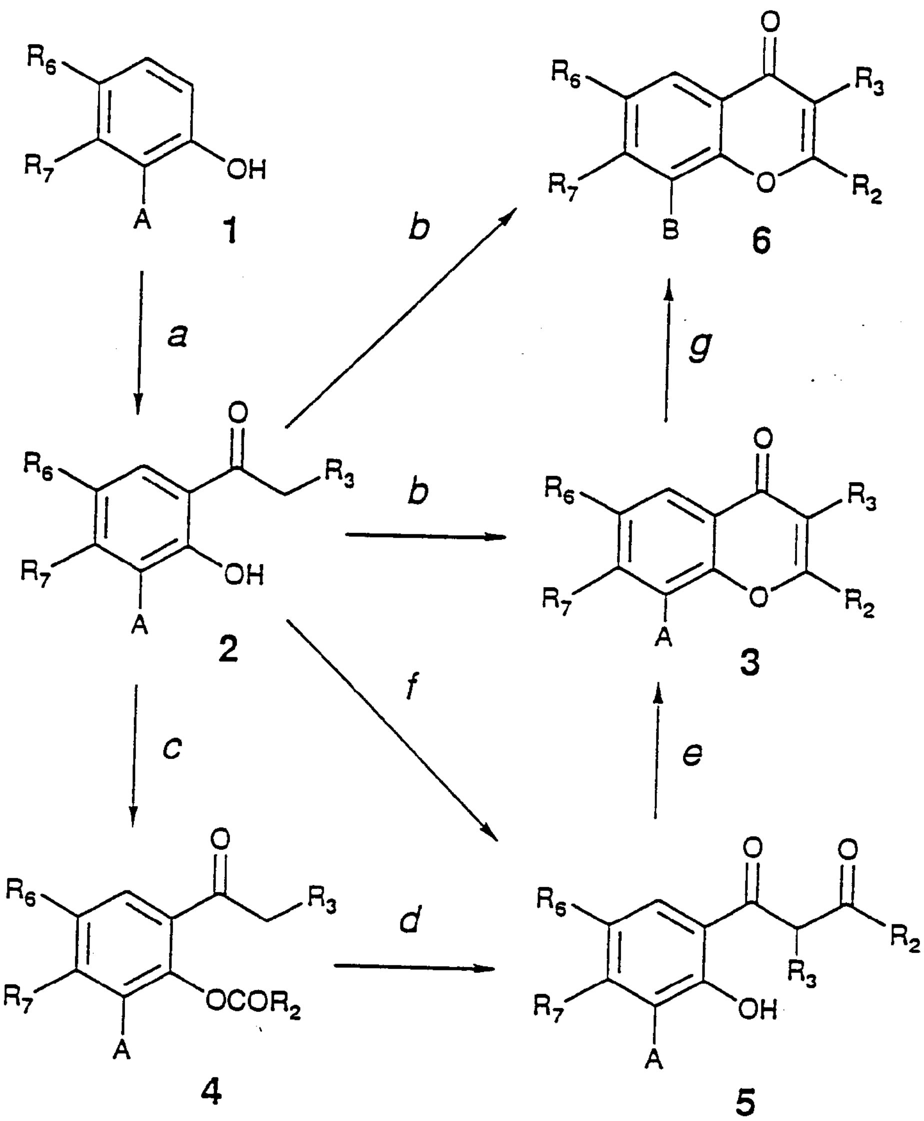


$A = CO_2CH_3$, $CO_2C_2H_5$, $NO_2$, $CH{=}CHCH_3$,
$B = CO_2H$, $NH_2$

Step 1a

Procedure without isolation of the intermediate phenyl ester:

- $R_3CH_2COCl$ or $(R_3CH_2CO)_2O$ and a Lewis acid (e.g. $AlCl_3$ or $ZnCl_2$), without solvent or in an aprotic solvent (e.g. nitrobenzene or chlorinated solvent) at 20-180°C.

Procedure with isolation of the intermediate phenyl ester:

- $R_3CH_2COCl$ or $(R_3CH_2CO)_2O$ heated with the starting material or other esterification methods, such as the Schotten-Bauman procedure. The isolated ester is then heated in nitrobenzene or another non protic solvent (e.g. a chlorinated solvent), or without any solvent, at 20-180°C, in the presence of a Lewis acid such as $AlCl_3$ or $ZnCl_2$ (A.M. Blatt, *Org. React.*, 1, 342, 1942).

Step 1b

- $R_2COCl$ or $(R_2CO)_2O$ and $R_2COONa$ alone or in a high-boiling non-protic solvent (such as o-dichlorobenzene) at 150-220°C; this reaction also allows the direct transformation of compounds (2) to compounds (6), when compounds (2) have A=COOH;
- $R_2C(OAlk)_3$ in the presence of $HClO_4$ at 20-40°C or in pyridine in the presence of piperidine at 60-80°C;
- $R_2COCl$ or $(R_2CO)_2O$ in a chlorinated solvent at -10 to 120°C in the presence of a base such as 1,8-diazabicycloundecene (DBU).

Step 1c

- $R_2COCl$ in pyridine at 20-100°C or in a non-protic solvent at 0-80°C, optionally in the presence of a base such as $NEt_3$ or 4-dimethylaminopyridine.

Step 1d

- $K_2CO_3$ in acetone or methyl ethyl ketone at 20-80°C;
- NaH in DMSO or THF at 0-40°C;
- KOH or potassium t-butoxide in pyridine at 20-100°C.

Step 1e

- HCl or $H_2SO_4$ in AcOH at reflux or in an alcohol (MeOH, EtOH, isopropanol) at 20°C to reflux temperature;
- $CF_3COOH$ in dichloromethane at 20-40°C;
- p-toluenesulphonic acid in benzene or toluene at reflux.

Step 1f

- $R_2COCl$ and $K_2CO_3$ or KOH in water and a phase transfer catalyst in benzene or toluene at reflux;
- $R_2COOAlk$ and lithium bis(trimethylsilyl)amide or lithium diisopropylamide in THF at -78 to 0°C.

Step 1g

When A is a $COOCH_3$ or $COOC_2H_5$ group:
- NaOH in aqueous EtOH at 0-75°C;
- LiOH in aqueous DMF, MeOH or THF or a mixture thereof at 10-100°C;
- HCl in an aprotic solvent such as dioxane at 60-120°C.

When A is $NO_2$:
- Reduction with Ni-Raney catalyst in a protic solvent (e.g. isopropanol) or a mixture of protic solvents at 20-100°C;
- Reduction with hydrogen and a catalyst (e.g. Ni-Raney or Pd/C) in a protic solvent (e.g. MeOH, EtOH, isopropanol or a mixture thereof) at 20-100°C;
- Reduction with $SnCl_2$ in the presence of aqueous HCl in a protic solvent (e.g. AcOH) at 20-100°C;
- Reduction in the presence of Fe and aqueous HCl in a protic solvent at 20-100°C.

When A is a $CH=CHCH_3$ group:
- Oxidation with $Na_2Cr_2O_7$ or other oxidizing agents such as $KMnO_4$ in acetone/$H_2SO_4$ at 0-100°C.

Reaction Scheme 2 leads to compounds in which X represents a sulphur atom or a sulphinyl or sulphonyl group and W represents a carbonyl group. The starting o-mercaptobenzoates (1) are commercially available or can be prepared by known methods, for example by transformation of the corresponding o-alkoxycarbonylbenzenediazonium salts upon treatment with potassium ethylxanthate (M.S. Cohen et al., *J. Org. Chem.*, 18, 1394, 1953).

SCHEME 2

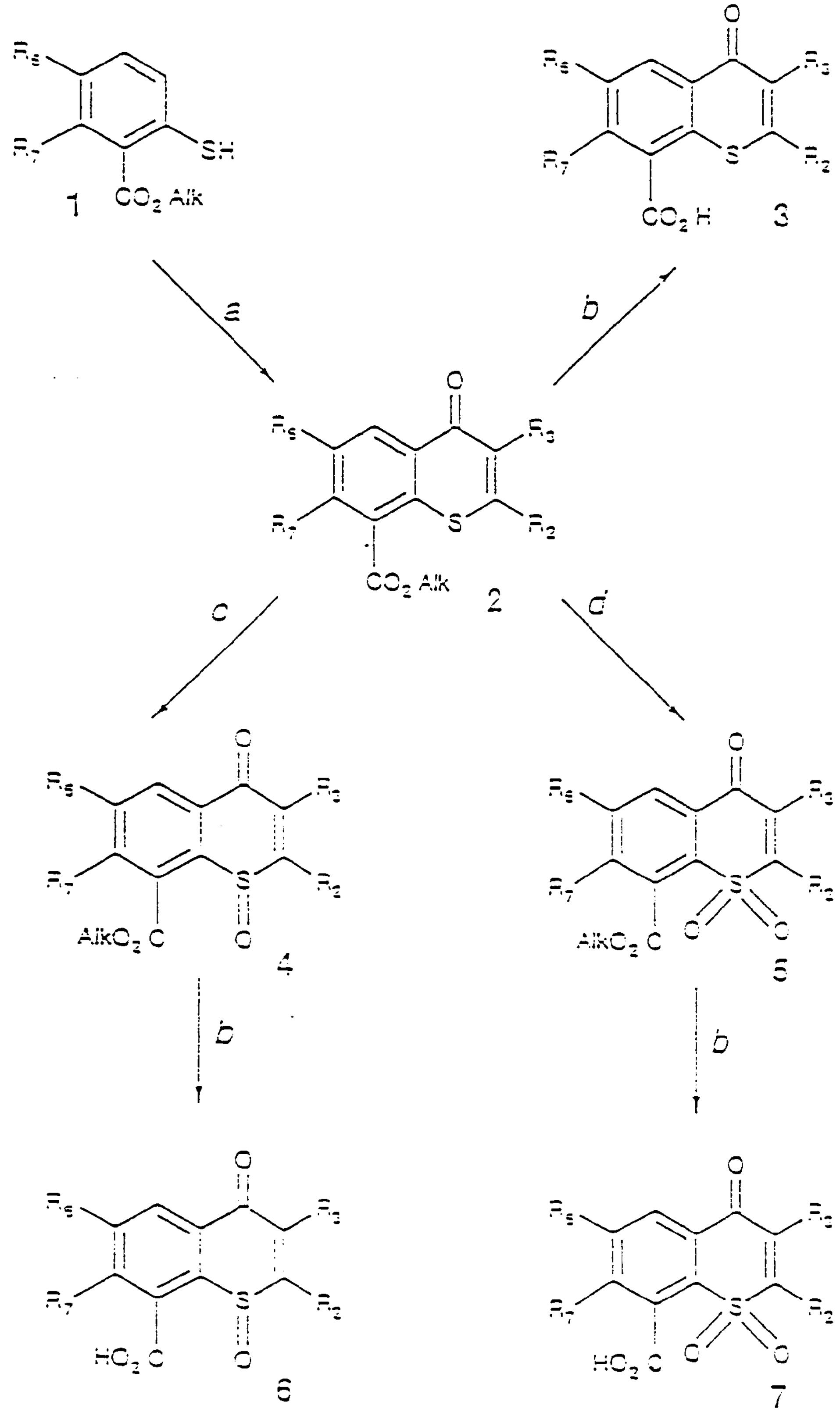


Step 2a

- $R_2COCH(R_3)CN$ or $R_2COCH(R_3)COOAlk$ in polyphosphoric acid at 50-120°C;

- $R_2C{\equiv}C$-COOAlk and $Al_2O_3$ in an aprotic solvent (e.g. $Et_2O$) at 0-40°C;
- $R_2C{\equiv}C$-COOAlk and a base in an aprotic solvent (e.g. THF or DMF) at 20-140°C.

The last two options are both followed by treatment with polyphosphoric acid at 50-120°C.

Step 2b

- NaOH in aqueous EtOH at 40-75°C;
- LiOH in aqueous DMF at 40-100°C.

Step 2c

- Stoichiometric 30% $H_2O_2$ in AcOH at 25-60°C;
- m-chloroperbenzoic acid in chloroform at 0-30°C.

Step 2d

- 30% $H_2O_2$ in AcOH at 50-80°C.

Reaction Scheme 3 leads to compounds (2) in which $R_7$ represents a methoxy group, W represents a carbonyl group and X represents an oxygen or sulphur atom. Compounds (1) can be prepared according to Reaction Schemes 1 and 2 starting from the appropriate phenols or thiophenols (not substituted at position 2 or 6 with COOAlk or $NO_2$).

## SCHEME 3

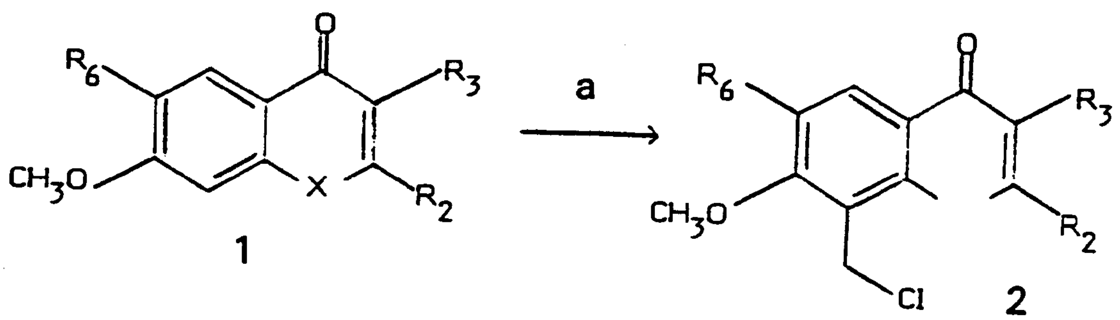


Step 3a

- HCHO and gaseous HCl in AcOH containing aqueous HCl (d=1.18) at 50-100°C (P. Da Re et al., *Ann. Chim.*, 46, 904, 1956). This method can be used when $R_3$ is other than H or $CH_2OH$.

Simple 2,3-dihydro intermediates ( ---- = single bond) can be prepared according to Reaction Scheme 4 provided that other reactive groups possibly present (e.g. $NH_2$, OH) have been previously protected as described before. The Compounds (4) thus obtained can be converted to the corresponding derivatives having A=COOH or $NH_2$ according to the method of Step 1g.

SCHEME 4

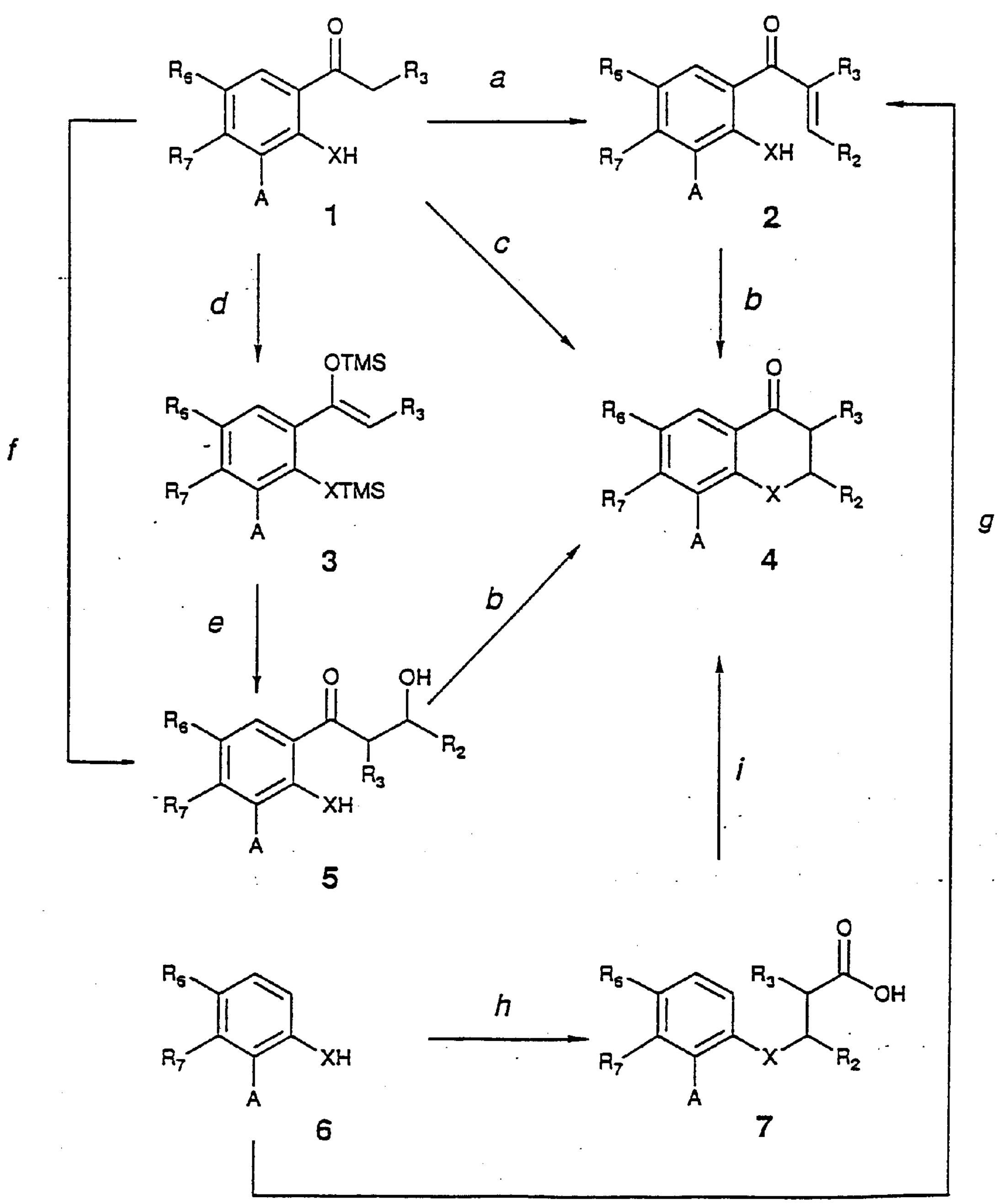


Step 4a

- $R_2$-CHO, aqueous NaOH in EtOH or another protic solvent;
- $R_2$-CHO, NaH or potassium t-butoxide in THF (or another dipolar aprotic solvent) at 0-150°C.

Step 4b

- Mineral acid (e.g. HCl or $H_2SO_4$) in water or another protic solvent (e.g. EtOH, AcOH) at 0-100°C.

Step 4c

- $R_2$-CHO, 0.1N to 1N aqueous NaOH or another suitable base in a protic solvent;
- $R_2$-CHO, pyrrolidine in a protic (e.g. MeOH) or polar aprotic solvent at 0-100°C (H.J.Kabbe, *Synthesis*, 1978, p.886).

Step 4d

- Lithium diisopropylamide in THF at 0-20°C; then trimethylsilyl chloride and an organic base (e.g. $NEt_3$) (S.E. Kelly et al., *J. Org. Chem.*, 56, 1325, 1991).

Step 4e

- $R_2$-CHO in a chlorinated solvent (e.g. dichloromethane) at -78°C, then $TiCl_4$ or other Lewis acid (S.E. Kelly et al., *J. Org. Chem.*, 56, 1325, 1991).

Step 4f

- Lithium diisopropylamide in THF at -78°C, then $R_2$-CHO (A. Banerij et al., *Tetrahedron Letter*, 1979, 3685).

Step 4g

- $R_2$-CH=$CR_3$COCl, a Lewis acid (e.g. $AlCl_3$) in a suitable solvent (e.g. nitrobenzene) or without solvent at 20-180°C.

Step 4h

- $R_2$-CH=$CR_3$COOAlk, triethylbenzylammonium hydroxide in an aprotic solvent (e.g. benzene) or without solvent at 50-150°C; then aqueous NaOH in MeOH at 20-50°C or LiOH in aqueous DMF. (In this case compounds having A=$COOCH_3$ or $COOC_2H_5$ are also hydrolyzed to compounds having A=COOH).

Step 4i

- Concentrated $H_2SO_4$ or $P_2O_5$ or polyphosphoric acid or a Lewis acid in nitrobenzene or toluene or without solvent at 0-180°C. (Also in this case hydrolysis of A=COOAlk to A=COOH occurs).

Simple starting materials having $R_3$=OH or $OR_8$, where $R_8$ is alkyl or aralkyl, may be prepared according to Reaction Scheme 5 where A has the same meaning as in Reaction Scheme 1. Compounds (1) and (2) (which are the same as (2) and (4) in Reaction Scheme 4 but with $R_3$=H) can be prepared according to Reaction Scheme 4 starting from the appropriate phenols or thiophenols having $R_3$=H. The compounds (4) used in Reaction Scheme 5 can be prepared by known methods from the corresponding salycilates or thiosalycilates (see J.March, *Advanced Organic Chemistry*, 486, John Wiley and Sons, New York, 1985; L. Renè et al., *Eur. J. Med. Chem. - Chim. Ter.*, 4, 385, 1977 and references cited therein). The substituent A in compounds (3) and (6) of Reaction Scheme 5 can be transformed by the processes of Step 1g into a substituent B as defined in Reaction Scheme 1.

# SCHEME 5

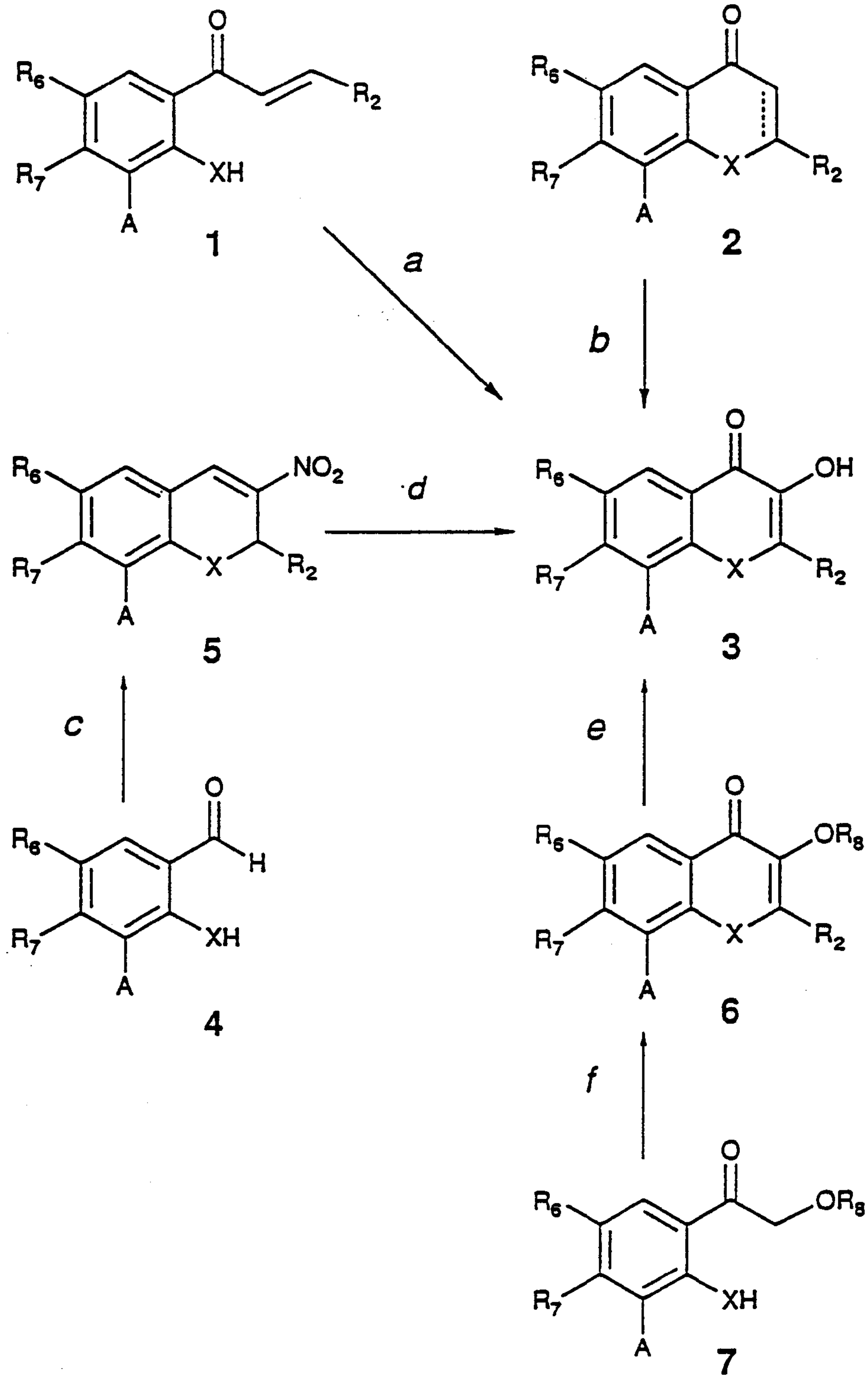


<u>Step 5a</u>

- Aqueous NaOH in an alcoholic solvent (e.g. MeOH or EtOH) followed by 30% $H_2O_2$ at -10 to -78° C. (N.D. Meyer et al., *J. Med. Chem.*, <u>34</u>, 736, 1991 and references cited therein). (Not when A is $CH=CH-CH_3$; when A=COOR it is simultaneously transformed into COOH).

Step 5b

When ---- = single bond:

- Amyl nitrite or other alkyl nitrite without solvent or in a suitable solvent (e.g. EtOH or benzene) in the presence of a catalyst such as 37% HCl (*Org. React.*, 7, 327, 1953 and references cited therein); then aqueous $H_2SO_4$ in a protic solvent (e.g. AcOH) at 10-100°C (Acheson R.M., *An Introduction to the Chemistry of Heterocyclic Compounds*, 347, John Wiley and Sons, New York, 1976).

When ---- = double bond:

- lithium diisopropylamide in dry THF at -78°C; then AcOH and 30% $H_2O_2$ (B.D.M. Cunningham et al., *Anti-Cancer Drug Design*, 7, 365, 1992).

Step 5c

- $R_2$-CH=CH-$NO_2$ (1 to 1.5 equivalent) in a suitable solvent (e.g. diisobutyl ether, DMSO or DMF) in the presence of a base (e.g. KOH or NaOH) in catalytic or stoichiometric quantity at 20-150°C (see L. Renè, *supra*, and T. Sakakibara et al., *Bull. Chem. Soc. Jpn.*, 51, 3095, 1978).

Step 5d

- 15% $H_2O_2$, NaOH or another base (e.g. $NEt_3$) in a protic solvent such as MeOH at 20-100°C (S.R. Deshpande et al., *Synthesis*, 835, 1983) or photolysis and alkaline hydrolysis (Rao T.S. et al., *Heterocycles*, 22, 1377, 1984), or $KO_2$ in benzene containing 18-crown-6 ether at 20-100°C (Rao T.S., *Heterocycles*, 26, 2117, 1987). (Not when A is CH=CH-$CH_3$; when A=COOR it is simultaneously transformed into COOH).

Step 5e

- $R_8$L, where L represents a leaving group (e.g. alkylsulphate, halogen, tosyl) and a base (e.g. $K_2CO_3$, NaH, KOH, NaOH or LiOH) in a suitable solvent (e.g. THF, DMSO, DMF, benzene) in the presence or otherwise of a phase transfer catalyst (e.g. benzyltriethylammonium bromide) at 0-180°C.

Step 5f

- By the methods of Step 1b.

Reaction Scheme 6, in which A has the same meaning as in Reaction Scheme 1, leads to compounds in which W represents a thiocarbonyl group. The compounds (1) and (2) of Reaction Scheme 6 can be prepared according to Reaction Schemes 1, 2, 4 and 5. The substituent A in compound (4) of Reaction Scheme 6 can be transformed by the processes of Step 1g into a substituent B as defined in Reaction Scheme 1.

SCHEME 6

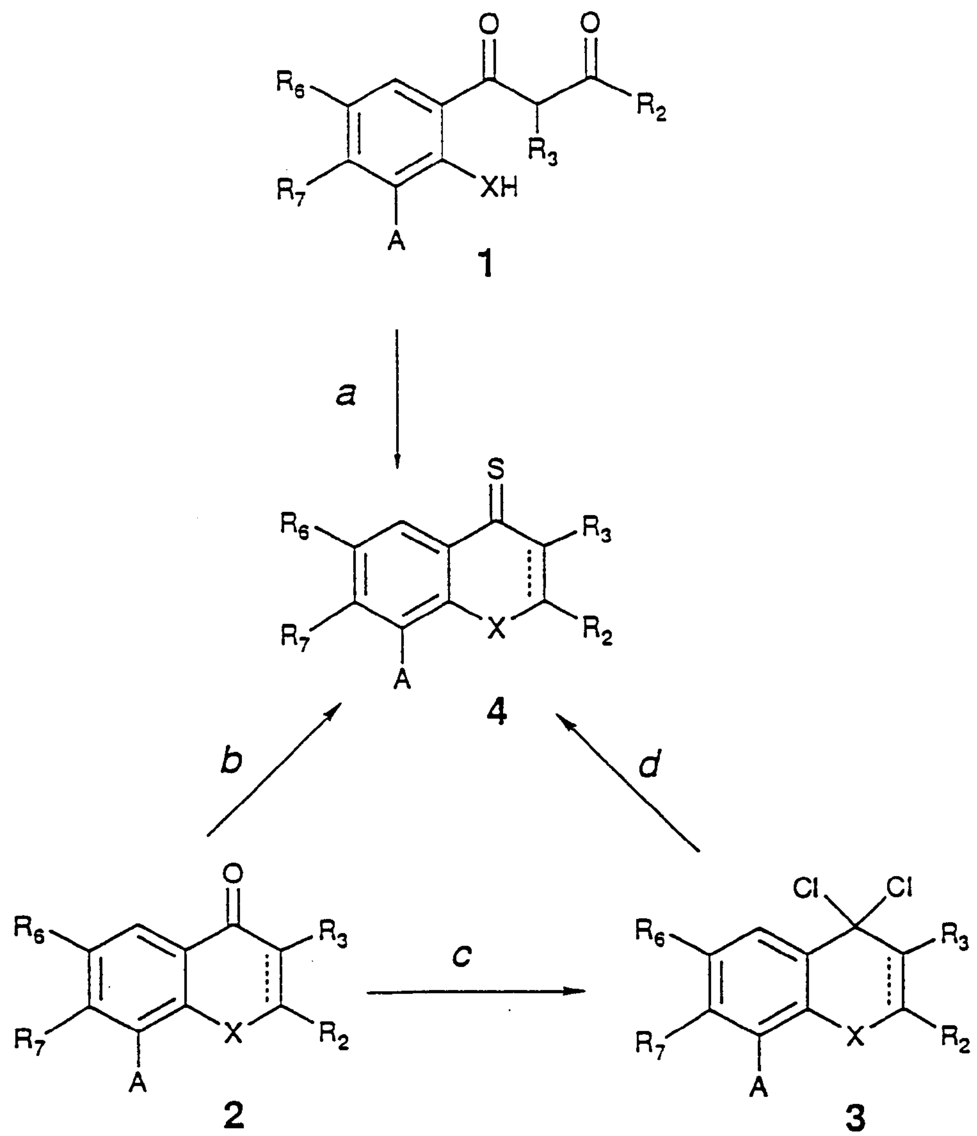


Step 6a

- $P_2S_5$ in pyridine at 50-100°C (Stavaux et al., *Bull. Soc. Chim. Fr.*, 2082, 1967).

Step 6b

- $P_2S_5$ or $B_2S_3$ or $SiS_2$ or Lawesson's reagent in a chlorinated solvent (e.g. chloroform) or in an aromatic solvent (e.g. benzene, toluene, xylene) at reflux (Dean et al., *J. Chem. Soc. C*, 2192, 1963; R.K. Razdan et al., *J.Med. Chem.*, 21, 643, 1978; K. Clausen et al., *Tetrahedron*, 37, 3635, 1991).

Step 6c

- $COCl_2$ without solvent or with an inert solvent (e.g. benzene) at 40-90°C (A. Schonberg et al., *Chem. Ber.*, 101, 701, 1968).

Step 6d

- Thioacetic or thiobenzoic acid or potassium diethylxantogenate in a suitable solvent (e.g. benzene) at reflux (A. Schonberg, *supra*).

Reaction Scheme 7, in which A has the same meaning as in Reaction Scheme 1, leads to compounds in which W represents a methylene or hydroxymethylene group. The compounds (1), (2) and (4) of Reaction Scheme 7 can be prepared according to Reaction Schemes 1, 2, 5 and 6. The substituent A in compounds (7) of Reaction Scheme 7 can be transformed by the processes of Step 1g into a substituent B as defined in Reaction Scheme 1.

SCHEME 7

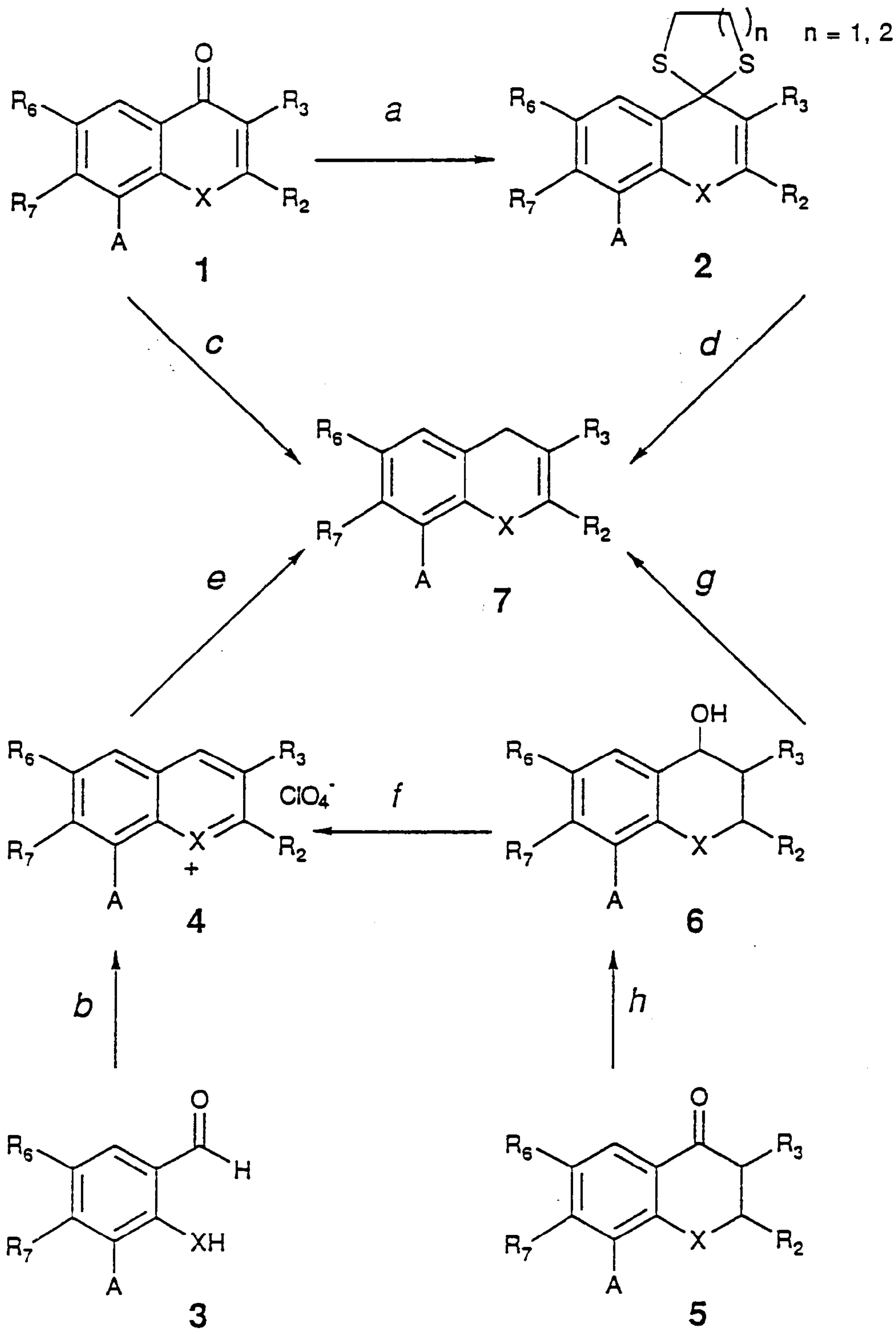


1 2 7 4 6 3 5

Step 7a

- 1,2-ethanedithiol or 1,3-propanedithiol in an aprotic solvent (e.g. dichloromethane or benzene or toluene) at 0-110°C in the presence of a catalyst (e.g. p-toluenesulphonic acid or borontrifluoride etherate).

Step 7b

- $R_2COCH_2R_3$ in a suitable mixture of solvents (e.g. EtOAc or dichloromethane plus EtOH or MeOH) saturated with gaseous HCl at 0-40°C; then aqueous $HClO_4$ in AcOH at 20-100°C (L. Jurd, *Tetrahedron*, 28, 493, 1972).

Step 7c

- $LiAlH_4$ in THF at reflux (if A is other than COOR and $NO_2$);
- $ZnI_2$ and sodium cyanoborohydride (6 equivalents) in a chlorinated solvent (e.g. 1,2-dichloroethane) at room temperature to reflux (C.K. Lau et al., *J. Org. Chem.*, 51, 3083, 1986).

Step 7d

- Raney-Ni in an alcoholic solvent (e.g. isopropanol) at r.t. to reflux (Hilton et al., *J. Am. Chem. Soc.*, 90, 6887, 1968).

Step 7e

- $NaBH_4$ in a suitable solvent (e.g. MeOH or EtOH or DMSO) at -10 to 50°C (L. Jurd, *supra*);
- $LiAlH_4$ in THF (or another suitable solvent) at 0-50°C (when A is other than COOR or $NO_2$) (Degani et al., *Ann. Chim.*, 61, 793, 1971; Kurosawa, *Bull. Chem. Soc. Jpn.*, 51, 1175, 1978).

Step 7f

- Trityl perchlorate in acetonitrile at room temperature (Degani et al., *supra*).

Step 7g

- Melting with $P_2O_5$ at 80-180°C (Hortmann et al., *J. Am. Chem. Soc.*, 96, 6118, 1974).

Step 7h

- $NaBH_4$ in EtOH or another suitable solvent at O°C to reflux (K. Anaya, *Bull. Chem. Soc. Jpn.*, 40, 1884, 1967).
- Hydrogen (1-10 atm) in EtOH (or another suitable solvent) in the presence of a catalyst such as 5% or 10% Pd/C or Raney-Ni or $PtO_2$ at r.t. to 80°C (K. Hanaya, *supra*). Not when A is $CH=CH-CH_3$. When $A=NO_2$ it is simultaneously reduced to $NH_2$.
- Aluminium triisopropoxide in isopropanol at room temperature to 92°C.

Reaction Scheme 8 shows the preparation of simple starting materials such as (4), (5), (6) and (9), where A has the same meanings as in Reaction Scheme 1. Compounds (1), (2), (3), (7), (8) can be prepared according to Reaction Scheme 1, 2, 4, 5, 7, 9, 11. The substituent A in compounds (4), (5), (6) and (9) of Reaction Scheme 8 can be transformed by the processes of Step 1g into a substituent B as defined in Reaction Scheme 1.

SCHEME 8

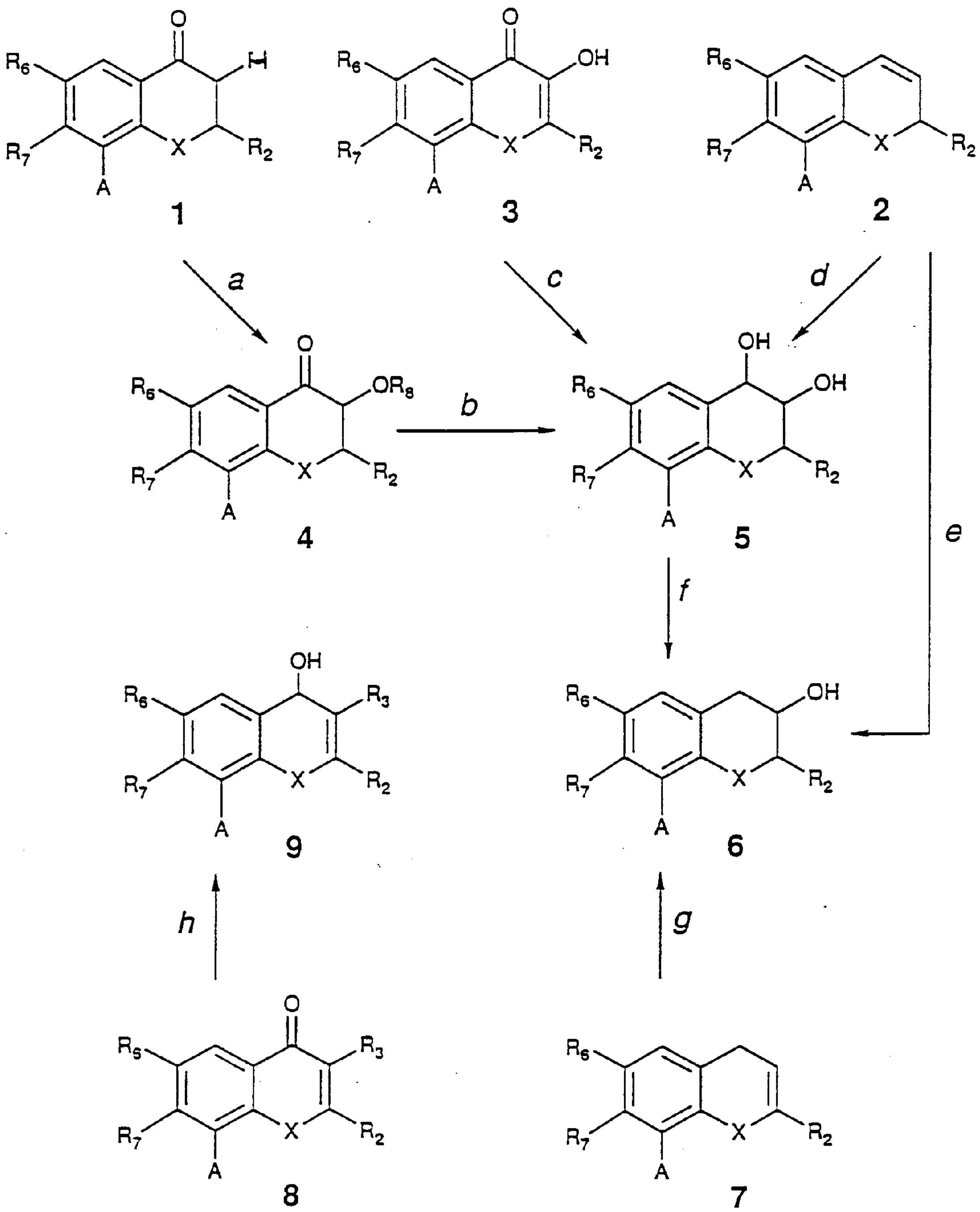


1 3 2 4 5 9 6 8 7

$R_8$ = H, Ac

Step 8a

- $Pb(OAc)_4$ in a suitable solvent (e.g. benzene, toluene) at reflux (G.A. Russel et al., *J. Am. Chem. Soc.*, 1906, 1975).

Step 8b

- $NaBH_4$ in alcohols (see Reaction Scheme 7, step 7a), then alkaline hydrolysis (when A=COOR it can be simultaneously converted into COOH);
- aluminium isopropoxide as described in Reaction Scheme 7, step 7f;
- diborane in THF at -10°C to r.t.; then aqueous $H_2O_2$ in the presence of NaOH (not when A is CH=CH-$CH_3$; when A=COOR it is simultaneously converted into COOH). (Kirkiacharian et al., *C.R. Hebd. Seances Acad. Sci. Ser. C*, 289, 227, 1979);
- $LiAlH_4$ and $AlCl_3$ in a suitable solvent (e.g. THF) at O°C to reflux (not for A=COOR or $NO_2$) (Bokadia et al., *J.Chem. Soc.*, 4663, 1961).

Step 8c

- Hydrogen (100 atm), copper chromite in EtOH at 140°C, see M.A. Vickars, *Tetrahedron*, 20, 2873, 1964. When A=$NO_2$ it is simultaneously converted into a $NH_2$ group.

Step 8d

- $KMnO_4$ in t-butanol (or another suitable solvent) in the presence of aqueous NaOH at -10 to 0°C. (K. Hanaya, *Bull. Chem. Soc. Jpn.*, 40, 1884, 1967). (Not when A is CH=CH-$CH_3$). (See also A.H. Haines, *Methods for the Oxidation of Organic Compounds*, Academic Press Inc, (London), 1985, chapter 3.2.2).
- Osmium tetroxide (see A.H. Haines, *supra*, chapter 3.2.1) in a suitable solvent (e.g. $Et_2O$) at room temperature (Baranton et al., *Bull. Soc. Chim. Fr.*, 4203, 1968) (not when A is CH=CH-$CH_3$);
- aqueous $H_2O_2$ in formic or acetic acid at -20 to -50°C; then NaOH, $H_2O$, 45°C (Baranton et al., *supra*; A.H. Haines, *supra*, chapter 3.2.7.) (not when A is CH=CH-$CH_3$; when A=COOR it is simultaneously converted into COOH);
- silver acetate and iodine in wet AcOH at 0-20°C (K. Hanaya, *supra*; A.H. Haines, *supra*, chapters 3.2.3, 3.2.4, 3.2.9) (Not when A is CH=CH-$CH_3$).

Step 8e

- 30% $H_2O_2$ in the presence of $NaHCO_3$ in benzonitrile at 0-110°C, then $LiAlH_4$ in THF at 0-40°C (not for A-=COOR and CH=CH-$CH_3$) (Clark et al., *Austr. Journ. of Chem.*, 27, 865, 1974).

Step 8f

- Hydrogen (1-50 atm) in a suitable solvent (e.g. EtOH) in the presence of a metallic catalyst (e.g. $PdCl_2$) at r.t. to 78°C. (when A=$NO_2$ it is simultaneously converted into $NH_2$). (Bolger et al., *Tetrahedron*, 23, 341, 1967).

Step 8g

- see step 8b (Clark et al., supra).

Step 8h

- 0.4M cerium trichloride heptahydrate in MeOH, in a suitable solvent (e.g. MeOH); then $NaBH_4$ at 0° to 78°C. (W0 89/06650);
- $NaBH_4$ in diglyme at 0°C to reflux (G.P. Thakar, *Indian J. Chem.*, 3, 74, 1965) (when A=$NO_2$ it is converted into $NH_2$);
- $NaBH_4$ and $AlCl_3$ in a suitable solvent (e.g. THF or benzene) at 0°C to reflux (not with A=COOR) (G.P. Thakar, *supra*);
- diborane in THF at room temperature (not when A is CH=CH-$CH_3$) (G.P. Thakar, *supra*).

Simple starting materials having W=$CH_2$ and a single bond at position 2,3 may be prepared according to

Reaction Scheme 9 where A has the same meanings as in Reaction Scheme 1. Compounds (1) of Reaction Scheme 9 may be prepared according to Reaction Scheme 6. Compounds (1) of Reaction Scheme 9 may alternatively be obtained from compounds (2) by conversion of the latter into a 4-toluenesulphonic acid ester or a methanesulphonic acid ester or into a halogenoderivative, which may be transformed into a thioether derivative (1) by nucleophilic substitution with a thiol. These simple conversions can be performed by techniques known to those skilled in the art. Compounds (2) of Reaction Scheme 9 may be prepared according to Reaction Scheme 7. Compounds (3) of Reaction Scheme 9 where P=OC(S)-aryl or OC(S)-heteroaryl or OC(S)O-alkyl or OC(S)O-aryl or OC(S)S-alkyl may be obtained by reacting compounds (2) with the appropriate chlorothioformate or chlorothiocarbonate or 1,1'-thiocarbonyl-diimidazole as described in *J. Org. Chem.*, 55, 924, 1990 and Synthesis, 362, 1991 and references cited therein. Compounds (4) may be obtained from compounds (1) or (3) by simple elimination reactions with bases. Compounds (5) may be obtained according to Reaction Scheme 4. The substituent A in compounds (6) of Reaction Scheme 9 can be transformed by the processes of Step 1g into a substituent B as defined in Reaction Scheme 1.

SCHEME 9

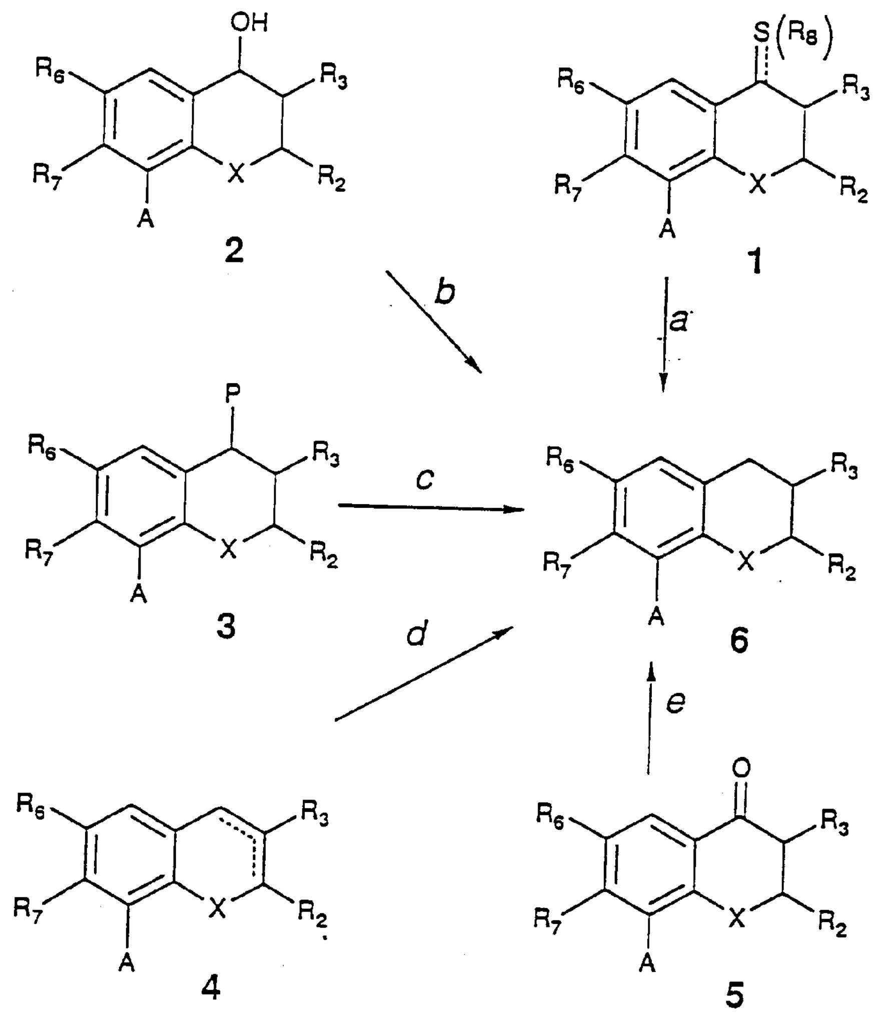


2 1 3 6 4 5

$R_8$ = Alkyl, aryl, heteroaryl, H or nothing

P = halogen, O-tosyl, O-mesyl, OC(S)aryl, OC(S) heteroaryl, 1-imidazolyl, OC(S)O-aryl, OC(S)O-heteroaryl

<u>Step 9a</u>

- Raney-Ni in a suitable solvent (e.g. isopropanol) at r.t. to 100°C. When A is $NO_2$ it is simultaneously converted to $NH_2$;
- triethyltin hydride in benzene or another aromatic solvent at 30-150°C. For other methods like nickel chloride and $NaBH_4$ in MeOH or borane-pyridine complex in trifluoracetic acid or in dichloromethane in the presence of $AlCl_3$, see J. March, *Advanced Organic Chemistry*, page 728, J. Wiley and Sons, New

York, 1992 and references cited therein. (not when A is $CH=CH-CH_3$).

Step 9b

- Hydrogen with a catalyst according to Reaction Scheme 8, step 8f. When A is $NO_2$ it is simultaneously converted to $NH_2$.

Step 9c

Where P is an O-C derivative:

- tributyltin hydride or tris(trimethylsilyl)silane in the presence of azaisobutyronitrile in a suitable solvent (e.g. toluene) at 80-150°C; (M. Drescher, *Synthesis*, 362, 1991; M. Sekine, *J. Org. Chem.*, 55, 924, 1990);
- a silane (e.g. triethylsilane or diphenylsilane) in a suitable solvent (e.g. dichloromethane) at -20°C to reflux in the presence of $CF_3COOH$ or $BF_3$ (F.M. Mauser, *J. Org. Chem.*, 55, 555, 1990);
- triethylchlorosilane, sodium iodide in acetonitrile; then Zn powder in AcOH and acetonitrile at r.t. to 80°C (T.Morita et al., Synthesis, 32, 1981)

Where P is halogen or an 0-S derivative:

- a reducing agent (e.g. sodium cyanoborohydride in hexamethylphosphotriamide or $NaBH_4$ in DMSO) chosen from those cited in J. March, *Advanc. Org. Chem.*, J. Wiley, New York, 1992, chapter 0-76, 0-77.

Step 9d

- Hydrogen (1-5 atm) in a suitable solvent (e.g. EtOH) in the presence of a catalyst (e.g. 10% Pd/C at 50-78°C) (Sarcevic, *Helv. Chim. Acta*, 56, 1457, 1973). (When A=$NO_2$ it is simultaneously converted to $NH_2$);
- Zn and gaseous HCl in $Et_2O$, or $Ac_2O$ in toluene at 0°-80°C. (Todah, *Bull. Chem. Soc. Jpn.*, 45, 264, 1972) (not for A=$NO_2$).

Step 9e

- Zn and aqueous HCl in a suitable solvent (e.g. EtOH) at 0-78°C;
- according to step 9d above (when A=$NO_2$ it is simultaneously converted to $NH_2$);
- hydrazine, NaOH in ethane-1,2-diol at 200°C (*Chemical Abstracts*, 74, (1971): 22699) (not for A=COOR, $NO_2$) or other methods cited in J.March, *supra* (not for A=COOR, $NO_2$);
- according to Step 7c (not for A= $NO_2$).

Reaction Scheme 10 shows routes to compounds in which W represents a valence bond and X represents an oxygen atom or a sulphur atom:

SCHEME 10

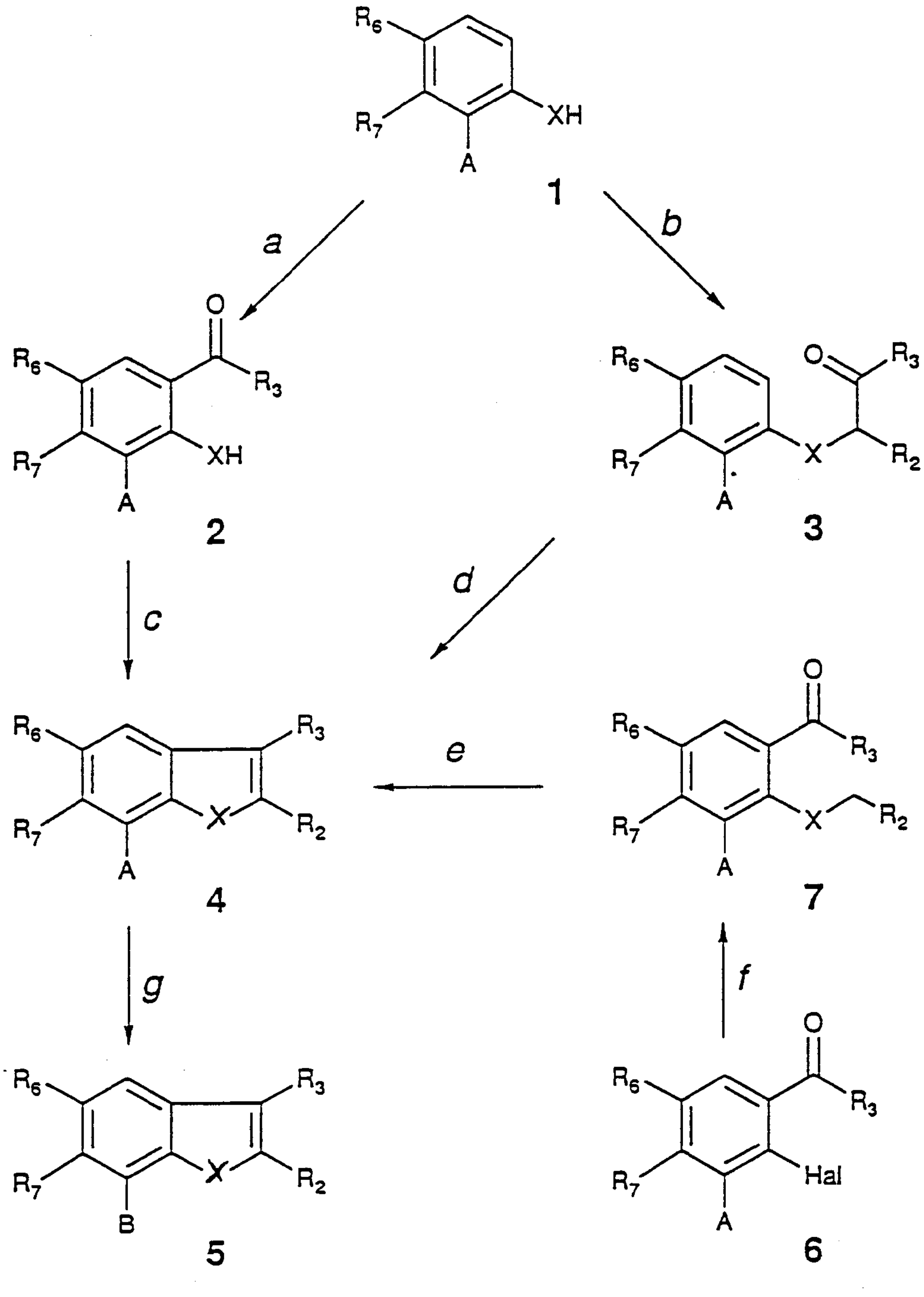


A = COOAlk, $NO_2$, $CH_3$
B = COOH, $NH_2$

Step 10a

- according to Step 1a, but utilizing $R_3COCl$ or $(R_3CO)_20$ instead of $R_3CH_2COCl$ or $(R_3CH_2CO)_20$, with or without isolation of the intermediate phenyl ester;
- hexamethylenetetramine in $CF_3COOH$ at reflux followed by addition of aqueous HCl. If A=COOAlk, it may be hydrolysed to COOH in such strong acid conditions, and thus need re-esterification with the appropriate alcohol (e.g. using thionyl chloride at reflux temperature) before Step 10c.

Step 10b

- $R_3COCH(R_2)Hal$ in acetone or methyl ethyl ketone or dichloromethane or chloroform in the presence of a suitable base such as $K_2CO_3$, $NEt_3$ or NaH, at 20-80°C.

Step 10c

- $R_2CH(Hal)COOAlk$ in an aprotic solvent, e.g. DMF, in the presence of a base, e.g. $K_2CO_3$, at 70-100°C followed by hydrolysis of the crude intermediates with a strong base (e.g. KOH) in a protic solvent such as EtOH at reflux, and finally submitting to decarboxylation - dehydration conditions using a non-protic solvent (e.g. xylene) and an acid catalyst (e.g. p-toluenesulphonic acid) at reflux or simply heating at 240°C in quinoline;
- $R_2CH_2Hal$ and KOH in refluxing EtOH followed by cyclization of the isolated intermediate phenyl(thio) ether with sodium methoxide in a boiling DMF/MeOH mixture. When A is COOAlk, intermediates (4) having A=COOH can be obtained;
- Using $ArCOCH_2Br$ and $K_2CO_3$ in acetone at reflux, Compounds (4), having $R_2$=ArCO, are obtained.

Step 10d

- Vigorous stirring in preheated polyphosphoric acid at 90-140°C;
- Lewis acid (e.g. $AlCl_3$) in chlorobenzene at 70-90°C. The cyclizations carried out on Compounds (3) having $R_3$=Cl with a Lewis acid (e.g. $AlCl_3$) in o-dichlorobenzene at 45°C or with $BF_3$ in $Et_2O$ at 20-25°C gives the Compounds (4) where $R_3$=OH, as reported by K.Davies, *J. Chem. Soc. P.T.*, 1, 2624, 1957, for compounds having X=S and $R_2$=H.

Step 10e

- sodium alcoholate (1 equivalent) in the same alcohol at 0-90°C; when A=COOAlk it may be suitable to use the corresponding AlkOH as reaction solvent;
- When $R_2$=COOAlk and X=S, compounds (4) can be hydrolyzed to the corresponding $R_2$=COOH with sulphuric-acetic acid mixture, (if A=COOAlk is present, it also can give A=COOH), and can be selectively decarboxylated with copper in anhydrous quinoline at 210-220°C, to give Compounds (4) where $R_2$=H according to J.Cooper et al., *J. Chem Soc. (C)* 1971, 3405.

Step 10f

- $R_2CH_2XH$ and one equivalent of sodium in EtOH at reflux or with $NaHCO_3$ in EtOH:water mixture at 60-90°C.

Step 10g

- When A=COOAlk or $NO_2$, the methods described in Step 1g can be used. It must be noted that reduction of the $NO_2$ group to the $NH_2$ group by catalytic hydrogenation can simultaneously afford hydrogenation of the double bond at position 2-3, as reported by S.L. Meisel et al., *Heterocyclic Compounds*, Ed. Interscience Publ.: "Compounds with Condensed Thiophene Rings", page 34, (1954) and M. Ahmed, *ibidem*, Ed. Wiley-Interscience: "Benzofurans", p.56, (1974).

  When A=$NO_2$ and $R_2$=COAr the reduction carried out with hydrogen in the presence of Pt on carbon as catalyst gives the 2,3-dihydro compounds (5) where B=$NH_2$ and $R_2$=$CH_2Ar$ as reported in WO 86/07056;

  when A=$CH_3$ and $R_2$, $R_3$, $R_6$ are not $CH_3$ or $R_2$ does not bear a $CH_3$ group, the compounds can be transformed into the corresponding:

$A=CH_2Br$ by reaction with N-bromosuccinimide in $CCl_4$ and 2,2'-azobisisobutyronitrile or benzoyl peroxide as catalysts at reflux;

A=CHO by reaction of the above Compounds with hexamethylenetetramine in refluxing chloroform followed by acid hydrolysis of the salt in boiling AcOH or by reaction of compounds having $A=CH_3$ with tetrabutylammonium dichromate in refluxing chloroform according to Valenti et al, *Arzneim. Forsch.*, 40, 122 (1990);

A=COOH by oxidation of the above Compounds (A=CHO) with silver oxide in a mixture of protic aqueous solvent (e.g. EtOH-DMF at 0-70°C according to H.R. Rodriguez et al. *Tetrahedron* 24, 6587, 1968) or with $KMnO_4$ in t-butanol in the presence of $NaH_2PO_4$ aqueous solution at 70-75°C according to S. Maruzama et al., *Tetrahedron Letters* 27, 4537 (1986).

Compounds (4) of the above Reaction Scheme 10 having $R_3=C_6H_5$ or t-butyl, $R_2=H$ and X=O can be transformed into the corresponding intermediates having $R_2=C_6H_5$ or t-butyl and $R_3=H$ by reacting with polyphosphoric acid at 132°C according to Davies et al. *J. Chem. Soc.*, 1958, 822.

When X represents an imino or alkylimino group and W is as above defined, other than a valence bond, the simple starting materials may be prepared according to the following Reaction Scheme 11:

## SCHEME 11

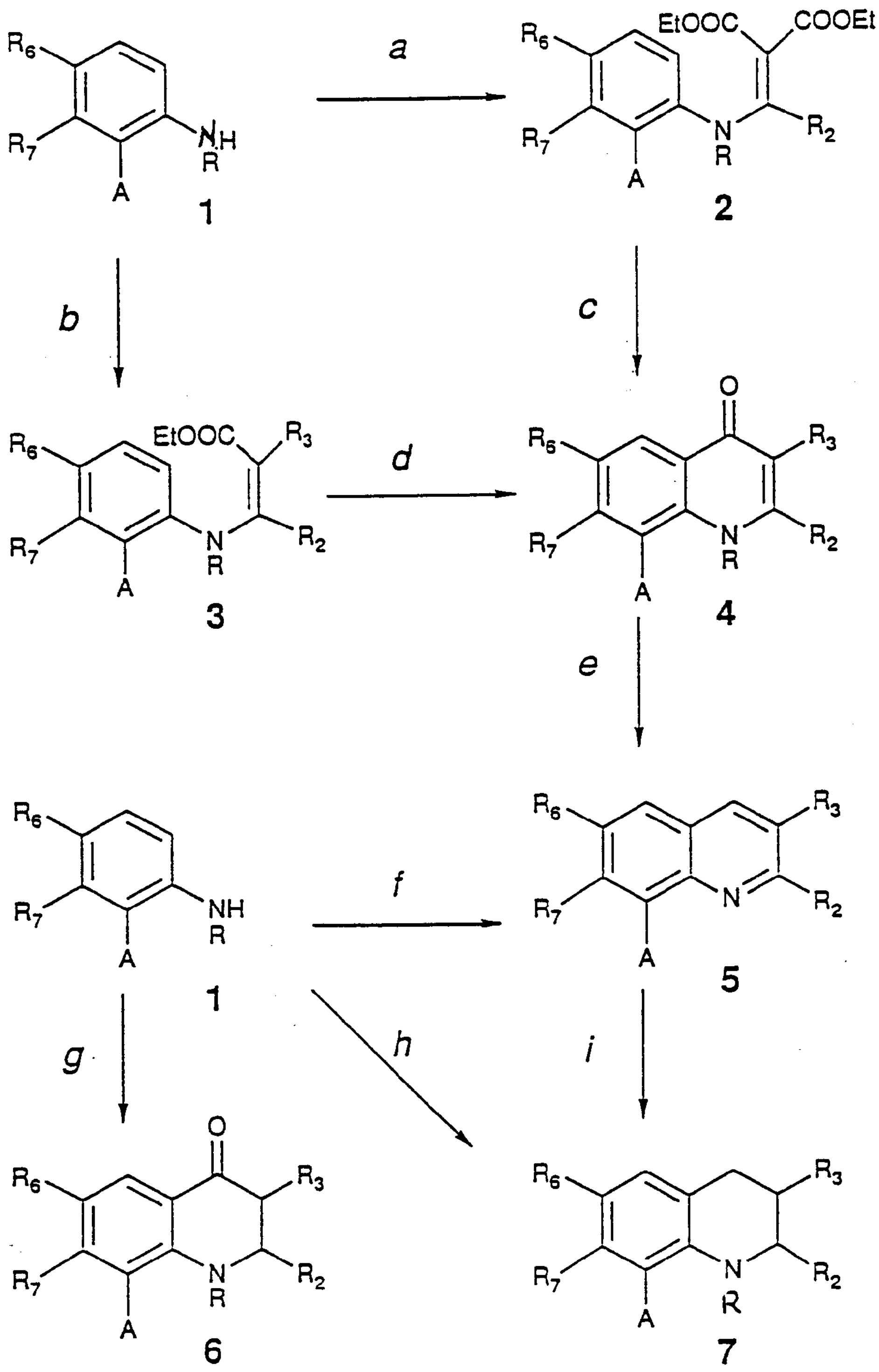


A = COOAlk, $NO_2$

R = H, alkyl

<u>Step 11a</u>

- $EtOC(R_2)=C(COOEt)_2$ at 80-140°C without solvents or in a polar solvent (e.g. isopropanol).

Step 11b

- $R_2COC(R_3)COOAlk$ and p-toluenesulphonic acid or methanesulphonic acid in a chlorinated solvent (e.g. chloroform or dichloromethane) or aprotic solvent (e.g. benzene) at reflux under azeotropic conditions.

Step 11c

- heating in $Ph_2O$ in the presence of p-toluenesulphonic acid or phosphoric acid or ZnO as catalysts at 245-255°C according to Hung. Teljies 6251 (*Chemical Abstracts*, 79, 92026v, 1973);
- heating in a high boiling solvent (e.g. $Ph_2O$) followed by hydrolysis of the not isolated Compounds (4) ($R_3$=COOEt) with a strong acid (e.g. HCl) in a protic solvent (e.g. acetic acid) at reflux to give Compounds (4) where $R_3$=COOH. The above isolated acids can be decarboxylated by heating in a high boiling solvent (e.g. $Ph_2O$) to give Compounds (4) where $R_3$=H according to R. Albrecht et al. *Ber.* 105, 3118 (1972);

Step 11d

- heating in a high-boiling solvent (e.g. $Ph_2O$) at 255°C;
- when R=Alk Compounds (4) are obtained directly from Compounds (1), without isolation of Compounds (3), by condensation with $R_2COCH(R_3)COOAlk$ in polyphosphoric acid at 90-150°C according to F. Piozzi et al., *Gazz. Chim. It.*, 100, 678, 1970.

Step 11e

- Al/Hg amalgam in aqueous EtOH solution at reflux followed by acidification with a strong acid (e.g. HCl) and treatment with $FeCl_3$ at reflux according to W.A. Denny et al., *J. Med. Chem.*, 32, 396, 1989.
- When A=COOAlk Compounds (4) should be hydrolyzed to the corresponding A=COOH before performing Step 11e.
- When A=$NO_2$, intermediates (5) having A=$NH_2$ are obtained;

Step 11f

- $R_2CH$=CHCHO and arsenic acid in a strong acidic medium (e.g. concentrated $H_2SO_4$) and water at 105-115°C according to EP 0206802.
When A=COOAlk, Compounds (1) should be hydrolyzed to the corresponding A=COOH before performing Step f. All Compounds (1) have R=H and the obtained Compounds (5) have $R_3$=H.

Step 11g

- $R_2CH(Hal)$-$CH(R_3)COOH$ in a protic solvent (e.g. water) in the presence of a strong base such as NaOH at 100-125°C followed by cyclization of the isolated Compounds β-anilinopropionic acids with preheated polyphosphoric acid at 120-125°C or with phosphorous pentoxide in a high-boiling aprotic solvent (e.g. xylene) at 120-140°C. In some cases, it is useful to start from Compounds (1), where R=tosyl or other suitable protective groups; the obtained Compounds (6), where R=tosyl, can be easily converted into Compounds (6), where R=H, by hydrolysis with a strong acid (e.g. HCl) in a protic solvent (e.g. AcOH) at reflux.
When A=COOAlk, Compounds (6) having A=COOH, are obtained.

Step 11h

- $R_2CHO$ and ethylene in AcOH and HCl at 25-30°C according to K.D. Hesse, *Liebigs Ann. Chem.* 741, 117 (1970). Where Compounds (1) have R=H starting materials (7), having R=$R_3$=H are obtained.
- Epichlorohydrin followed by cyclization of the isolated anilinopropanol derivatives in refluxing N,N-diethylaniline or o-dichlorobenzene in the presence of a proton acceptor (e.g. $NEt_3$) according to S.D. Boyd et al., *J. Org. Chem.* 30, 2801 (1965). In this case, Compounds (7) having R=$R_2$=H and $R_3$=OH are obtained;

Step 11i

- by hydrogenation in the presence of a catalyst (e.g. platinum oxide) in a protic solvent (e.g. EtOH) at 20-30°C

and 2-4 atm according to G.M. Coppola, *J. Heter. Chem.* 15, 645, 1978.
When A=$NO_2$, Compounds (7) having A=$NH_2$ are obtained.
Compounds (4), (6) and (7) thus obtained can be converted to the corresponding derivatives having A=COOH or $NH_2$ according to the methods of Reaction Scheme 1, step 1g.

The synthesis of the simple Compounds (7) of Reaction Scheme 11 having R=H and A=COOH can be also pursued using the method showed in the Reaction Scheme 12:

SCHEME 12

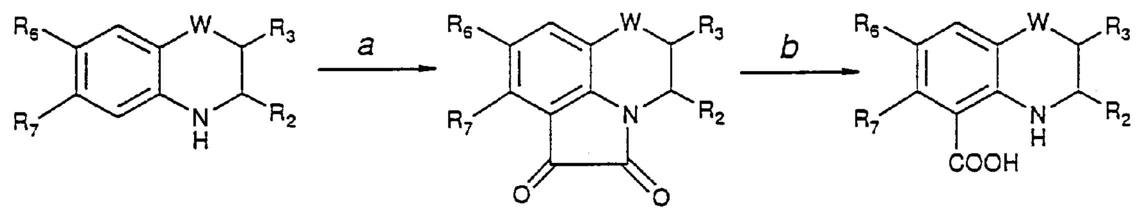


1 W = $CH_2$ or bond 2 3

Step 12a

- oxalyl chloride in a polar solvent (e.g. THF) at reflux followed by internal Friedel-Crafts acylation of the crude chlorooxalylamide with a Lewis acid (e.g. $AlCl_3$) in a non-polar solvent (e.g. $CS_2$) at reflux, according to EP 0402859;

Step 12b

- 30-35% aqueous $H_2O_2$ and a strong base (e.g. NaOH) in a polar solvent (e.g. water) at 20-30°C followed by addition of a strong acid (e.g. HCl), as reported in EP 0402859.

Reaction Schemes 13 and 14 lead to simple starting materials in which X represents an imino group and W represents a valence bond. In these two Reaction Schemes, A has the same meanings as in Reaction Scheme 1.

SCHEME 13

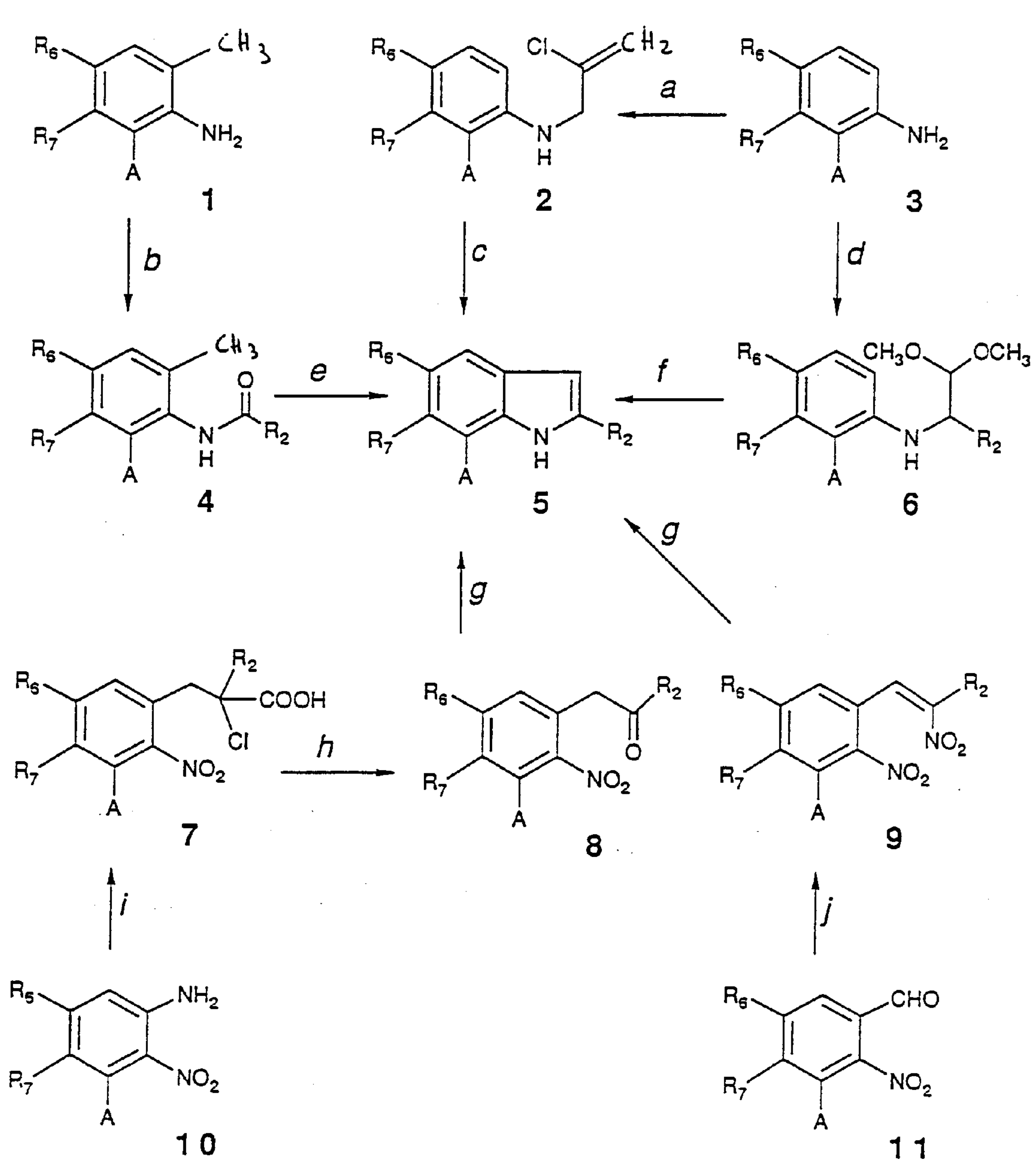


Step 13a

- $ClCH_2C(Cl)=CH_2$ in the presence of $K_2CO_3$ at 40-80°C according to L. Purdie, *J.Chem. Soc. (C)* 1970, 1126.

Step 13b

- $R_2COHal$ in pyridine or in a chlorinated solvent (e.g. dichloromethane) in the presence of proton acceptor (e.g. $NEt_3$) at 20-100°C or in a polar solvent (e.g. acetone) in the presence of $K_2CO_3$ at 20-80°C.

Step 13c

- $BF_3$ in MeOH at 130 to 155°C;
- heating at 100 to 110°C.

The compounds (5) obtained by Step 13c always have $R_2=CH_3$.

Step 13d

- $R_2COCH(OAlk)_2$ in a non polar solvent (e.g. toluene) in the presence of iodine as catalyst at reflux in azeotropic conditions followed by reduction of the isolated (or not isolated) imino Compound with $NaBH_4$ in a polar solvent (e.g. MeOH) in the presence of NaOH as catalyst at reflux. If A=COOAlk, it will be hydrolysed to COOH.

Step 13e

- sodium amide in a high boiling solvent (e.g. N,N-diethylaniline) at 220-250°C according to F. Piozzi et al., *Gazz. Chim. It.*, 93, 1382, 1963;
- potassium t-butoxide in a polar solvent (e.g. DMF) at 20-100°C according to EP 0042298.

Step 13f

- $BF_3$ in an apolar solvent (e.g. benzene) at 5 to 10°C.

Step 13g

- Zn or Fe dust in an acidic medium (e.g. AcOH) and water at 70-100°C. If $A=NO_2$, it will be reduced to $NH_2$.

Step 13h

- thionyl chloride at reflux. The resultant acyl chlorides are isolated, and reacted with sodium azide in an acidic medium (e.g. AcOH) at 10-20°C, subsequently heating at 50-70°C.

Step 13i

- diazotation with $NaNO_2$ in concentrated $H_2SO_4$ followed by aqueous $ZnCl_2$ addition at 5-10°C and by reaction of the isolated diazonium salts with $CH_2=C(R_2)COOH$ in a polar solvent (e.g. acetone) in the presence of a copper salt (e.g. $CuCl_2$) at 25-30°C. Examples of steps 13g, 13h and 13i are given by A. Allais et al., *Eur. J. Med. Chem.*, 10, 187, 1975.

Step 13j

- $R_2CH_2NO_2$ in a polar solvent (e.g. EtOH) in the presence of a base (e.g. n-butylamine) and catalytic amounts of an acid (e.g. AcOH) at reflux.

The Compounds (5) thus obtained can be converted to the corresponding derivatives having A=COOH or $NH_2$ according to the methods of Reaction Scheme 1 step 1g.

With regard to Reaction Scheme 14, it is to be intended that Compounds (4) having $R_3$=H correspond to Compounds (5) of Reaction Scheme 13.

SCHEME 14

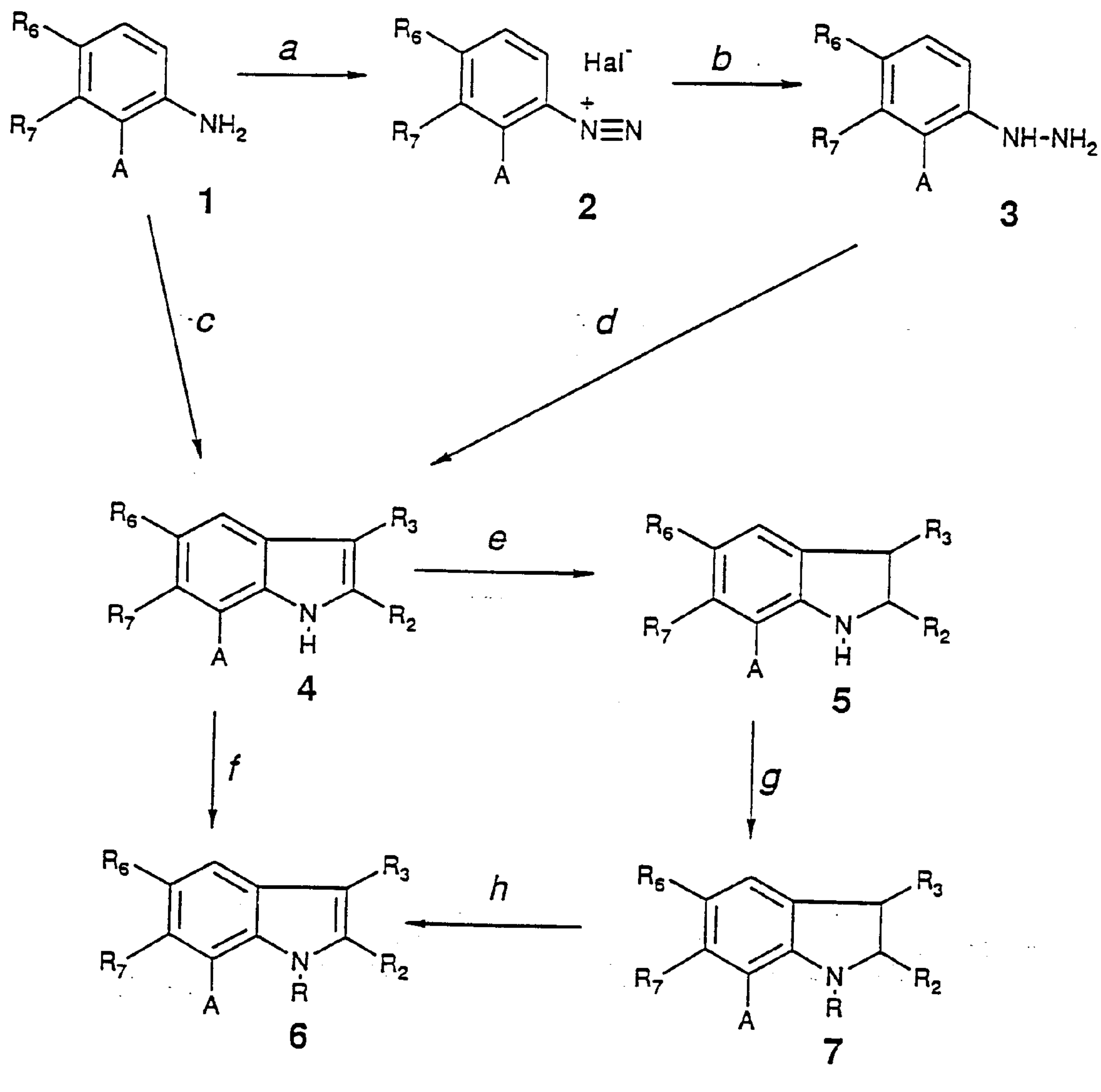


R = Alkyl

Step 14a

- $NaNO_2$ in aqueous acidic medium (e.g. HCl) at -5 to +5°C;
- isoamyl nitrite in a polar solvent (e.g. EtOH) at 5-10°C;

Step 14b

- aqueous solution of $SO_2$ at 0-10°C according to Pfannstiel et al., *Ber.* 75, 1096, 1942;
- triphenylphosphine and heating of the isolated phosphonium salt in an aqueous-alcoholic HCl solution at reflux according to Horner et al., *Ber.* 85, 1073, 1953.

Step 14c

- $R_2COCH(R_3)Hal$ in a basic high boiling solvent (e.g. N,N-diethylaniline) at 160-180°C or by simply heating without solvents at 180°C;
- $R_3COCH(R_2)Hal$ in a polar solvent (e.g. acetone) in the presence of a suitable proton acceptor (e.g. $K_2CO_3$) at reflux followed by cyclization of the isolated β-anilinoketone intermediates with freshly melted $ZnCl_2$ in a protic solvent (e.g. EtOH) at reflux;
- $R_2CH(Hal)CN$ in the presence of $BCl_3$ and a Lewis acid (e.g. $TiCl_4$) in an apolar solvent (e.g. benzene) at reflux followed by cyclization of the isolated 2-amino-α-haloacetophenones Compounds with a suitable reducing agent (e.g. $NaBH_4$) in a polar medium (e.g. dioxane-water) at reflux, according to T. Sagusawa et al., *J. Org. Chem.*, 44, 578, 1979. By the above method, Compounds (4), having $R_3$=H are obtained;
- $R_2COCH(R_3)Hal$ (half an equivalent) in a polar solvent (e.g. MeOH) at reflux followed by cyclization of the isolated Schiff base intermediates with a strong acid (e.g. $CF_3COOH$) at 20-30°C;

Step 14d

- $R_2COCH_2R_3$ by heating at 100°C without solvents or at reflux in a polar solvent (e.g. MeOH) followed by cyclization of the isolated hydrazone Compounds with polyphosphoric acid at 100-130°C or by simply heating in ethyleneglycol or aqueous formic acid or ethanolic formic acid.
- Cyclization can be also carried out by heating in ethanolic HCl at reflux or in an AcOH/HCl mixture at reflux or in orthophosphoric acid at 95-105°C or by simply heating with anhydrous $ZnCl_2$ at 100-220°C. When A=COOAlk, Compounds (4) having A=COOH can be obtained.

Step 14e

- borane-pyridine complex at 0-30°C followed by a protonating agent addition (e.g. HCl);
- tin or zinc and aqueous HCl at 50-100°C;
- $NaBH_4$ in the presence of a Lewis acid (e.g. $AlCl_3$) in pyridine at 0-30°C or alternatively in the presence of a salt like cobalt or zinc chloride;
- sodium borocyanohydride in AcOH at 20-80°C;
- hydrogen in the presence of a catalyst (e.g. Pt) in a polar solvent (e.g. EtOH) at 20-80°C..
  Other general methods are reported by Houlihan in *Heterocyclic Compounds*, part one, Ed. Wiley-Interscience: "Indoles", page 462 (1972). When A=$NO_2$, Compounds (4) can be reduced to the corresponding Compounds (5) having A=$NH_2$;

Step 14f

- NaH and RHal in an anhydrous polar solvent (e.g. DMF) at 20-80°C;
- RHal in the presence of potassium carbonate in a polar solvent (e.g. acetone) at reflux;
- sodium amide and RHal in a polar anhydrous solvent (e.g. THF) at low temperature (-70°C).
  Compounds (4), bearing other reactive groups such as $NH_2$ or OH, have to be protected using suitable protective groups which can be selectively cleaved at the end by deprotecting methods;

Step 14g

- RHal in the presence of alkaline metal carbonates (e.g. potassium carbonate) as reported by Houlihan in *Heterocyclic Compounds*, part two, Ed. Wiley-Interscience: "Indoles", page 90 (1972) and references cited therein.
- Compounds (5), bearing other reactive groups such as $NH_2$ or OH, have to be protected as reported above;

Step 14h

- tetrachloro-[1,4]-benzoquinone in a polar solvent (e.g. ethylene glycol monomethyl ether) at reflux;
- copper (II) chloride in pyridine at reflux according to Kikugawa et al. *J. Heter. Chem.*, 16, 1325, 1979.

Compounds (6) having $R_2$ and $R_3$ other than H, can be reduced to the corresponding starting materials (7) by lithium aluminium hydride according to H.C. Printy et al., *J. Am. Chem. Soc.*, 71, 3206, 1949.

Compounds (4) of Reaction Scheme 14 having $R_2$=H and $R_3$=OH may be obtained from Compounds (7) of Reaction Scheme 11 having R=$R_2$=H and $R_3$=OH by ring contraction using an oxidant (e.g. sodium periodate) and a base (e.g. NaOH) in aqueous EtOH at reflux according to S.D. Boyd et al., *J. Org. Chem.*, 30, 2801, 1965.

Starting materials (4), (5), (6) and (7) can be converted into the corresponding A=COOH or $NH_2$ according to the method of Reaction Scheme 1, step 1g, and from these into the alternative final products. When NH is present and might interfere on the following reactions, it can be protected as reported by T.W. Green in *Protective Groups in Organic Synthesis*, Wiley Interscience, 1981. Alternatively, unreactive groups (e.g. $NO_2$) can be converted to reactive ones (e.g. $NH_2$) as a final step of the pathway.

Starting materials in which W represents a valence bond, X represents an imino group and the 7-substituent is a carboxymethyl group can be obtained according to Reaction Scheme 15.

SCHEME 15

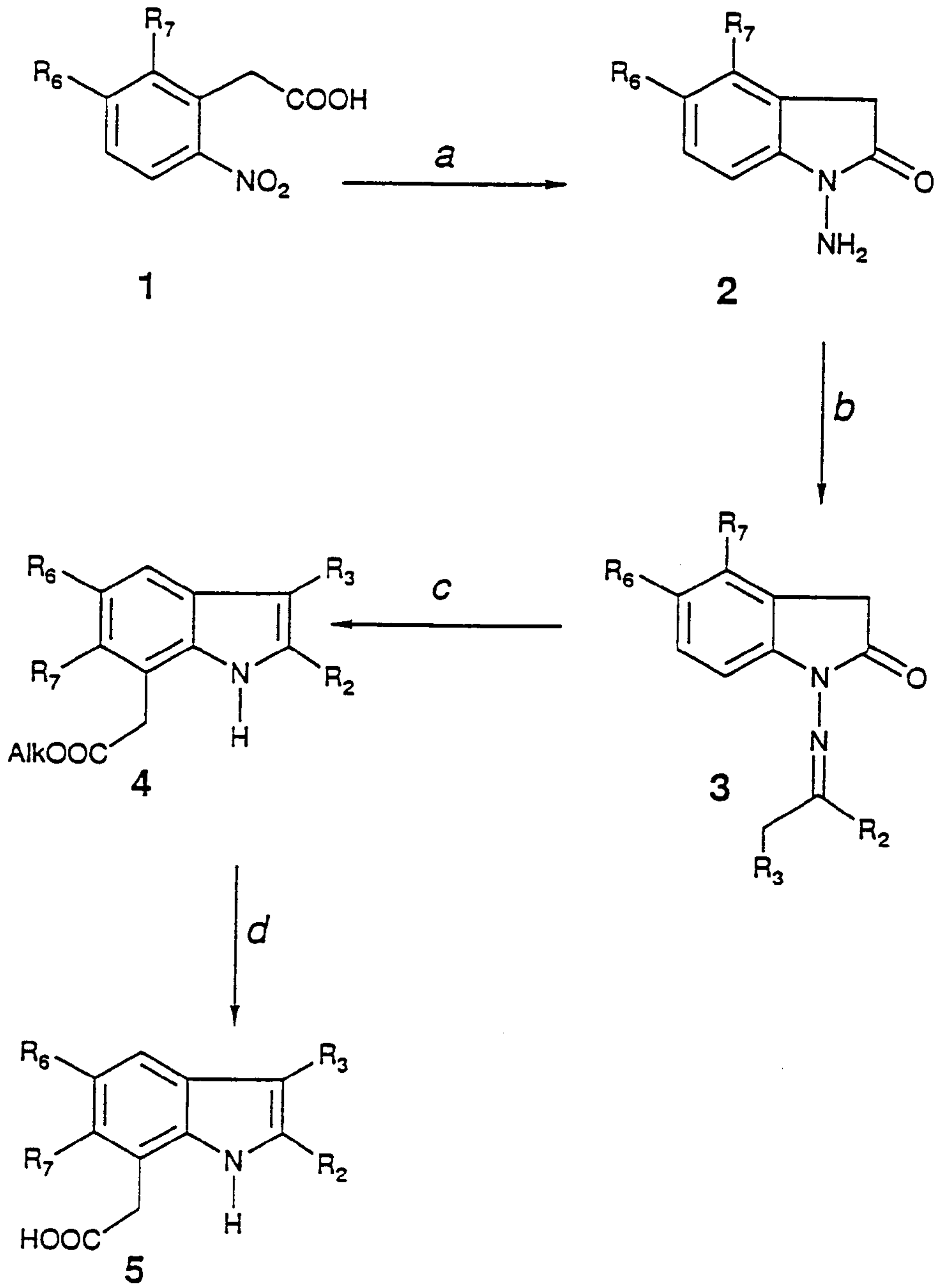


1

2

3

4

5

Step 15a

- Hydrogen in the presence of 10% Pd/C as catalyst at 45 lbs in water containing one equivalent of NaOH, followed by diazotation with sodium nitrite in HCl at 0-5°C and stannous chloride. Cyclization is perfomed during acidification of the tin salt with $H_2S$ and completed by refluxing in xylene, see H.E. Baumgarten et al., *J. Am. Chem. Soc.*, 82, 3977, 1960.

Step 15b

- $R_3CH_2COR_2$ in the presence of an acid (e.g. acetic acid) in a polar solvent (e.g. EtOH) at reflux as reported

by W.J. Welstead et al., *J. Med.Chem.*, 22, 1074 (1979) for $R_2$=$CH_3$ and $R_3$=$C_6H_5$, where also step 15c and 15d are reported;

Step 15c

- Lower alkanol (e.g. MeOH, EtOH) at reflux in the presence of a hydrogen chloride stream;

Step 15d

- a strong base (e.g. KOH) in a polar solvent (e.g. water) at reflux.

The preparation of simple starting materials having $R_3$ = hydroxyalkyl and/or the corresponding ethers can be carried out by reacting either Compounds (3) of Reaction Scheme 1, Compounds (2), (4) or (5) of Reaction Scheme 2, Compounds (4) of Reaction Schemes 6, 10, 11 and 15, Compounds (5) of Reaction Scheme 13 and Compounds (4) and (6) of Reaction Scheme 14 having $R_3$ = H, $CH_3$ according to the Reaction Scheme 16, wherein A and B have the same meanings as in Reaction Scheme 1, $R_4$ represents an alkyl or aralkyl group and $R_5$ represents an H or alkyl group:

## SCHEME 16

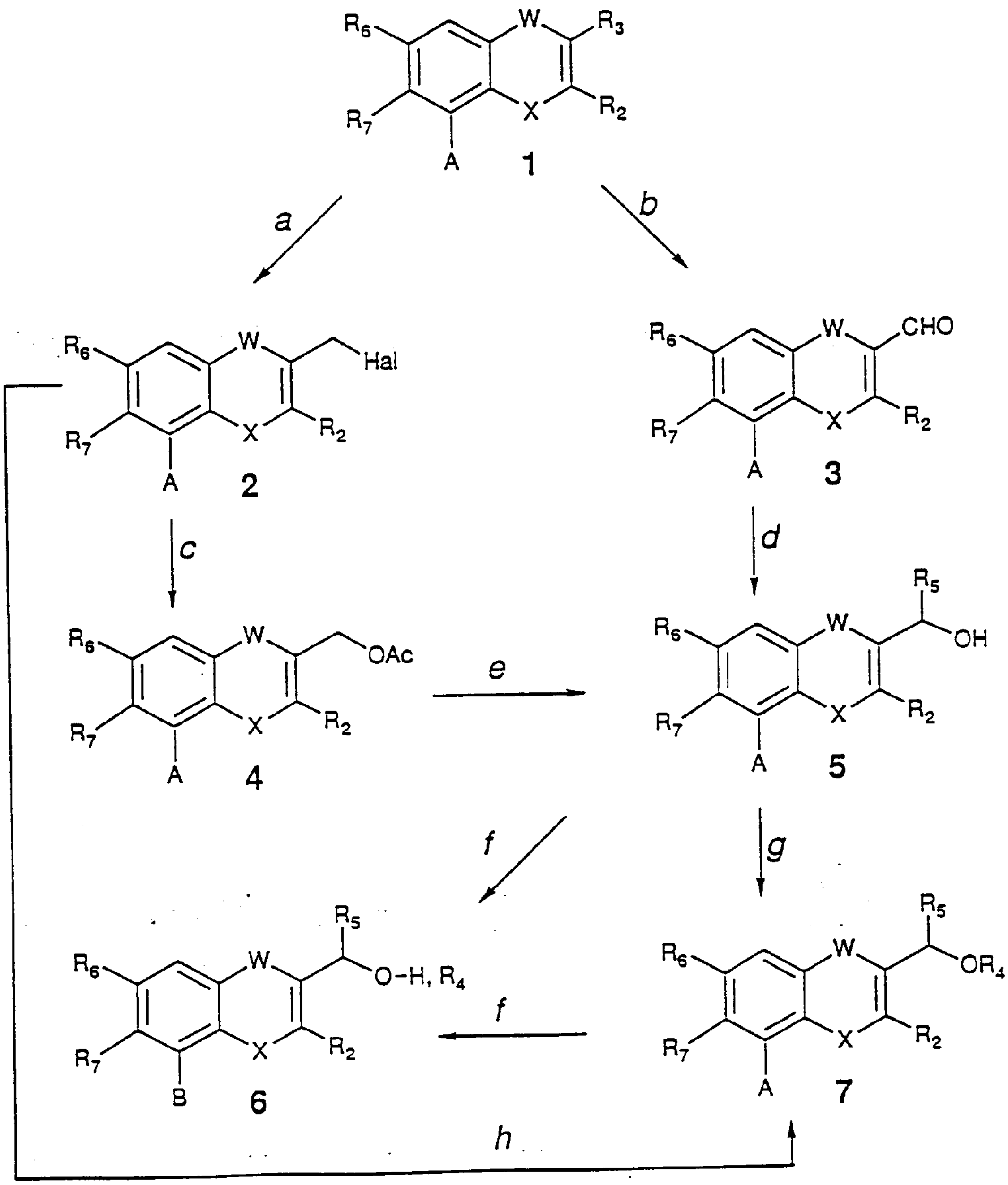


$R_4$ = Alk, Aralk
$R_5$ = H, Alk

Step 16a:

$R_3$ = H, W = CO, CS (and no activated phenyl rings present):
- Formaldehyde and HCl in water, EtOH or AcOH at 50-100°C;

- Chloromethyl methyl ether and fuming sulphuric acid at 50-70°C (H. Nakarumo et al., *Bull. Chem. Soc. Jap.*, 57, 2323, 1984);

$R_3 = CH_3$, W = CO, CS, bond, and no other methyl groups in the molecule:

- N-bromosuccinimide in presence of benzoyl peroxide or 2,2'-azobisisobutyronitrile in $CCl_4$ at 50-80°C;

Step 16b

$R_3$ = H, W = bond, X = O, S, NH or N-Alk and no electrondonating groups are present on the other rings of the molecule:

- Phosphorous oxychloride and DMF at 50-140°C, or other Vilsmeyer - Haack reagents (see Jutz, *Adv. Org. Chem.*, 9, 225, 1976);

$R_3 = CH_3$, W = bond, X = O, S, NH or N-Alk and no other $CH_3$ groups are present:

- Irradiation with a Hg high-pressure lamp in a protic solvent (e.g. AcOH) at 20-100°C as reported by Frasca et al., *Tetrahedron*, 23, 603, 1973.

Step 16c

- Sodium or potassium acetate in aprotic solvents (e.g. acetone, DMF) at 40-120°.

Step 16d

$R_5$ in Compounds (5) = H:

- A reducing hydride (e.g. $NaBH_4$) in a polar solvent (e.g. MeOH or EtOH or dioxane) at 0-80°C;

$R_5$ in Compounds (5) = alkyl:

- Alkyl magnesium bromide in aprotic solvents (e.g. $Et_2O$, THF) at 0-60°C;

Step 16e

- NaOH or LiOH in protic solvents (e.g. alcohols, water) or mixture thereof at 25-50°C. (In this case, when A = COOAlk it can be simultaneously hydrolized to COOH);

Step 16f

- The same methods reported in step 1g of Reaction Scheme 1, but the oxidation of CH = $CHCH_3$ to COOH for compounds (5);

Step 16g

- A strong base (e.g. NaH) and an $R_4$-L reagent (where L is an halogen atom or a tosyloxy group) in anhydrous aprotic solvents (e.g. DMF or THF) at 20-140°C;

Step 16h

- $R_4OH$ and a base (e.g. Na, NaH) in excess $R_4OH$ or in aprotic solvents (e.g. DMF or THF) at 20-140°C.

The simple Compounds (6) having an hydroxyalkyl group at position 3, obtained in this way, can be reacted as such or, alternatively, derivatized at the hydroxymethyl group with known reagents and methods, so that said group does not interfere in the further reaction steps necessary to prepare those compounds of formula (I) which bear a protected hydroxyalkyl group such as $R_3$.

The protected final compounds are finally converted by deprotecting methods to compounds of formula (I) having $R_3$ = hydroxalkyl group.

Prodrugs, as above defined, may be prepared from the corresponding hydroxy compounds by Method 1 below or from the corresponding amide compounds by Method 2 below.

Method 1

- by reacting with a chloroformate, an isocyanate or isothiocyanate, a carbonyl chloride or bromide or another activated acid derivative (e.g. anhydride) in a suitable solvent (e.g. chlorinated solvent, DMF, THF, dioxane, acetonitrile, pyridine) in the presence or absence of a base (for instance, $NEt_3$, pyridine, 4-di-

methylaminopyridine, NaOH, potassium carbonate or 1,10-diazabicycloundecene or others not specified) at -20/100°C;

- by reacting with a carboxylic acid in the same solvents as above, in the presence of a condensing agent such as N, N'-carbonyldiimidazole, carbodiimides or others known to the people skilled in the art;
- by reacting with a dialkyl or diaryl chlorophosphate or dialkyl cyanophosphonate in the same conditions described above (for examples of such derivatization methods see Example 114 below and S.O. Thorberg et al., *J. Med. Chem.*, 30, 2008, 1987).

Method 2

Prodrugs derivatives of "acidic" NH groups as discussed above can be synthesized from the compounds of formula I by preparing an N-hydroxy(substituted)methyl derivative and reacting that in the same described above for oxygen derivatization.

The intermediate N-hydroxy(substituted)methyl derivative can be isolated or directly reacted to give the desired compound.

N-hydroxy(substituted)methyl derivatives of the type Ny-CH($R_1$)OH where $R_1$ = H or $CCl_3$ can be obtained by reacting the appropriate compounds of formula I with formaldehyde or $CCl_3CHO$ as described in H.E. Zaugg, *Organic Reactions*, 14, Chapter 2, 52 J. Wiley and Sons New York, 1965 or in J. P. Chupp, *J. Org. Chem.*, 28, 2592, 1965.

In the case where $R_1$ = phenyl said compounds can be synthesized by reacting with benzaldehyde and cyclic amine (e.g. morpholine) in MeOH or dichloromethane:MeOH 1:1 at 0°C to reflux and hydrolyzing the intermediate with 0.1N HCl at pH 4. (O. Jacobseen, *Annalen*, 157, 243, 1884; H. Bundgaard et al. *Int. J. Pharm.*, 22, 45, 1984).

All the above described reaction pathways and steps are to be intended as examples and are not limiting the scope of the invention. Those skilled in the art are aware that said chemical transformations are performed on polyfunctional substrates and that the reagents used might interfere, reacting also with other groups present in the molecules. For example, catalytic hydrogenation can transform nitro groups into amino groups as desired, but also isolated double bonds might be hydrogenated and halogen atoms removed; lithium aluminium hydride can reduce conjugated ketones to alkanes as desired (e.g. step 7c in Reaction Scheme 7) but also COOAlk groups to $CH_2OH$ or $NO_2$ groups to -N=N- etc.. The undesired side reactions can be avoided or minimized by choosing the appropriate conditions or by using alternative reagents or different synthetic pathways. Should this "alternative" approach give negative results, the undesired obtained intermediates must be transformed into useful ones using methods known to those skilled in the art.

## DETAILED PREPARATION OF INTERMEDIATES

8-(3-bromopropoxycarbonyl)-3-methyl-4-oxo-2-phenyl-4H-1-benzopyran (Intermediate I)

30 g of 1,3-dibromopropane was added dropwise at ambient temperature to a suspension of 30 g of sodium 3-methyl-4-oxo-2-phenyl-4H-1-benzopyran-8-carboxylate in 150 ml of dimethylformamide and 35 ml of water. The reaction mixture was stirred at ambient temperature for 5 days. 100 ml of water was added and stirring was continued for a further 15 minutes. The precipitate was filtered off by suction, washed with water and purified by flash chromatography on silica gel, eluting with chloroform:ethyl acetate 95:5. The collected fractions were evaporated to dryness in vacuo and the residue was recrystallized from ethanol to give 27.7 g of the title compound, m.p. 114-115°C.

The benzopyran carboxylate salt used in the foregoing synthesis was prepared by dissolving 104 g of the corresponding acid in 560 ml of hot methanol and adding 280 ml of an aqueous solution of 31 g of sodium hydrogen carbonate. 850 ml of acetone was added to the solution to precipitate the desired salt, which was collected by suction filtration (62 g, m.p. > 280°C). The corresponding acid was prepared according to Da Re, P. et al., *J. Med. Pharm. Chem.*, 2, 263, 1960.

8-hydroxymethyl-3-methyl-4-oxo-2-phenyl-4H-1-benzopyran (Intermediate II)

467 ml of a 1.48N solution of sodium borohydride in anhydrous dimethylformamide was added over a period of 30 minutes, under stirring at ambient temperature, to a solution of 100 g of 3-methyl-4-oxo-2-phenyl-4H-1-benzopyran-8-carbonyl chloride in 1 litre of anhydrous dimethylformamide. The reaction mixture was stirred for 2½ hours at ambient temperature. 88 ml of 2N hydrochloric acid was added while maintaining the temperature at 0-5°C. 102 ml of 12.7N sodium hydroxide solution was then added. The mixture was poured into

6 litres of water, stirred for 3 hours, and filtered on a Buchner funnel. The filter cake was washed with 4N sodium hydroxide solution and then with water. The resultant white solid was crystallized from methanol to give 50 g of the title compound, m.p. 145-147°C.

E-8-(2-carboxyvinyl)-3-methyl-4-oxo-2-phenyl-4H-1-benzopyran (Intermediate III)

A mixture of 7.92 g of 8-formyl-3-methyl-4-oxo-2-phenyl-4H-1-benzopyran (prepared as described in Uneyama, K. et al., *Bull. Chem. Soc. Jap.*, 58, 2361, 1985), 3.75 g of malonic acid and 0.46 ml of piperidine in 15 ml of anhydrous pyridine was stirred at 100°C for 3 hours. After cooling to 20-25°C the reaction mixture was poured into a mixture of 90 g of crushed ice and 33 ml of hydrochloric acid (d=1.18). The resultant precipitate was collected by suction filtration, washed with water and crystallized twice from 95% ethanol to give 5.5 g of the title compound, m.p. 226-229°C.

E-8-(2-chlorocarbonylvinyl)-3-methyl-4-oxo-2-phenyl-4H-1-benzopyran (Intermediate IV)

A solution of 9.2 g of Intermediate III and 7.8 g of thionyl chloride in 75 ml of toluene was refluxed for 3 hours. After cooling to 20-25°C the resultant crystals were collected by suction filtration, washed with acetone and dried in vacuo to give 6.8 g of the title compound, m.p. (190) 196-198°C after recrystallization from toluene.

8-acetyl-3-methyl-4-oxo-2-phenyl-4H-1-benzopyran (Intermediate V)

1.17 g of magnesium turnings, 7.4 ml of anhydrous ethanol and 0.2 ml of anhydrous carbon tetrachloride were placed in a round bottomed flask under a stream of nitrogen. When the temperature began to rise, 7.5 ml of anhydrous chlorobenzene was added, followed by the slow dropping (25 minutes) of a solution of 5.28 ml of anhydrous diethylmalonate and 3.5 ml of anhydrous chlorobenzene in 16 ml of anhydrous ethanol. The reaction flask was heated to 75°C for two hours, cooled to 25°C and a solution of 8.8 g of 3-methyl-4-oxo-2-phenyl-4H-1-benzopyran-8-carbonyl chloride in 88 ml of anhydrous chlorobenzene was slowly added, without exceeding 35°C. The reaction mixture was further stirred for two hours at 35°C and then cooled to 0°C. 13 ml of water and 1.9 ml of sulphuric acid (d = 1.84) were added. The solution obtained was decanted from the insoluble inorganic matter and stripped in vacuo.

The crude acylmalonate obtained was refluxed for six hours with 10.4 ml of acetic acid, 7 ml of water and 1.3 ml of sulphuric acid (d = 1.84). After cooling, the solution was poured into iced water and the precipitate was collected by suction filtration and washed with aqueous sodium carbonate. Crystallization from 90% ethanol gave 6.5 g of the title compound, m.p. 159-161°C.

8-bromoacetyl-3-methyl-4-oxo-2-phenyl-4H-1-benzopyran (Intermediate VI)

A solution of 11.2 g of bromine in 250 ml of chloroform was added, over a period of two hours at 20-25°C, to a solution of 19.5 g of the Intermediate V in 700 ml of chloroform. After stirring for 1 hour at 20-25°C, the solution was washed with 400 ml of 2N aqueous sodium hydroxide solution and then repeatedly with water, dried with anhydrous sodium sulphate and stripped in vacuo. The crude product was treated with diethyl ether, collected by suction filtration and crystallized from acetone, yielding 16 g of the title compound, m.p 134-135°C.

8-(2-hydroxyethylcarbamoyl)-3-methyl-4-oxo-2-phenyl-4H-1-benzopyran (Intermediate VII)

The title compound was prepared in the same manner as Intermediate XXXVI, but using 2-aminoethanol instead of 3-aminopropanol. M.p. 206-208°C.

3-methyl-4-oxo-2-phenyl-4H-1-benzopyran-8-sulphonyl chloride (Intermediate VIII)

A solution of 4.55 g of sodium nitrite in 12 ml of water was added dropwise to a stirred mixture of 15.1 g of 8-amino-3-methyl-4-oxo-2-phenyl-4H-1-benzopyran [prepared as described in Da Re, P. et al., *Il. Farmaco* (Ed. Sci.), 11, 670, 1956] in 150 ml of hydrochloric acid (d=1.18) at -5°C. Stirring was continued at 0°C for 30 minutes. The solution was poured, over a period of 10 minutes and at -5 to 0°C, into 120 ml of a 30% by weight solution of sulphur dioxide in acetic acid containing 1.53 g of cupric chloride dihydrate and 13 ml of water. After 1 hour at 0°C and 1 hour at 20-25°C, 300 ml of iced water was added to the mixture. A precipitate formed and was collected by suction filtration, washed with water and dried in a desiccator over sodium hydroxide until of constant weight to give 18 g of crude title product, m.p. 165-170°C, for use without further purification.

8-(3-chloropropoxy)-3-methyl-4-oxo-2-phenyl-4H-1-benzopyran (Intermediate IX)

This compound was prepared in the same manner as Intermediate XI, but using 1-bromo-3-chloropropane instead of 1-bromo-2-chloroethane. m.p. 98-102°C after washing with petroleum ether:diethyl ether 7:3.

8-acrylamido-3-methyl-4-oxo-2-phenyl-4H-1-benzopyran (Intermediate X)

A solution of 1.75 ml of acryloyl chloride in 15 ml of anhydrous tetrahydrofuran was added dropwise at -10°C to a stirred mixture of 5 g of 8-amino-3-methyl-4-oxo-2-phenyl-4H-1-benzopyran and 3 ml of triethylamine in 60 ml of anhydrous tetrahydrofuran. After stirring at 0°C for 1 hour and at ambient temperature for 1 hour, the reaction mixture was poured into water and filtered under suction. The filter cake was washed with water. Desiccation gave 5.5 g of the title compound, m.p. 229-230°C.

8-(2-chloroethoxy)-3-methyl-4-oxo-2-phenyl-4H-1-benzopyran (Intermediate XI)

A mixture of 7.52 g of 8-hydroxy-3-methyl-4-oxo-2-phenyl-4H-1-benzopyran (prepared as described in Da Re, P. et al., *Ann. Chim.*, 1962, p.506 et seq.), 6.22 g of anhydrous potassium carbonate and 25.5 ml of 1-bromo-2-chloroethane in 70 ml of dimethylformamide was stirred at 60°C for 25 hours. The mixture was cooled to 20-25°C and poured into 600 ml of water. The organic solution, obtained by extraction with dichloromethane, was washed with aqueous sodium chloride solution and dried on anhydrous sodium sulphate. The solvents and excess 1-bromo-2-chloroethane were evaporated off in vacuo to yield 8.8 g of the title compound, m.p. 141-142°C after crystallization from chloroform:hexane.

8-(2-azidoethoxy)-3-methyl-4-oxo-2-phenyl-4H-1-benzopyran (Intermediate XII)

A mixture of 15.2 g of Intermediate XI and 6.24 g of sodium azide in 150 ml of anhydrous dimethylformamide was stirred at 70-75°C for 12 hours. After cooling to 20-25°C, the reaction mixture was poured into 1½ litres of water and extracted with dichloromethane. The organic solution was washed with aqueous sodium chloride solution and dried on anhydrous sodium sulphate. The solvents were evaporated off in vacuo. The residue was taken up in water, collected by suction filtration and dried to give 14 g of the title compound, m.p. 119-120°C.

8-[N-(2-hydroxyethyl)-N-methyl-carbamoyl]-3-methyl-4-oxo-2-phenyl-4H-1-benzopyran (Intermediate XIII)

A solution of 1.6 ml of 2-methylamino-ethanol in 10 ml of water was added dropwise over a period of 5 minutes to a suspension of 6 g of 8-chlorocarbonyl-3-methyl-4-oxo-2-phenyl-4H-1-benzopyran and 1.52 g of potassium carbonate in 60 ml of acetone. After stirring for 2½ hours at 20-25°C, the solvent was removed in vacuo and the residue was taken up in 150 ml of acetone. The mixture was refluxed for 15 minutes, and was then filtered. The solvent was evaporated from the filtrate and the residue was dissolved in 20 ml of dimethylformamide, treated with 14 ml of 1.4% sodium carbonate solution, stirred for 30 minutes at 20-25°C and diluted by addition of 150 ml of water. The mixture was extracted with chloroform and the organic layer was washed with 0.5N hydrochloric acid and then with water. The solution was dried over anhydrous sodium sulphate and the chloroform was evaporated off. The resulting oil was taken up in 200 ml of diethyl ether and stirred for 2 hours at 20-25°C. The solids were collected by filtration and crystallized from ethyl acetate to give 4.97 g of the title compound, m.p. 128-130°C.

8-(2-chloroethylcarbamoyl)-3-methyl-4-oxo-2-phenyl-4H-1-benzopyran (Intermediate XIV)

The title compound was prepared in the same manner as Intermediate XXXVII, but using Intermediate VII in place of Intermediate XXXVI and carrying out the reaction at ambient temperature. M.p. 181-182°C (ethyl acetate).

8-(N-methyl-2-chloro-ethylcarbamoyl)-3-methyl-4-oxo-2-phenyl-4H-1-benzopyran (Intermediate XV)

A solution of 1.1 ml of thionyl chloride in 2 ml of dichloromethane was added to a solution of 3.37 g of Intermediate XIII in 20 ml of dichloromethane, and the mixture was stirred for 4 hours at ambient temperature. Removal of the solvent gave an oil which was taken up in diethyl ether. The title compound precipitated as a white solid, collected by filtration for use without further purification. M.p. (118) 126-128°C (diethyl ether).

8-(4-bromobutoxy)-3-methyl-4-oxo-2-phenyl-4H-1-benzopyran (Intermediate XVI)

A mixture of 5 g of 8-hydroxy-3-methyl-4-oxo-2-phenyl-4H-1-benzopyran, 4.2 g of anhydrous potassium carbonate and 43.6 g of 1,4-dibromobutane in 45 ml of dimethylformamide was stirred at 75°C for 2 hours. The mixture was cooled to 20-25°C, poured into 100 ml of water and extracted with dichloromethane. The organic solution was washed with aqueous sodium chloride solution and dried on anhydrous sodium sulphate. The solvents and excess 1,4-dibromobutane were evaporated off in vacuo. The residue was rinsed with 55 ml of petroleum ether:diethyl ether 7:4 and collected by suction filtration to yield 5.6 g of the title compound, m.p. 91-92°C.

8-(5-bromopentyloxy)-3-methyl-4-oxo-2-phenyl-4H-1-benzopyran (Intermediate XVII)

This compound was prepared by the method described for the preparation of Intermediate XVI, but using 1,5-dibromopentane in place of 1,4-dibromobutane and purifying the crude product by column chromatography on silica gel (elution with dichloromethane:ethyl acetate 99:1). m.p. 75-76°C, after rinsing with petroleum ether:diethyl ether 30:4.

8-(2-chloroethoxymethyl)-3-methyl-4-oxo-2-phenyl-4H-1-benzopyran (Intermediate XVIII)

6 ml of thionyl chloride in 18 ml of chloroform was added at 0°C to a stirred solution of 23 g of Intermediate XXII and 11 ml of triethylamine in 185 ml of chloroform. The reaction mixture was warmed to 70°C and stirred for 2 hours. After cooling to ambient temperature, it was poured into water. The organic layer was separated, washed with sodium chloride solution, dried on anhydrous sodium sulphate and evaporated to dryness in vacuo. Yield: 24 g of the title compound. A sample crystallized from ethanol had a melting point of 102-103°C.

8-chloromethyl-3-methyl-4-oxo-2-phenyl-4H-1-benzopyran (Intermediate XIX)

53.4 g of Intermediate II and 38.8 ml of anhydrous triethylamine were dissolved in 440 ml of chloroform. Into this solution, maintained at -10 to -2°C, there was dropped a solution of 19.8 ml of thionyl chloride in 80 ml of anhydrous chloroform. The reaction mixture was stirred at ambient temperature for 4 hours, and then diluted with 400 ml of water. The aqueous phase was extracted with chloroform, and the extracts were added to the chloroform phase. The chloroform solution was washed with brine, dried on anhydrous sodium sulphate and evaporated to dryness in vacuo. Yield: 56 g of the title compound, which on recrystallization from ethanol was shown to have a melting point of 112-113°C.

8-methylaminomethyl-3-methyl-4-oxo-2-phenyl-4H-1-benzopyran (Intermediate XX)

A solution of 15.1 g of anhydrous zinc chloride and 14.5 g of sodium cyanoborohydride in 400 ml of anhydrous methanol was added dropwise at 0°C into a stirred mixture of 58.8 g of 8-formyl-3-methyl-4-oxo-2-phenyl-4H-1-benzopyran, 60.7 g of methylamine hydrochloride and 125 ml of triethylamine in 600 ml of anhydrous methanol. After stirring for 5 hours at 20-25°C, the solvent was evaporated off in vacuo and the residue was taken up in 200 ml of water and collected by suction filtration. The crude product was dissolved in aqueous acetic acid, washed with ethyl acetate and reprecipitated by addition of cold 6N sodium hydroxide solution. 49 g of the title compound was obtained. m.p. 97-99°C, after crystallization from 75% ethanol.

8-(2-chloroethylthiomethyl)-3-methyl-4-oxo-2-phenyl-4H-1-benzopyran (Intermediate XXI)

A solution of 37 g of Intermediate XIX and 10.5 g of thiourea in 370 ml of ethanol was refluxed for 1 hour. The reaction mixture was cooled to ambient temperature, and 42 g of 8-amidinothiomethyl-3-methyl-4-oxo-2-phenyl-4H-1-benzopyran spontaneously crystallized. A sample recrystallized from ethanol had a melting point of 233-235°C.

48 ml of 35% aqueous sodium hydroxide solution was added to a vigorously stirred suspension of 35 g of the compound thus prepared and 1.05 g of benzyl triethylammonium chloride in 440 ml of 1,2-dichloroethane. The mixture was stirred for 2½ hours and then poured into 300 ml of water. The aqueous layer was extracted with 1,2-dichloroethane and the extracts were added to the organic layer which was washed with sodium chloride solution, dried on anhydrous sodium sulphate, and evaporated to dryness in vacuo. The residue was crystallized from methanol, giving 22 g of the title compound, m.p. 82-83°C.

8-(2-hydroxyethoxymethyl)-3-methyl-4-oxo-2-phenyl-4H-1-benzopyran (Intermediate XXII)

A solution of 2.5 g of Intermediate XIX in 25 ml of xylene and 3 ml of dioxane was prepared. 0.15 g of sodium was dissolved in 3.10 ml of anhydrous ethylene glycol, and this solution was added dropwise at ambient temperature to the solution of Intermediate XIX. After refluxing for 5½ hours, the reaction mixture was cooled to ambient temperature and poured into 50 ml of water. It was extracted with dichloromethane, and the extract was washed with sodium chloride solution, dried on anhydrous sodium sulphate and evaporated to dryness in vacuo. The solid residue was crystallized from ethanol, giving 2.1 g of the title compound, m.p. 132-133°C.

8-trifluoroacetamido-3-methyl-4-oxo-2-phenyl-4H-1-benzopyran (Intermediate XXIII)

A solution of 9.5 ml of trifluoroacetic anhydride in 20 ml of anhydrous dichloromethane was added dropwise at -5 to 0°C to a solution of 5 g of 8-amino-3-methyl-4-oxo-2-phenyl-4H-1-benzopyran in 50 ml of anhydrous dichloromethane. The reaction mixture was stirred for 2 hours at 20-25°C and then poured on to crushed ice. The organic solution obtained by extraction with dichloromethane was washed with cold 5% aqueous sodium bicarbonate solution and with water, and was dried on anhydrous sodium sulphate. The solvent was removed in vacuo and the residue was crystallized from ethanol to give 5.2 g of the title compound, m.p. 175-176°C.

8-aminomethyl-3-methyl-4-oxo-2-phenyl-4H-1-benzopyran (Intermediate XXIV)

A mixture of 21 g of Intermediate XXIX and 19 g of triphenylphosphine in 160 ml of tetrahydrofuran was stirred at ambient temperature for 8 hours. Thin layer chromatography showed the disappearance of Intermediate XXIX. 3 ml of water was added, and stirring was continued for a further 24 hours. The solvents were removed on a rotary evaporator, and the residue was dissolved in water as its acetate. The aqueous solution was washed with ethyl acetate, made basic with 37% sodium hydroxide solution and filtered on a Buchner funnel. The filter cake was washed with water and desiccated to give 18 g of the title compound. The hydrochloride, recrystallized from ethanol, had a melting point of 256-258°C.

8-(2-chloroethylsulphonylmethyl)-3-methyl-4-oxo-2-phenyl-4H-1-benzopyran (Intermediate XXV)

41.6 ml of aqueous 30% hydrogen peroxide was added dropwise at 40°C over a period of 20 minutes to a solution of 26.2 g of Intermediate XXI in 300 ml of glacial acetic acid. The mixture was heated to 60°C, stirred at that temperature for 4½ hours, cooled to ambient temperature and poured into 60 ml of water. Filtration on a Buchner funnel gave a filter cake which was washed with water and desiccated, yielding 29.4 g of the title compound. A sample was crystallized from ethanol. m.p. (89) 159-161°C.

8-(2-chloroethylsulphinylmethyl)-3-methyl-4-oxo-2-phenyl-4H-1-benzopyran (Intermediate XXVI)

36 ml of aqueous 30% hydrogen peroxide was quickly added dropwise at 10°C to a solution of 12 g of Intermediate XXI in 84 ml of glacial acetic acid. The reaction mixture was stirred for 4 hours at ambient temperature, and then poured into 220 ml of water. The title compound was collected by suction filtration, washed with water and desiccated. Yield 12.4 g, m.p. 142-145°C (methanol).

8-[N-methyl-N-(2-chloroethyl)-aminomethyl]-3-methyl-4-oxo-2-phenyl-4H-1-benzopyran (Intermediate XXVII)

A mixture of 22 g of Intermediate XX, 66 ml of 1-bromo-2-chloroethane and 11 g of anhydrous potassium carbonate in 88 ml of dimethylformamide was stirred at 20-25°C for 12 hours. The reaction mixture was then poured into 600 ml of water and extracted with dichloromethane. The organic layer was washed with water, dried on anhydrous sodium sulphate and acidified with ethanolic hydrogen chloride. The solvent and the excess 1-bromo-2-chloroethane were distilled off in vacuo at 70-80°C. The residue was taken up in cold 1N aqueous sodium hydroxide solution and extracted with dichloromethane. The organic solution was washed with water, dried on anhydrous sodium sulphate and evaporated to dryness in vacuo at 25-30°C. The crude title product was purified by flash chromatography on silica gel, eluting with ethyl acetate:petroleum ether 7:3, to give 18 g of the title compound melting at 118-120°C after crystallization from ethanol.

1-(2-hydroxy-2-methylpropyl)-4-(2-methoxyphenyl)-piperazine (Intermediate XXVIII)

A mixture of 7 g of 1-(2-methoxyphenyl)-piperazine, 7.33 g of anhydrous potassium carbonate, 1.75 g of potassium iodide and 5.6 ml of 1-chloro-2-methyl-2-propanol was stirred for 90 minutes at 70°C and for a further 6 hours at 90°C. The reaction mixture was poured into iced water and extracted with ethyl acetate. The organic layer was washed with aqueous sodium chloride solution, dried on anhydrous sodium sulphate and evaporated to dryness in vacuo. The title product was obtained as an oil, and was characterised as its dihydrochloride, crystallized from ethanol, melting at 225-227°C.

8-azidomethyl-3-methyl-4-oxo-2-phenyl-4H-1-benzopyran (Intermediate XXIX)

A mixture of 22.8 g of Intermediate XIX and 6.8 g of sodium azide in 110 ml of dimethylformamide was stirred for 3 hours at 100°C. After cooling to ambient temperature, 130 ml of water and 88 ml of ethanol were added to the reaction mixture. After 1 hour, the crystals were collected by vacuum filtration, washed with water, and desiccated. Yield: 22 g of the title product. A sample recrystallized from ethanol had a melting point of 132-134°C.

8-[N-(2-hydroxyethyl)-aminomethyl]-3-methyl-4-oxo-2-phenyl-4H-1-benzopyran (Intermediate XXX)

A solution of 2.38 g of anhydrous zinc chloride and 2.30 g of sodium cyanoborohydride in 71 ml of anhydrous methanol was added dropwise under stirring to a mixture of 9.24 g of 8-formyl-3-methyl-4-oxo-2-phenyl-4H-1-benzopyran and 9.12 g of ethanolamine in 90 ml of anhydrous methanol. Stirring was continued at 20-25°C for 5 hours, before removal of the solvent in vacuo. 250 ml of water was added to the residue, and the insoluble matter was collected by suction filtration and washed with water. The crude product was dissolved in 1N acetic acid and the solution was washed with ethyl acetate. The aqueous solution was then made alkaline by addition of 2N sodium hydroxide solution and the precipitate was collected by suction filtration and washed with water to give 8.5 g of the title compound, m.p. 117-121°C after drying at 60°C.

8-(N-methyl-N-chloracetyl-aminomethyl)-3-methyl-4-oxo-2-phenyl-4H-1-benzopyran (Intermediate XXXI)

A solution of 6 ml of chloracetyl chloride in 60 ml of 1,2-dichloroethane was added dropwise at -5 to 0°C to a solution of 20 g of Intermediate XX and 10 ml of triethylamine in 200 ml of 1,2-dichloroethane. After stirring at 20-25°C for 2 hours, 150 ml of water was added to the reaction mixture and the phases were separated. The organic phase was washed with water and dried on anhydrous sodium sulphate. The solvent was removed in vacuo, and the residue was crystallized from ethanol to give 22.5 g of the title compound, m.p. 146-148°C.

8-chloracetamidomethyl-3-methyl-4-oxo-2-phenyl-4H-1-benzopyran (Intermediate XXXII)

A solution of 3.2 ml of chloracetyl chloride in 32 ml of 1,2-dichloroethane was added dropwise, under stirring at -5°C, to a mixture of 10 g of Intermediate XXIV and 5.5 ml of triethylamine in 80 ml of 1,2-dichloroethane. The reaction mixture was stirred at ambient temperature for 1 hour and then 150 ml of water was added. The phases were separated; the aqueous phase was extracted with 1,2-dichloroethane and the extracts were added to the organic phase which was then washed with a cold saturated solution of sodium bicarbonate, washed with water, dried on anhydrous sodium sulphate and evaporated to dryness in vacuo. The residue was crystallized from ethanol to give 10.7 g of the title compound, m.p. 152-155°C.

8-[N-acetyl-N-(2-chloroethyl)-aminomethyl]-3-methyl-4-oxo-2-phenyl-4H-1-benzopyran (Intermediate XXXIII)

8.65 g of Intermediate XXX and 4.15 ml of triethylamine were dissolved in 70 ml of tetrahydrofuran. To this solution, at -10°C, there was added dropwise over a period of 40 minutes a solution of 2.35 ml of acetyl chloride in 23 ml of tetrahydrofuran. After stirring for 3 hours at 0-10°C and for 2 hours at 20-25°C, the solvent was evaporated off in vacuo. 100 ml of water was added to the residue, and extraction with dichloromethane was effected, pooling the successive organic extracts and then removing the solvent in vacuo. The residue was dissolved in 50 ml of methanol and 3 g of potassium carbonate and 10 ml of water were added. After stirring at 50°C for 20 minutes, to hydrolyse the N,O-diacetyl derivative which had formed, the solvent was removed in vacuo and the residue was treated with water and dichloromethane as above described. The dichloromethane solution was again evaporated to dryness, and 5.9 g of 8-[N-acetyl-N-(2-hydroxyethyl)-aminomethyl]-3-methyl-4-oxo-2-phenyl-4H-1-benzopyran, m.p. 171-172°C, was obtained. 3.6 ml of thionyl chloride in 30 ml of dichloromethane was added dropwise at O°C to a solution of 6.1 g of the compound thus prepared in 70 ml of dichloromethane. After stirring for 90 minutes at 20-25°C, the reaction mixture was washed with water and dried. The solvent was removed in vacuo to give the crude title product for use without further purification.

8-(3-chloropropylthio)-3-methyl-4-oxo-2-phenyl-4H-1-benzopyran (Intermediate XXXIV)

A solution of 20.1 g of stannous chloride dihydrate in 18 ml of hydrochloric acid (d=1.18) was added over a period of 5 minutes at 65°C to a solution of 6 g of Intermediate VIII in 70 ml of acetic acid. After 10 minutes, the reaction mixture was cooled to 30-35°C and the solvent was removed in vacuo. The residue was taken up in water, and the insoluble matter was collected by suction filtration, washed with water and dried. Yield 3.2 g of 8-mercapto-3-methyl-4-oxo-2-phenyl-4H-1-benzopyran, melting at 115-118°C after crystallization from ethanol.

A mixture of 8 g of the compound so prepared, 27 ml of 1-bromo-3-chloro-propane, 0.2 g of tetrabutylammonium bromide and 6.2 ml of 35% sodium hydroxide in 80 ml of benzene was vigorously stirred for 4 hours at 20-25°C. 100 ml of water and 40 ml of dichloromethane were added. The organic layer was separated off, washed with water and dried on anhydrous sodium sulphate. The solvents and excess 1-bromo-3-chloro-propane were removed in vacuo. The residue was purified by column chromatography on silica gel, eluting with petroleum ether:ethyl acetate 9:1, and 5.7 g of the title compound was obtained. After crystallization from methanol, it showed a melting point of 84-86°C.

8-(3-chloropropylsulphonyl)-3-methyl-4-oxo-2-phenyl-4H-1-benzopyran (Intermediate XXXV)

7 ml of 30% hydrogen peroxide was added at 20-25°C to a solution of 3.45 g of Intermediate XXXIV in 35 ml of acetic acid. After stirring for 4 hours at 60°C, the reaction mixture was cooled to 20-25°C. 30 ml of water was added. A precipitate formed, and was collected by suction filtration, washed with water and dried, yielding 3.4 g of the title compound. After crystallization from acetone, it showed a melting point of 160-163°C.

8-(3-hydroxypropylcarbamoyl)-3-methyl-4-oxo-2-phenyl-4H-1-benzopyran (Intermediate XXXVI)

A solution of 7.6 ml of 3-aminopropanol in 50 ml of water was added dropwise over a period of 30 minutes to a suspension of 30 g of 3-methyl-4-oxo-2-phenyl-4H-1-benzopyran-8-carbonyl chloride and 15.2 g of potassium carbonate in 400 ml of acetone. The thick suspension was stirred for 3 hours at 20-25°C. The solvents were removed in vacuo and the residue was taken up in 300 ml of water. After stirring for 1 hour, the precipitate was collected by suction filtration and washed with water. The crude product was purified by crystallization from 95% ethanol and 23.8 g of the tile compound were obtained, m.p. 191-193°C. An additional 4.7 g of the title compound was obtained by concentration in vacuo of the crystallization filtrate.

8-(3-chloropropylcarbamoyl)-3-methyl-4-oxo-2-phenyl-4H-1-benzopyran (Intermediate XXXVII)

A solution of 1.1 ml of thionyl chloride in 2 ml of chloroform was added to a boiling solution of 3.37 g of Intermediate XXXVI in 20 ml of chloroform. After stirring for 90 minutes under reflux, the solvent was removed in vacuo and the residue was crystallized from acetonitrile to give 3 g of pure title compound, m.p. (188) 193-194°C.

8-[1-hydroxy-4-(4-methylphenylsulphonyloxy)-butyl]-3-methyl-4-oxo-2-phenyl-4H-1-benzopyran (Intermediate XXXVIII)

1.12 g of sodium cyanide in 3 ml of water was added at 20-25°C to a stirred mixture of 3.96 g of 8-formyl-3-methyl-4-oxo-2-phenyl-4H-1-benzopyran, 2.61 g of morpholine and 4.48 g of p-toluenesulphonic acid in 20 ml of tetrahydrofuran and 30 ml of 1,2-dichloroethane. The reaction mixture was refluxed for 4 hours, and then 10 ml of cold water was added. The tetrahydrofuran was distilled off at normal pressure, and 10 ml of 1,2-dichloromethane and 10 ml of chloroform were added. The organic phase was separated, washed with aqueous sodium chloride solution, dried over anhydrous sodium sulphate and evaporated to dryness in vacuo. The residue was suspended in diethyl ether, filtered off, and crystallized from chloroform:ethyl acetate. Yield: 3.55 g of 8-(morpholino-cyanomethyl)-3-methyl-4-oxo-2-phenyl-4H-1-benzopyran, m.p. 236-238°C.

3.5 ml of a 30% solution of potassium hydroxide in anhydrous methanol was added under stirring at ambient temperature to a suspension of 22.8 g of the compound thus prepared in 520 ml of anhydrous tetrahydrofuran.

6.3 ml of acrylonitrile in 20 ml of tetrahydrofuran was dropped into this suspension, and the reaction mixture was stirred at ambient temperature for 1 hour. The solvents were evaporated off in vacuo. Crystallization of the residue from methanol gave 23.22 g of 8-(1,3-dicyano-1-morpholino-propyl)-3-methyl-4-oxo-2-phenyl-4H-1-benzopyran.

23.2 g of the compound thus prepared was dissolved in 250 ml of dioxane. 250 ml of 6M hydrochloric acid was added and the mixture was refluxed for 2½ hours. After cooling to ambient temperature, the mixture was poured into 700 ml of aqueous sodium chloride solution and extracted with ethyl acetate. The extracts were washed with aqueous sodium chloride solution and treated with 700 ml of 1M sodium hydroxide solution. The aqueous layer was washed with ethyl acetate and acidified with 37% hydrochloric acid. The precipitate was collected by suction filtration and crystallized from ethanol to give 10.2 g of 8-(3-carboxy-1-oxopropyl)-3-methyl-4-oxo-2-phenyl-4H-1-benzopyran, m.p. 191-192°C.

Diborane, generated by dropping a solution of 2.1 ml of freshly distilled boron trifluoride diethyl etherate in 10 ml of anhydrous diglyme into 19 ml of a 0.66 M solution of sodium borohydride in diglyme, was bubbled into a suspension of 2.28 g of the compound thus prepared in 23 ml of anhydrous tetrahydrofuran, stirred at 0°C under nitrogen flux. Stirring was continued for 20 minutes at 0°C and for a further 20 minutes at ambient temperature. Methanol was cautiously dropped into the mixture at 0°C to quench the reaction. The solvents were removed by evaporation in vacuo. The residue was purified by flash chromatography on silica gel, eluting with petroleum ether:ethyl acetate 3:7. The collected fractions were evaporated in vacuo to leave 2 g of 8-(1,4-dihydroxybutyl)-3-methyl-4-oxo-2-phenyl-4H-1-benzopyran, m.p. 133-134°C.

2.8 g of p-toluenesulphonyl chloride was added at 0°C to a stirred solution of 3.17 g of the compound thus prepared in 32 ml of anhydrous pyridine. The mixture was stirred for 6 hours at 0°C and stood overnight at -4°C without stirring. It was then poured into 200 ml of aqueous sodium chloride solution, acidified with 10 ml of 12N hydrochloric acid and filtered under suction. The filter cake was dissolved in chloroform, and the solution was washed with aqueous sodium chloride solution and dried on anhydrous sodium sulphate. The solvent was distilled off in a rotary evaporator. The residue was purified by flash chromotography on silica gel, eluting with petroleum ether:ethyl acetate 1:1. The collected fractions were evaporated to dryness in vacuo, yielding 3.04 g of pure title product, m.p. 123-124°C.

<u>4-[4-(2-methoxyphenyl)-1-piperazinyl]-butyraldehyde (Intermediate XXXIX)</u>

A solution of 5.4 g of 2-(3-chloropropyl)-dioxolan and 15.9 g of 1-(2-methoxyphenyl)-piperazine in 60 ml of dimethylformamide was stirred at 80°C for 4 hours. After cooling to 20-25°C, the reaction mixture was poured into 500 ml of ice cold 0.5N sodium hydroxide solution and extracted with dichloromethane. The organic phase was washed with water and dried on anhydrous sodium sulphate. The solvent was removed in vacuo, and the residue was purified by flash chromatography on silica gel, eluting with dichloromethane:ethanol 95:5. 9.8 g of 2-{3-[4-(2-methoxyphenyl)-1-piperazinyl]-propyl}-dioxolan was obtained as an oil.

**NMR $CDCl_3$ (δ)**

1.5-2.0 (4H, m, $C\underline{H}_2C\underline{H}_2CH$)
2.2-3.2 (10H, m, 5 x $C\underline{H}_2N$)
3.7-4.0 (7H, m, $OCH_3$ and 2 x $OCH_2$)
4.8 (1H, t, OCHO)
6.7-6.9 (4H, m, aromatic protons)

A solution of 12.8 g of the compound thus prepared in 200 ml of tetrahydrofuran and 420 ml of 1N hydrochloric acid was maintained at 20-25°C for 24 hours. It was then made alkaline with 5N sodium hydroxide solution and immediately extracted with dichloromethane. The organic layer was washed with water and dried on anhydrous sodium sulphate. The solvent was evaporated off in vacuo, and the residue was purified by flash chromatography on silica gel, eluting with dichloromethane:methanol 97:3. 6.4 g of the title compound was obtained as an oil.

**NMR $CDCl_3$ (δ)**

1.5-2.0 (2H, m, $CH_2C\underline{H}_2CH_2$)
2.2-2.8 (8H, m, 3 x $C\underline{H}_2N$ and $C\underline{H}_2CHO$)
2.9-3.2 (4H, m, 2 x $CH_2NAr$)
3.8 (3H, s, $OCH_3$)
6.8 (4H, s, aromatic protons)

9.3 (1H, s, CHO).

8-(2,3-epoxypropoxy)-3-methyl-4-oxo-2-phenyl-4H-1-benzopyran (Intermediate XL)

7 ml of 2,3-epoxypropyl chloride was added dropwise at 20-25°C to a stirred mixture of 5 g of 8-hydroxy-3-methyl-4-oxo-2-phenyl-4H-1-benzopyran and 9.7 ml of 2N sodium hydroxide in 10 ml of ethanol. After 6 hours at 20-25°C, the reaction mixture was poured into 100 ml of water and the precipitate which formed was collected by suction filtration. After drying and purifying by flash chromatography on silica gel (eluant petroleum ether: ethyl acetate 65:35), there was obtained 4.45 g of the title compound, m.p. 128-129°C.

8-[N-methyl-2-(4-methylphenylsulphonyloxy)-ethylsulphamoyl]-3-methyl-4-oxo-2-phenyl-4H-1-benzopyran (Intermediate XLI)

A solution of 5 g of Intermediate VIII in 60 ml of dichloromethane and 20 ml of tetrahydrofuran was added dropwise at 0°C to a mixture of 2.5 ml of 2-methylaminoethanol and 2.1 ml of triethylamine in 20 ml of dichloromethane. After stirring for 2 hours at 20-25°C, 100 ml of water and 100 ml of dichlormethane were added to the reaction mixture. The phases were separated and the organic solution was dried on anhydrous sodium sulphate. The solvent was removed in vacuo, and the residue was purified by column chromatography on silica gel, eluting with petroleum ether:ethyl acetate 3:7. There was thus obtained 4.5 g of 8-(N-methyl-2-hydroxyethylsulphamoyl)-3-methyl-4-oxo-2-phenyl-4H-1-benzopyran, melting at 146-147°C after crystallization from ethanol.

The compound thus prepared was converted to the title compound by p-toluenesulphonylation according to the second step of the procedure described below for the preparation of Intermediate XLII. The title compound was used without further purification.

8-[2-(4-methylphenylsulphonyloxy)-ethylsulphamoyl]-3-methyl-4-oxo-2-phenyl-4H-1-benzopyran (Intermediate XLII)

A solution of 5 g of Intermediate VIII in 37 ml of tetrahydrofuran was added dropwise at 0°C to a mixture of 2.5 ml of ethanolamine and 2.5 ml of triethylamine in 25 ml of tetrahydrofuran. After stirring at 20-25°C, the reaction mixture was poured into 400 ml of water. A precipitate formed, and was collected by suction filtration, washed with water and air dried, yielding 4.6 g of 8-(2-hydroxyethylsulphamoyl)-3-methyl-4-oxo-2-phenyl-4H-1-benzopyran, melting at 186-187°C after crystallization from ethyl acetate.

2.1 g of p-toluenesulphonyl chloride was added portionwise at 0°C to a solution of 3.6 g of the compound thus prepared in 25 ml of pyridine. After 6 hours at 20-25°C, the reaction mixture was slowly poured on to crushed ice containing a slight excess of hydrochloric acid. A precipitate formed and was collected by suction filtration and washed with water. 4.9 g of the title compound was obtained, melting at (163) 166-169°C after crystallization from ethyl acetate.

8-(3-aminopropylcarbamoyl)-3-methyl-4-oxo-2-phenyl-4H-1-benzopyran hydrochloride (Intermediate XLIII)

A solution of 21.6 g of 3-methyl-4-oxo-2-phenyl-4H-1-benzopyran-8-carbonyl chloride in 250 ml of anhydrous tetrahydrofuran was dropped at 0-10°C into a stirred solution of 17 g of 3-(2-methyl-2-propoxycarbamoyl)-propylamine (prepared as described in Saari, W.S. et al., *J. Med. Chem.*, 33, 97, 1990) and 13 ml of triethylamine. After stirring for 2 hours at ambient temperature, the reaction mixture was poured into water and filtered to recover 12.3 g of 3-(2-methyl-2-propoxycarbamoyl)-propyl 3-methyl-4-oxo-2-phenyl-4H-1-benzopyran-8-carboxamide which was recrystallized from ethanol. M.p. 178-180°C.

A solution of 4.3 ml of trifluoroacetic acid in 15 ml of anhydrous dichloromethane was added dropwise at -5°C under stirring to a solution of 3.3 g of the compound thus prepared in 35 ml of anhydrous dichloromethane. After warming to ambient temperature, the mixture was stirred for 8 hours. The dichloromethane and the excess trifluoroacetic acid were evaporated off at 20-25°C using a rotary evaporator. The oily residue was dissolved in dichloromethane and 1N sodium hydroxide solution was added. The organic layer was washed with water, dried on anhydrous sodium sulphate and filtered. Excess ethanolic hydrogen chloride was added to the filtrate, and the solvent was removed in vacuo. The residue was crystallized from ethanol to give 1.5 g of the title compound, m.p. 253-255°C.

8-(2-chloroethylureido)-3-methyl-4-oxo-2-phenyl-4H-1-benzopyran (Intermediate XLIV)

4 ml of 2-chloroethyl isocyanate were added, under stirring at ambient temperature, to a solution of 3.9 g of 8-amino-3-methyl-4-oxo-2-phenyl-4H-1-benzopyran in 52 ml of anhydrous dimethylformamide. Stirring was continued at 70°C for 5 hours. Water was added to the reaction mixture, which was then extracted with ethyl acetate. The organic phase was evaporated to dryness in vacuo. The residue was suspended in diethyl ether under stirring. The title product was then filtered off and recrystallized from methanol. Yield 3.74 g, m.p. 213-214°C.

(Z,E)-8-{4-[2-(1,3-dioxanyl)]-1-butenyl}-3-methyl-4-oxo-2-phenyl-4H-1-benzopyran (Intermediate XLV)

1.6 ml of 2.5N butyllithium in hexane was added dropwise at -20°C to a solution of 1.53 g of 2-[2-(1,3-dioxanyl)]-ethyl triphenylphosphonium bromide in 10 ml of anhydrous tetrahydrofuran. The mixture was stirred for 20 minutes at -20°C. A solution of 0.8 g of 8-formyl-3-methyl-4-oxo-2-phenyl-4H-1-benzopyran in 11 ml of anhydrous tetrahydrofuran was dropped into the mixture, which was then warmed to 0°C over a period of 90 minutes and then to ambient temperature over a period of 30 minutes. The reaction was quenched by addition of methanol. The solvents were evaporated off in vacuo and the residue was purified by flash chromatography on silica gel, eluting with ethyl acetate:petroleum ether 3:7, to give the title compound, as a mixture of diastereoisomers E and Z, m.p. (93) 98-100°C. The ratio of the two isomers was determined by NMR spectroscopy and resulted in E:Z = 65:35.

**NMR $CDCl_3$ (δ)**

| | |
|---|---|
| 8.1-8.2 | (m, 1H, CH in position 5 of the benzopyran ring) |
| 7.2-7.8 | (m, 7H, other aromatic CH groups of the benzopyran and phenyl rings) |
| 6.9 | (dt, 1H, Fl-CH of the E isomer) |
| 6.8 | (dt, 1H, Fl-CH of the Z isomer) |
| 6.4 | (dt, 1H, Fl-CH=C$\underline{H}$ of the E isomer) |
| 5.9 | (dt, 1H, Fl-CH=C$\underline{H}$ of the Z isomer) |
| 4.6-4.7 | (m, 1H, OCHO) |
| 3.6-4.2 | (m, 4H, $OCH_2O$ of the dioxane ring) |
| 2.4-2.7 | (m, 2H, $CH\underline{CH_2}CH$) |
| 1.9-2.3 | (m, 5H, $CH_3$ and $CH_2$ in position 5 of the dioxane ring) |

8-{4-[2-(1,3-dioxanyl)]-butyl}-3-methyl-4-oxo-2-phenyl-4H-1-benzopyran (Intermediate XLVI)

A mixture of 0.2 g of 10% palladium-on-carbon catalyst and of 1 g of Intermediate XLV in 24 ml of methanol was hydrogenated in a Parr apparatus at ambient temperature with a hydrogen pressure of 1.5 atmospheres. After the theoretical hydrogen consumption, the catalyst was filtered off and the solvent was removed by evaporation in vacuo. The residue was crystallized from cyclohexane to give the title compound, m.p. 118-119.5°C.

8-carboxymethyl-3-methyl-4-oxo-2-phenyl-4H-1-benzopyran (Intermediate XLVII)

4.5 g of potassium permanganate was added, portionwise over a period of 1½ hours under stirring at 0-10°C, to a mixture of 2.76 g of 8-allyl-3-methyl-4-oxo-2-phenyl-4H-1-benzopyran (P. Da Re, US 3350411), 0.17 g of Aliquat 336, 1.12 ml of acetic acid, 56 ml of dichloromethane, 3.2 ml of sulphuric acid (d=1.84) and 60 ml of water. Stirring was continued at ambient temperature for 5 hours. 3.4 g of sodium metabisulphite were added portionwise at 0-5°C within 15 minutes. The organic layer was separated, washed with water and extracted with 60 ml of 1N aqueous sodium hydroxide solution. The aqueous phase was acidified by addition of dilute hydrochloric acid and extracted with ethyl acetate. The organic phase was washed with water, dried on anhydrous sodium sulphate and, after filtration, evaporated to dryness in vacuo. The residue was treated with carbon tetrachloride and the solid was collected by suction filtration to give 1 g of the title compound, m.p. 191-192°C (acetonitrile).

8-(4-chlorobutyramido)-3-methyl-4-oxo-2-phenyl-4H-1-benzopyran (Intermediate XLVIII)

The title compound was prepared in the same manner as Intermediate X, but using 4-chlorobutyryl chloride instead of acryloyl chloride. The solid obtained, filtered from water and dried, was rinsed with hot diethyl ether

and collected by suction to give the title compound. A sample, crystallized from 50% aqueous ethanol and washed with diethyl ether, melted at (153) 162-164°C.

8-methylamino-3-methyl-4-oxo-2-phenyl-4H-1-benzopyran (Intermediate XLIX)

A solution of 0.5 g of Intermediate XXIII in 1.5 ml of anhydrous dimethylformamide was added dropwise under stirring, at -5°C, to a suspension of 0.045 g of sodium hydride (80% in mineral oil). After stirring at room temperature for 1 hour, 0.092 ml of methyl iodide in 0.6 ml of anhydrous dimethylformamide was added dropwise. Then the reaction mixture was stirred at 50°C for 1 hour, cooled to 20°C, poured into water, filtered by suction and dried at 60°C for 3 hours to recover 0.6 g of 8-(N-methyltrifluoroacetamido)-3-methyl-4-oxo-2-phenyl-4H-1-benzopyran.

**NMR ($CDCl_3$, $\delta$)**

| | |
|---|---|
| 8.15 | (dd, 1H, benzopyran CH in 5) |
| 7.10-7.60 | (m, 7H, other benzopyran and phenyl CHs) |
| 3.30 | (s, 3H, $CH_3$-N) |
| 2.10 | (s, 3H, benzopyran $CH_3$ in 3) |

A mixture of 0.44 g of the above compound and 0.05 g of sodium borohydride in 4 ml of ethanol and 1 ml of dimethylsulphoxide was stirred at room temperature for 1 hour, and then quenched with an excess of 4 N hydrochloric acid. After removal of ethanol by evaporation in vacuo, the residue was rinsed with water, then with 3N sodium hydroxide and extracted with ethyl acetate. The organic layer was washed with water, dried on anhydrous sodium sulphate and evaporated to dryness in vacuo. The solid residue was crystallized from ethanol to give 0.22 g of the title compound, melting at 143-146°C.

8-(N-methylacrylamido)-3-methyl-4-oxo-2-phenyl-4H-1-benzopyran (Intermediate L)

This compound was prepared in the same manner as Intermediate X, but using Intermediate XLIX instead of 8-amino-3-methyl-4-oxo-2-phenyl-4H-1-benzopyran. Instead of diluting with water, THF was removed by evaporation in vacuo and the crude residue was dissolved in ethyl acetate and washed with water. The organic solution was dried on anhydrous sodium sulphate and evaporated to dryness in vacuo to give the title compound. A sample, purified by column chromatography on silica gel (eluting with ethyl acetate:petroleum ether 4:6) and crystallized from cyclohexane, melted at 136-137°C.

1-[2-(1,3-dihydro-1,3-dioxo-2H-isoindol-2-yloxy)-ethyl]-4-(2-methoxyphenyl)-piperazine (Intermediate LI)

A mixture of 6.73 g of N-hydroxyphthalimide, 3.73 g of sodium acetate and 10 g of 1-(2-chloroethyl)-4-(2-methoxyphenyl)-piperazine in 100 ml of anhydrous dimethylsulphoxide was stirred at 100°C for 4 hours. The reaction mixture was then cooled to room temperature, poured into water and extracted with ethyl acetate. The collected organic layers were washed with 1N sodium hydroxide, dried on anhydrous sodium sulphate and evaporated to dryness in vacuo to give 7.58 g of the title compound. A sample was crystallized from cyclohexane and found to melt at (76) 80-83°C.

1-(2-aminooxyethyl)-4-(2-methoxyphenyl)-piperazine hydrochloride (Intermediate LII)

A solution of 6.59 g of Intermediate LI and 1.10 ml of 85% hydrazine hydrate in 130 ml of 95% ethanol was refluxed for 4 hours. Ethanol was removed by evaporation in vacuo. The residue was rinsed with water and then with an excess of 37% hydrochloric acid and filtered. The acid aqueous solution was made basic with 5% sodium hydroxide and extracted with chloroform. The organic layer was dried on anhydrous sodium sulphate and evaporated to dryness in vacuo to give 4.3 g of the title compound as an oil. A sample was converted to the hydrochloride by salification with ethanolic hydrogen chloride in dichoromethane. The solvents were removed by evaporation in vacuo and the crude residue was crystallized from ethanol, giving the title compound, m.p. 208-209°C.

8-(4-chlorobutylthio)-3-methyl-4-oxo-2-phenyl-4H-1-benzopyran (Intermediate LIII)

The title compound was prepared in the same manner as Intermediate XXXIV, but using 1-bromo-4-chlorobutane instead of 1-bromo-3-chloropropane. M.p. 81-84°C (ethanol).

8-(4-chlorobutylsulphinyl)-3-methyl-4-oxo-2-phenyl-4H-1-benzopyran (Intermediate LIV)

The title compound was prepared in the same manner as Intermediate XXVI, but using Intermediate LIII instead of Intermediate XXI. A sample, crystallized from cyclohexane:benzene 0.5:1, melted at 124-125°C.

8-carboxy-4-oxo-3-phenyl-4H-1-benzopyran (Intermediate LV)

A solution of 38.22 g of silver nitrate in 75 ml of water was added dropwise, under stirring, at 20-25°C, to a solution of 22.5 g of 8-formyl-4-oxo-3-phenyl-4H-1-benzopyran (prepared as described by G. Atassi et al., *Eur. J. Med. Chem. - Chim. Ter.*, 20, 393 (1985)) in 150 ml of 85% ethanol and 450 ml of dimethylformamide. Then, a solution of 32.67 g of 85% potassium hydroxide in 195 ml of water was added dropwise under stirring at 15-20°C. After additional stirring at room temperature, the reaction mixture was filtered by suction; the mother liquor was acidified with 37% hydrochloric acid and diluted with 1.2 litres of water. Filtration by suction and washing with water to neutrality gave the title compound as a crude product. This was suspended in 150 ml of ethyl acetate and stirred with 444 ml of 0.3 M sodium hydrogen carbonate until clear layers were obtained. The aqueous layer was washed with 75 ml of ethyl acetate, then made acidic with 37% hydrochloric acid, filtered and dried at 60-65°C to give 19.12 g of the title compound, melting at (215) 218°C. A sample, crystallized from ethanol, had the same melting point.

8-chlorocarbonyl-4-oxo-3-phenyl-4H-1-benzopyran (Intermediate LVI)

A mixture of 15.97 g of Intermediate LV and 15.6 ml of thionyl chloride in 75 ml of anhydrous toluene was stirred at 80-85 °C for 4 hours. After removal of the solvent under vacuo, the residue was rinsed twice with 20 ml of toluene and evaporating to dryness in vacuo to give, after drying, 16 g of the title compound melting at (126) 138-140°C which was used without further purification. M.p. (130) 138-140°C (toluene).

8-(N-acetylcarbamoyl)-3-methyl-4-oxo-2-phenyl-4H-1-benzopyran (Intermediate LVII)

A mixture of 3.5g of 8-carbamoyl-3-methyl-4-oxo-2-phenyl-4H-1-benzopyran (described in JP 61-238783), 4.8 ml of acetic anhydride and 0.25 ml of sulphuric acid (d=1.098) was stirred at 140°C for 3 minutes. The reaction was cooled to ambient temperature, diluted with water and filtered by suction to give, after washing with water and dessication, 3.88g of the title compound

**NMR ($CDCl_3$, $\delta$)**

| | |
|---|---|
| 10.50 | (bs, 1H, imidic NH) |
| 8.35-8.70 | (m, 2H, CH in position 5 and 7 of the benzopyran ring) |
| 7.45-8.00 | (m, 6H, other aromatic CHs) |
| 2.60 | (s, 3H, $CH_3CO$) |
| 2.20 | (s, 3H, $CH_3$ in position 3 of the benzopyran ring) |

2-(2-methylthiophenoxy)-acetaldehyde diethyl acetal (Intermediate LVIII)

A mixture of 15.2 ml of 97% 2-bromoacetaldehyde diethyl acetal, 14 g of 2-(methylthio)-phenol, 13.7 g of anhydrous potassium carbonate and 3.13 g of tricaprylmethylammonium chloride in 140 ml of anhydrous dimethylformamide was stirred at 95°C for 38 hours. At the end of this period, the reaction mixture was cooled to ambient temperature, poured into 1 litre of water and extracted with diethyl ether. The organic layer was washed with water, dried on anhydrous sodium sulphate and evaporated to dryness in vacuo. The oily residue was purified by column chromatography on silica gel eluting with petroleum ether:ethyl acetate 99:1. Evaporation in vacuo of the collected fractions yielded 12.9 g of pure title compound. A sample was crystallized from n-hexane and melted at 50-52°C.

2-(2-methylthiophenoxy)-acetaldehyde (Intermediate LIX)

A mixture of 10.5 g of Intermediate LVIII and 140 ml of 2N hydrochloric acid in 85 ml of anhydrous tetrahydrofuran was stirred at 50°C for 2 hours. The organic solvent was then removed by evaporation in vacuo, and the aqueous residue was extracted with ethyl acetate. The organic layer was washed with water, dried on anhydrous sodium sulphate and evaporated to dryness in vacuo giving 9.5 g of the title compound as a

solid which was used without further purification. A sample was crystallized from cyclohexane yielding the pure title compound, m.p. 102-104°C.

8-(4-chlorobutylsulphonyl)-3-methyl-4-oxo-2-phenyl-4H-1-benzopyran (Intermediate LX)

The title compound was prepared by the same method as Intermediate XXV, but using Intermediate LIII instead of Intermediate XXI. It was crystallized from diisopropyl ether and melted at 112-115°C.

8-ethoxycarbonyl-4-oxo-4H-1-benzopyran (Intermediate LXI)

4.35 g of sodium metal was added in pieces at ambient temperature to a solution of 9.85 g of ethyl 3-acetyl-2-hydroxy benzoate (synthesized from 3-acetyl-2-hydroxybenzoic acid (prepared as described in R.E. Ford, J. Med. Chem., 29, 538 (1986)) refluxing in 6N ethanolic hydrogen chloride for 1.5 hours, evaporating to dryness in vacuo and purifying the crude by column chromatography on silica gel (eluant ethyl acetate-petroleum ether 8:2) - m.p. 47°C (hexane)) in 98 ml of ethyl formate.

The reaction mixture went spontaneously to reflux for 20 minutes; then it was stirred at ambient temperature for 4 hours and the ethyl formate was removed by evaporation to dryness in vacuo. The crude solid obtained was rinsed with 120 ml of ethanol and 67 ml of 5.6M ethanolic hydrogen chloride. The mixture was stirred at reflux for 30 minutes; after this period it was cooled to ambient temperature and evaporated to dryness in vacuo. The residue was purified by column chromatography on silica gel eluting with ethyl acetate:petroleum ether 3:7 to 6:4 to give 8.31 g of the title compound. A sample crystallized from cyclohexane melted at 88-89°C.

8-carboxy-4-oxo-4H-1-benzopyran (Intermediate LXII)

30 ml of 6N hydrochloric acid was added to a solution of 4.0 g of Intermediate LXI in 30 ml of dioxane and the resulting mixture was stirred at reflux for 5 hours. The reaction mixture was cooled to ambient temperature and poured into 200 ml of water. After 12 hours at 0 to 5°C, the title compound was filtered off under suction. Washing with water and diethyl ether yielded, after desiccation, 2.8 g of the title compound, used without further purification. A sample washed with boiling acetonitrile:methanol 25:1, filtered and crystallized from acetic acid, melted at 253-254°C.

8-carboxy-6-hydroxy-3-methyl-4-oxo-2-phenyl-4H-1-benzopyran (Intermediate LXIII)

A mixture of 1.5 g of 8-carboxy-6-methoxy-3-methyl-4-oxo-2-phenyl-4H-1-benzopyran (prepared as described in JP 61-15880) and 28 ml of 57% hydroiodic acid in 47 ml of acetic acid was stirred at reflux for 18 hours. The reaction mixture was cooled to ambient temperature and poured into water; 1N sodium hydroxide was added to adjust the pH to 4 to 5. 2 g of sodium thiosulphate was added and stirring was continued for 15 minutes. After this period, the crude title compound was filtered off under suction and dissolved in 0.5M sodium hydroxide; the basic solution was washed with ethyl acetate and acidified to pH 1 by adding 37% hydrochloric acid. The title compound was collected under suction and desiccated. Yield: 1.12 g of the title compound, used without further purification and melting at 279-281°C after crystallization from 50% ethanol.

2-hydroxy-5-nitro-3-propionyl-benzoic acid (Intermediate LXIV)

97.1 g of 2-hydroxy-3-propionyl-benzoic acid, prepared as described in Brit 1, 343, 119 (1974), were added in 5 minutes to 500 ml of sulphuric acid (d=1.84) stirred at -25°C. A mixture of 40 ml of 65% nitric acid and 100 ml of sulphuric acid (d=1.84) was added in 40 minutes maintaining the temperature of the reaction mixture between -20 and -13°C. The mixture was stirred at -18°C for an additional 30 minutes. After this period, it was cautiously poured into a mixture of 2.0 kg of crushed ice and 500 ml of water, stirred for 10 minutes and filtered to give the title compound, after washing with water and drying at 50°C for 6 hours. Crystallization of this solid from 50% ethanol yielded 91.5 g of title compound melting at 186-189°C, used without further purification. A sample was recrystallized from 50% ethanol and melted at 189-191°C.

ethyl 2-hydroxy-5-nitro-3-propionyl-benzoate (Intermediate LXV)

A solution of 93.3 g of Intermediate LXIV and 25 ml of sulphuric acid (d=1.84) in 490 ml of ethanol was refluxed for 17 hours. After cooling to ambient temperature, 47.7 g of sodium carbonate was added portionwise and the ethanol was evaporated off in vacuo. The residue was rinsed with 1.2 litres of water, made alkaline

by adding 37% sodium hydroxide and stirred for 15 minutes. 37% hydrochloric acid was added to this suspension to adjust the pH to 6. Filtration yielded 85.4 g of the title compound used without further purification (m.p. 75-77°C). A sample was crystallized twice from ethanol and melted at 76-77°C.

8-ethoxycarbonyl-3-methyl-6-nitro-4-oxo-2-phenyl-4H-1-benzopyran (Intermediate LXVI)

A mixture of 48.1 g of Intermediate LXV, 63 ml of benzoyl chloride and 85.6 g of sodium benzoate was stirred at 180°C (bath temperature) for 8 hours. The pasty mixture was cooled to 60-70°C; 700 ml of 50% ethanol was added and the resulting mixture was stirred again at 50°C for 30 minutes. 60 ml of 35% sodium hydroxide was added at 5°C, ensuring that the temperature did not rise above 15°C. Filtration by suction, followed by washing with 50% ethanol and water afforded a crude product, which was purified by double passage on column chromatography on silica gel eluting first with dichloromethane:petroleum ether graduated 8:2 to 9:1, then with dichloromethane and finally with dichloromethane:ethyl acetate 95:5. Evaporation in vacuo of the collected fractions gave the title compound, which was washed with 140 ml of ethanol. Yield 43 g, m.p. 132-133°C (ethanol).

8-carboxy-3-methyl-6-nitro-4-oxo-2-phenyl-4H-1-benzopyran (Intermediate LXVII)

A mixture of 15.9 g of Intermediate LXVI and 48 ml of 1N sodium hydroxide in 320 ml of ethanol was stirred at reflux for 30 minutes. The organic solvent was removed by evaporation in vacuo and the resulting suspension was diluted with 200 ml of water and made acidic with 37% hydrochloric acid. Filtration and washing with diethyl ether yielded 11.1 g of the title compound melting at (258) 286-292°C and used without further purification. After crystallization from dimethylformamide:water 6:4 the title compound exhibited the same melting point.

8-chlorocarbonyl-3-methyl-6-nitro-4-oxo-2-phenyl-4H-1-benzopyran (Intermediate LXVIII)

A mixture of 6.2 g of Intermediate LXVII, 5.2 ml of thionyl chloride and 0.1 ml of anhydrous dimethylformamide in 60 ml of toluene was stirred at 90°C for 2 hours. Evaporation to dryness in vacuo and desiccation yielded 6.5 g of the title compound, m.p. 161-162°C, used without further purification. A sample was crystallized from toluene and had the same melting point.

8-carboxy-7-methoxy-3-methyl-4-oxo-2-phenyl-4H-1-benzopyran (Intermediate LXIX)

216 ml of a 0.3M solution of potassium permanganate in water was added dropwise over 40 minutes to a mixture of 7.94 g of 8-formyl-7-methoxy-3-methyl-4-oxo-2-phenyl-4H-1-benzopyran (prepared as described in Da Re et al., *J.Org.Chem.*, 25, 1097, 1960) and 54 ml of 5% sodium dihydrogen phosphate in 162 ml of t-butanol, stirred at 75°C. After a further 2½ hours stirring at the same temperature, the reaction mixture was cooled to ambient temperature and 81 ml of 1M sodium dithionite was dropped slowly therein. The mixture was extracted with ethyl acetate. The organic layer was washed four times with 160 ml of 0.5N sodium hydroxide; the collected basic aqueous layers were washed with diethyl ether and made acidic with 37% hydrochloric acid. The title compound precipitated. It was filtered off and washed with water, yielding after desiccation 3.3 g, used for further reactions without further purification and melting at 180-181°C after crystallization from 95% ethanol.

Ethyl 3-propionyl-2-(4-trifluoromethylbenzoyloxy)-benzoate (Intermediate LXX)

A solution of 6.7 g of 4-trifluoromethylbenzoyl chloride (prepared from the corresponding benzoic acid and thionyl chloride in benzene at reflux and used without purification) in 50 ml of chloroform was added dropwise to a solution of 7.13 g of ethyl 2-hydroxy-3-propionyl-benzoate and 4.9 ml of triethylamine in 50 ml of chloroform. The mixture was stirred at ambient temperature for 2 hours; the solvent was then evaporated off in vacuo and the residue was purified by column chromatography on silica gel eluting with petroleum ether:ethyl acetate 85:15. Evaporation in vacuo to dryness of the collected fractions yielded 7.4 g of the title compound as on oil.

**NMR spectrum at 60 MHz ($CDCl_3$, δ)**

| | |
|---|---|
| 7.6-8.5 | (m, 6H, aromatic CHs) |
| 7.5 | (t, 1H, phenol ring, CH in 5) |
| 4.2 | (q, 2H, $COOCH_2$) |

| | |
|---|---|
| 2.9 | (q, 2H, $COCH_2$) |
| 1-1.3 | (2t ,6H, $2xCH_3$) |

8-ethoxycarbonyl-3-methyl-4-oxo-2-(4-trifluoromethylphenyl)-4H-1-benzopyran (Intermediate LXXI)

A mixture of 6.96 g of Intermediate LXX and 2.58 g of potassium t-butoxide in 35 ml of pyridine was stirred at 100°C for 2 hours. After this period, the reaction mixture was cooled to ambient temperature, poured into a solution of 50 ml of acetic acid in 600 ml of water and extracted with ethyl acetate. The organic layer was washed with 10% hydrochloric acid and with water, dried on anhydrous sodium sulphate and evaporated to dryness in vacuo to give 6.9 g of 1-(2-hydroxy-3-ethoxycarbonyl)-2-methyl-3-(4-trifluoromethylphenyl)-1,3-propanedione. A solution of the compound thus prepared and 2.2 ml of 37% hydrochloric acid in 35 ml of glacial acetic acid was stirred at 100°C for 1½ hours. After cooling to ambient temperature, the mixture was poured into 630 ml of 1N sodium hydroxide and extracted with ethyl acetate. The organic layer was washed with water, dried on anhydrous sodium sulphate and evaporated to dryness in vacuo. The crude was purified by column chromatography on silica gel eluting with petroleum ether:ethyl acetate 85:15. Evaporation in vacuo to dryness yielded 2.95 g of the title compound, melting at 111-113 °C after crystallization from cyclohexane.

8-carboxy-3-methyl-4-oxo-2-(4-trifluoromethylphenyl)-4H-1-benzopyran (Intermediate LXXII)

A mixture of 2.95 g of Intermediate LXXI and 0.43 g of lithium hydroxide monohydrate in 12.5 ml of methanol and 12.5 ml of tetrahydrofuran containing 8 ml of water was stirred at ambient temperature for 1½ hours. The mixture was poured into a solution of 30 ml of 1N hydrochloric acid in 300 ml of water and filtered by suction to give 2.47 g of the title compound used without further purification. A sample was crystallized from 60% ethanol and melted at 253-254°C.

8-ethoxycarbonyl-2-(4-benzoylphenyl)-3-methyl-4-oxo-4H-1-benzopyran (Intermediate LXXIII)

The title compound was synthesized following the procedure of Intermediate LXX and LXXI in the established order but starting from 4-benzoylbenzoyl chloride, instead of 4-trifluoromethyl-benzoyl chloride and reacting this compound in 1,2-dicloro-ethane instead of chloroform and in the presence of 4-dimethylaminopyridine instead of triethylamine. After the usual workup, the residue was purified by column chromatography on silica gel eluting with dichloromethane:ethyl acetate 9:1. Evaporation in vacuo to dryness of the collected fractions yielded the title compound used without further purification. A sample was crystallized from cyclohexane and melted at 125-136°C (dec.).

8-carboxy-2-(4-benzoylphenyl)-3-methyl-4-oxo-4H-1-benzopyran (Intermediate LXXIV)

The title compound was prepared in the same manner as Intermediate LXXII but starting from Intermediate LXXIII instead of Intermediate LXXI. It was purified by dissolving the crude in 0.5M sodium hydroxide, washing the aqueous layer with ethyl acetate and precipitating the pure title compound by addition of 37% hydrochloric acid. A sample was crystallized from acetic acid and melted at 260-262°C.

ethyl 2-(4-phenoxybenzoyloxy)-3-propionyl-benzoate (Intermediate LXXV)

The title compound was prepared by the procedure described for Intermediate LXX, but starting from 4-phenoxybenzoyl chloride instead of 4-trifluoromethylbenzoyl chloride. Evaporation of the solvent yielded the pure title compound.

**NMR spectrum at 200 MHz ($CDCl_3$, $\delta$)**

| | |
|---|---|
| 8.17 | (dd, 3H, phenyl CHs in position ortho to the carboxylate groups) |
| 7.92 | (dd, 1H, phenyl CH in position ortho to the CO group) |
| 7.38-7.48 | (m, 3H, phenyl CHs in position meta to the carboxylate groups) |
| 7.25 | (d, 1H, CH in position 4 of the phenoxy ring) |
| 7.05; 7.10 | (2d, 4H, other CHs of the phenoxy ring) |
| 4.25 | (q, 2H, $CH_2O$) |
| 2.90 | (q, 2H, $CH_2CO$) |
| 1.05-1.20 | (m, 6H, $2xCH_3$) |

8-ethoxycarbonyl-3-methyl-4-oxo-2-(4-phenoxyphenyl)-4H-1-benzopyran (Intermediate LXXVI)

The title compound was prepared in the same manner as Intermediate LXXI, but starting from Intermediate LXXV instead of Intermediate LXX. Purification was by column chromatography on silica gel eluting with petroleum ether:ethyl acetate 6:4. Evaporation in vacuo yielded the pure title compound, m.p. 98-100°C.

8-carboxy-3-methyl-4-oxo-2-(4-phenoxyphenyl)-4H-1-benzopyran (Intermediate LXXVII)

This compound was obtained in the same manner as Intermediate LXXII, but starting from Intermediate LXXVI instead of Intermediate LXXI. M.p. 216-218°C.

8-carboxy-2-(t-butyl)-3-methyl-4-oxo-4H-1-benzopyran (Intermediate LXXVIII)

6 ml of pivaloyl chloride was added dropwise into a stirred solution of 8.9 g of ethyl 2-hydroxy-3-propionyl-benzoate in 20 ml of anhydrous pyridine. The reaction mixture was stirred at 80°C for 6 hours, cooled at ambient temperature and poured into a mixture of 200 g of crushed ice and 30 ml of 10N hydrochloric acid. After extraction with diethyl ether, the organic phase was washed with brine, dried on anhydrous sodium sulphate and evaporated to dryness in vacuo, yielding 11.4 g of crude ethyl 2-pivaloyloxy-3-propionyl-benzoate.

2.4 g of this compound were dissolved in 4 ml of anhydrous pyridine and 1 g of anhydrous potassium t-butoxide was added. The resultant mixture was heated for 15 minutes at 100°C, cooled to ambient temperature and poured into 50 g of iced water containing 8 ml of 10N hydrochloric acid. After extraction with diethyl ether, the organic phase was washed with brine, dried on anhydrous sodium sulphate and evaporated to dryness in vacuo, giving 2.1 g of crude ethyl 2-hydroxy-3-(2-pivaloylpropionyl)-benzoate, which was used without purification in the next step. 2 g of the compound thus prepared were heated at 100°C for 15 minutes after dissolution in a mixture containing 15 ml of acetic acid and 1.5 ml of 37% hydrochloric acid. After cooling to ambient temperature, the mixture was poured into 100 ml of water and extracted with diethyl ether. The organic phase was washed with 5% aqueous sodium hydrogen carbonate followed by water, dried on anhydrous sodium sulphate and evaporated in vacuo, yielding 1.6 g of crude 8-ethoxycarbonyl-2-(t-butyl)-3-methyl-4-oxo-4H-1-benzopyran.

1.5 g of the above ester was dissolved in 20 ml of methanol. 3 ml of 10N sodium hydroxide was slowly added, maintaining the temperature between 25 and 35°C. After 1½ hours at ambient temperature, the reaction mixture was diluted with 100 ml of water and extracted with ethyl acetate. The aqueous layer was acidified with 3N hydrochloric acid. The precipitate was collected by suction, washed with water and crystallized from ethanol, yielding 0.8 g of the title compound, melting at 225-228°C.

8-carboxy-2-cyclohexyl-3-methyl-4-oxo-4H-1-benzopyran (Intermediate LXXIX)

This compound was prepared according to the reaction sequence and methods described for Intermediate LXXVIII, but starting from cyclohexylcarboxylic acid chloride instead of pivaloyl chloride and with other minor differences. In particular, ethyl 2-cyclohexylcarbonyloxy-3-propionyl-benzoate was obtained after 8 hours stirring in pyridine at ambient temperature and transposed to ethyl 2-hydroxy-3-(2-cyclohexylcarbonyl-propionyl)-benzoate upon heating with potassium t-butoxide for 2½ hours at 100°C. Cyclization to 8-ethoxycarbonyl-2-cyclohexyl-3-methyl-4-oxo-4H-1-benzopyran was carried out by heating in a mixture of acetic and hydrochloric acids at 100°C for 1½ hours and the hydrolysis to the title compound was performed in 20 minutes at ambient temperature. The title compound melted at 224°C after crystallization from 40% ethanol.

8-ethoxycarbonyl-2-(2-furyl)-3-methyl-4-oxo-4H-1-benzopyran (Intermediate LXXX)

A mixture of 3.2 g of Intermediate XC and 1.3 g of anhydrous potassium t-butoxide in 8 ml of anhydrous pyridine was stirred at 60°C for 15 minutes, cooled to ambient temperature and poured into 60 ml of iced water containing 15 ml of 10N hydrochloric acid. After extraction with ethyl acetate, the organic phase was washed with 5% aqueous sodium bicarbonate and water and dried on anhydrous sodium sulphate. Upon evaporation in vacuo 2.5 g of crude ethyl 3-(2-furoyl-propionyl)-2-hydroxy-benzoate was obtained.

2.5 g of the compound so obtained was stirred at 100°C for 30 minutes with 10 ml of acetic acid and 0.7 ml of 37% hydrochloric acid. After cooling to ambient temperature, the mixture was poured into 180 ml of water. The title compound precipitated and was collected by suction filtration, washed with water and crystallized from isopropanol. Yield 1.5 g, m.p. 137-139°C.

8-carboxy-2-(2-furyl)-3-methyl-4-oxo-4H-1-benzopyran (Intermediate LXXXI)

A mixture of 3.5 g of Intermediate LXXX and 6 ml of 10N sodium hydroxide in 40 ml of methanol was stirred at ambient temperature for 1 hour and poured into 500 ml of water. After extraction with ethyl acetate, the aqueous layer was acidified with 3N hydrochloric acid. The title compound precipitated and was collected by suction filtration, washed with water and crystallized from a 7:3 mixture of methanol:chloroform. Yield 2.55 g, m.p. 272-277°C.

8-ethoxycarbonyl-3-methyl-4-oxo-2-(2-thienyl)-4H-1-benzopyran (Intermediate LXXXII)

This compound was prepared in two steps according to the methods reported for Intermediate LXXX, but using Intermediate XCI instead of Intermediate XC. The title compound melted at 116-118°C after crystallization from isopropanol.

8-carboxy-3-methyl-4-oxo-2-(2-thienyl)-4H-1-benzopyran (Intermediate LXXXIII)

This compound was prepared according to the method described for Intermediate LXXXI, but using Intermediate LXXXII instead of Intermediate LXXX. Melting point 287-294°C after crystallization from a 7:3 mixture of methanol and chloroform.

8-carboxy-4-oxo-2-phenyl-4H-1-benzothiopyran (Intermediate LXXXIV)

A mixture of 1 g of 8-methoxycarbonyl-4-oxo-2-phenyl-4H-1-benzothiopyran (Intermediate XCII), 2.2 ml of 12.5 N sodium hydroxide, 15 ml of methanol and 5 ml of dioxane was stirred at ambient temperature for 2½ hours. After evaporation in vacuo, water was added until complete solution and this solution was extracted with chloroform. The separated aqueous phase was acidified with dilute hydrochloric acid until complete precipitation of the crude, which was filtered off and purified by crystallization from acetic acid. Yield 0.62 g, m.p. 302°C.

(E)-8-ethoxycarbonyl-3-methyl-4-oxo-2-(2-styryl)-4H-1-benzopyran (Intermediate LXXXV)

This compound was prepared in three steps according to the methods described for Intermediate XC (first step) and Intermediate LXXX (second and third steps). In the first step, (E)-cinnamoyl chloride was used instead of 2-furoyl chloride and the obtained (E)-ethyl 2-hydroxy-3-(2-styryl-propionyl)-benzoate was used without purification for the second step. The title compound melted at 129-130°C after crystallization from isopropanol.

(E)-8-carboxy-3-methyl-4-oxo-2-styryl-4H-1-benzopyran (Intermediate LXXXVI)

This compound was prepared according to the method described for Intermediate LXXXI, but starting from Intermediate LXXXV instead of Intermediate LXXX, and maintaining the reaction at ambient temperature for 10 hours. The title compound melted at 284-286°C after crystallization from ethanol.

8-carboxy-3-methyl-2-(4-methylphenyl)-4-oxo-4H-1-benzopyran (Intermediate LXXXVII)

A mixture of 1.9 g of 2-hydroxy-3-propionyl-benzoic acid (prepared as described in Brit. 1, 343, 119, 1974), 5.2 g of anhydrous sodium 4-methylbenzoate and 3.9 ml of 4-methylbenzoyl chloride was stirred at 185-195°C for 8½ hours. After cooling to ambient temperature, the solidified mass was stood overnight with 100 ml of chloroform. The mixture was then shaken with 5% aqueous sodium carbonate solution, added until the pH of the aqueous phase reached 8.9. The organic phase was extracted again with 3% sodium carbonate and the aqueous phases were pooled, repeatedly extracted with diethyl ether and acidified with 10N hydrochloric acid. The precipitate was purified by flash chromatography on silica gel eluting with chloroform:methanol 100:2 to 100:20. The title compound, obtained by evaporating in vacuo the pooled fractions containing it, melted at 249-251°C, after crystallization from ethanol.

8-ethoxycarbonyl-2-(4-fluorophenyl)-3-methyl-4-oxo-4H-1-benzopyran (Intermediate LXXXVIII)

This compound was prepared in three steps according to the methods described for Intermediate XC (first step) and Intermediate LXXX (second and third steps). In the first step, 4-fluorobenzoyl chloride was used instead of 2-furoyl chloride and the reaction lasted 20 hours at ambient temperature, yielding ethyl 2-(4-fluorobenzoyloxy)-3-propionyl-benzoate. This compound was used without purification for the second step. The title compound melted at 128-130°C after rinsing with diethyl ether and crystallization from ethanol.

8-carboxy-2-(4-fluorophenyl)-3-methyl-4-oxo-4H-1-benzopyran (Intermediate LXXXIX)

A solution of 3.3 g of Intermediate LXXXVIII and 0.6 g of lithium hydroxide hydrate in 50 ml of tetrahydrofuran, 10 ml of methanol and 10 ml of water was maintained at ambient temperature for 5 hours and then poured into 300 ml of 1N hydrochloric acid. The precipitate formed was collected by suction, washed with water and dried, yielding 2.3 g of the title compound, melting at 249-250°C after crystallization from 95% ethanol.

ethyl 2-(2-furoyloxy)-3-propionyl-benzoate (Intermediate XC)

4.35 ml of 2-furoyl chloride were added dropwise at 10-15°C into a stirred mixture of 8.9 g of ethyl 2-hydroxy-3-propionyl-benzoate and 5.4 g of 4-dimethylaminopyridine in 25 ml of dichloromethane. After 2 hours at ambient temperature, the reaction was quenched with 200 ml of water. The organic layer was washed with 5% sodium bicarbonate, dried on anhydrous sodium sulphate and evaporated in vacuo. The residue was purified by flash chromatography on silica gel eluting with petroleum ether:ethyl acetate 4:1, obtaining 9.4 g of the title compound as a low melting solid, used without further purification in the next step.

**NMR Spectrum at 60 MHz ($CDCl_3$, $\delta$)**

| | |
|---|---|
| 8.2 | (1H, dd, CH in position 4 of the benzene ring) |
| 8.0 | (1H, dd, CH in position 6 of the benzene ring) |
| 7.7-7.8 | (1H, dd, CH in position 5 of the furan ring) |
| 7.43 | (1H, t, CH in position 5 of the benzene ring) |
| 7.45 | (1H, s, CH in position 3 of the furan ring) |
| 6.6-6.8 | (1H, m, CH in position 4 of the furan ring) |
| 4.3 | (2H, q, $COOC\underline{H}_2CH_3$) |
| 2.9 | (2H, q, $COC\underline{H}_2CH_3$) |
| 0.95-1.35 | (6H, m, $2xC\underline{H}_3$) |

ethyl 3-propionyl-2-(2-thienylcarbonyloxy)-benzoate (Intermediate XCI)

This compound was prepared by the method described for Intermediate XC, but using 2-thienylcarboxylic acid chloride instead of 2-furoyl chloride.

**NMR Spectrum at 60 MHz ($CDCl_3$, $\delta$)**

| | |
|---|---|
| 7.1-8.35 | (6H, m, aromatic CHs) |
| 4.25 | (2H, q, $COOC\underline{H}_2CH_3$) |
| 2.9 | (2H, q, $COC\underline{H}_2CH_3$) |
| 0.95-1.3 | (6H, m, $2xC\underline{H}_3$) |

8-methoxycarbonyl- 4-oxo-2-phenyl-4H-1-benzothiopyran (Intermediate XCII)

A mixture of 16.8 ml of methyl thiosalicylate, 25.6 ml of ethyl benzoylacetate and 360 g of polyphosphoric acid was stirred at 90°C for 3 hours. After cooling to ambient temperature, the mixture was poured into crushed ice and the crude was collected by filtration, washed with water and purified by crystallization from ethanol. M.p. 170-171°C.

**Elemental Analysis for $C_{17}H_{12}O_3S$:**

| | |
|---|---|
| Calculated (%) : | C, 68.90; H, 4.08; S, 10.82. |
| Found (%) : | C, 68.59; H, 4.13; S, 10.69. |

**NMR spectrum at 200 MHz ($CDCl_3$, δ)**

| | |
|---|---|
| 8.83-8.95 | (dd, 1H,benzothiopyran CH in 5) |
| 8.45-8.53 | (dd, 1H,benzothiopyran CH in 7) |
| 7.68-7.80 | (m, 2H, 2-phenyl CHs in 2 and 6) |
| 7.55-7.65 | (t, 1H, benzothiopyran CH in 6) |
| 7.45-7.55 | (m, 3H, 2-phenyl CHs in 3,4 and 5) |
| 7.24 | (s, 1H, benzothiopyran CH in 3) |
| 4.00 | (s, 3H, $COOCH_3$) |

8-ethoxycarbonyl-3-bromomethyl-4-oxo-2-phenyl-4H-1-benzopyran (Intermediate XCIII)

A mixture of 9.2 g of 8-ethoxycarbonyl-3-methyl-4-oxo-2-phenyl-4H-1-benzopyran (prepared as described by Da Re, P. et al., *J. Med. Pharm. Chem.*, 2, 263, 1960), 6.4 g of N-bromosuccinimide and 0.04 g of benzoylperoxide in 80 ml of anydrous carbon tetrachloride was stirred at reflux for 1½ hours. After cooling to ambient temperature the formed succinimide was collected by suction and washed with cold carbon tetrachloride. The mother liquors were evaporated to dryness in vacuo and the residue was rinsed with diethyl ether and collected by suction filtration yielding 9.2 g of the title compound, melting at 133-134°C after crystallization from acetone:n-hexane.

8-ethoxycarbonyl-3-acetoxymethyl-4-oxo-2-phenyl-4H-1-benzopyran (Intermediate XCIV)

A solution of 10.2 g of sodium acetate trihydrate in 30 ml of water was added dropwise at ambient temperature to a solution of 29 g of Intermediate XCIII in 300 ml of dimethylformamide. After stirring at 50°C for 1½ hours, the reaction mixture was poured into 2 litres of water and the precipitated title compound was collected by suction filtration and crystallized from acetone yielding 20 g (two crops collected), m.p. 151-152°C.

8-carboxy-3-hydroxymethyl-4-oxo-2-phenyl-4H-1-benzopyran (Intermediate XCV)

116 ml of 1N sodium hydroxide were added over 10 minutes to a stirred suspension of 14.8 g of Intermediate XCIV in 300 ml of 95% ethanol. The reaction mixture was then heated at 60-65°C for 15 minutes, obtaining a clear solution which was maintained at ambient temperature for a further hour. After evaporation in vacuo, the residue was dissolved in 200 ml of water and the solution acidified by slow addition of 10 ml of hydrochloric acid (d=1.18). After stirring for 1 hour at ambient temperature the title compound was collected by suction filtration, washed with water and crystallized from isopropanol, yielding 9.3 g, m.p. (225) 237-240°C.

ethyl 2-(4-nitrobenzoyloxy)-3-propionyl-benzoate (Intermediate XCVI)

The title compound was prepared according to the procedure described for Intermediate XC, but using 4-nitrobenzoyl chloride instead of 2-furoyl chloride. The product was obtained as a low melting solid (m.p. (40) 78-80°C).

**NMR Spectrum at 60 MHz ($CDCl_3$, δ)**

| | |
|---|---|
| 7.85-8.50 | (m, 6H, aromatic CHs) |
| 7.50 | (t, 1H, CHs in position 5 of the phenol ring) |
| 4.25 | (q, 2H, $CH_2O$) |
| 3.95 | (q, 2H, $CH_2$) |
| 0.95-1.30 | (m, 6H, $CH_3$) |

8-ethoxycarbonyl-3-methyl-2-(4-nitrophenyl)-4-oxo-4H-1-benzopyran (Intermediate XCVII)

A mixture of 29.7 g of Intermediate XCVI and 10.18 g of anhydrous potassium t-butoxide in 89 ml of anhydrous pyridine was stirred at 100°C for 13 hours. The reaction mixture was cooled to ambient temperature, poured into 400 ml of 4N hydrochloric acid and extracted with dichloromethane. The organic layer was washed repeatedly with water, then with 2,5% sodium hydrogen carbonate, dried on anhydrous sodium sulphate and evaporated to dryness in vacuo. The crude product was purified by column chromatography on silica gel eluting with hexane:ethyl acetate 7:3. Evaporation in vacuo of the collected fraction yielded 7 g of the title compound,

melting at (130) 145-148°C.

8-carboxy-3-methyl-2-(4-nitrophenyl)-4-oxo-4H-1-benzopyran (Intermediate XCVIII)

A suspension of 0.38 g of Intermediate XCVII in 4.75 ml of dioxane and 4.75 ml of methanol was stirred at 50°C. 1.29 ml of 1N sodium hydroxide was added and stirring was continued for 3 hours at the same temperature. The reaction mixture was cooled to ambient temperature and 3N hydrochloric acid was added until the pH was 1. The suspension was filtered by suction giving 0.13 g of the title compound, which melted at 320-321°C after crystallization from dioxane.

8-ethoxycarbonyl-3-methyl-4-oxo-2-trifluoromethyl-4H-1-benzopyran (Intermediate XCIX)

3.16 ml of 1,8-diazabicyclo[5.4.0]undec-7-ene was added dropwise at 0°C by a syringe to a stirred mixture of 3 g of ethyl 2-hydroxy-3-propionyl-benzoate and 5.53 ml of trifluoroacetic anhydride. The reaction mixture was stirred at 60°C for 4 hours; after this period it was cooled to ambient temperature and diluted with ethyl acetate and water. The organic layer was washed with 1N sodium hydroxide and water, dried on anhydrous sodium sulphate and evaporated to dryness in vacuo. The residue was purified by column chromatography on silica gel eluting with petroleum ether:ethyl acetate 95:5 yielding 0.8 g of the title compound.

**NMR spectrum at 200MHz ($CDCl_3$, $\delta$)**

| | |
|---|---|
| 8.41;8.37 | (2dd; 2H, CHs in position 5 and 7 of the benzopyran ring) |
| 7.51 | (t, 1H, CH in position 6 of the benzopyran ring) |
| 4.46 | (q, 2H, $COOCH_2$) |
| 2.22-2.27 | (m, 3H, $J_{H-F}$=2.16Hz, $CH_3$ in position 3 of the benzopyran ring) |
| 1.39 | (t, 3H, $CH_2C\underline{H}_3$) |

8-carboxy-3-methyl-4-oxo-2-trifluoromethyl-4H-1-benzopyran (Intermediate C)

The title compound was prepared by the same method as Intermediate LXII but using Intermediate XCIX instead of Intermediate LXI and, after dilution with water, extracting with ethyl acetate instead of filtering. After drying on anhydrous sodium sulphate and evaporating the organic layer to dryness in vacuo, the title compound was obtained as a solid which melted at 175-178°C.

3-[4-(2-methoxyphenyl)-1-piperazinyl]-N-methyl-propylamine (Intermediate CI)

42 ml of 35% aqueous methylamine was added to a solution of 8.2 g of 3-[4-(2-methoxyphenyl)-1-piperazinyl]-propyl chloride in 48 ml of dimethylformamide. The mixture was heated at 60°C in a closed vessel for 5 hours, and then cooled to 30°C. The solvent was evaporated off in vacuo, and the residue was stirred for 30 minutes with 100 ml of diethyl ether. The solids, collected by suction filtration, were dissolved in 200 ml of chloroform:5N methanolic ammonia 100:3. After 30 minutes stirring at ambient temperature, the solution was adsorbed on a chromatographic column, which was eluted with chloroform:5N methanolic ammonia graduated 100:5 to 100:15. The fractions containing the title compound were pooled and evaporated in vacuo, yielding 3 g of Intermediate CI as a thick oil.

**NMR spectrum at 60MHz (DMSO-$d_6$, $\delta$)**

| | |
|---|---|
| 6.80 | (s, 4H, aromatic CHs) |
| 3.75 | (s, 3H, $OCH_3$) |
| 3.20-2.75 | (m, 4H, piperazinic $CH_2$s at pos. 3,5) |
| 2.75-2.10 | (m, 8H, piperazinic $CH_2$s at pos. 2,6 and $C\underline{H}_2CH_2C\underline{H}_2$) |
| 2.40 | (s, 1H, NH) |
| 2.30 | (s, 3H, $NCH_3$) |
| 1.80-1.40 | (m, 2H, $CH_2C\underline{H}_2CH_2$) |

ethyl 2-benzoyl-3-ethylbenzo[b]furan-7-carboxylate (Intermediate CII)

A mixture of 11.1 g of ethyl 2-hydroxy-3-propionyl-benzoate, 9.9 g of phenacyl bromide, 6.9 g of anhydrous

potassium carbonate and 200 ml of acetone was stirred at refluxing temperature for 7 hours. After cooling to ambient temperature, the inorganic salts were separated by filtration and the solution was evaporated in vacuo. The residue was purified by flash chromatography on silica gel eluting with toluene. The title compound, obtained by evaporating in vacuo the pooled fractions containing it, was crystallized from 90% ethanol. Yield 9.8 g, m.p. 64-66°C.

7-carboxy-2-benzoyl-3-ethyl-benzo[b]furan (Intermediate CIII)

A mixture of 7 g of Intermediate CII, 275 ml of 0.95N sodium hydroxide and 400 ml of dioxane was stirred at refluxing temperature for 4 hours. After cooling to ambient temperature, the dioxane was evaporated in vacuo and replaced with the same volume of water. After filtering with charcoal, the solution was acidified with dilute hydrochloric acid and the precipitate was filtered and purified by crystallization from acetone.

Yield 4.94 g, m.p. 193-195°C.

8-methoxycarbonyl-3-methyl-2-(4-methylphenyl)-4-oxo-4H-1-benzopyran (Intermediate CIV)

This compound was prepared in three steps according to the methods described for Intermediate XC (first step) and Intermediate LXXX (second and third step). In the first step, 4-methylbenzoyl chloride was used instead of 2-furoyl chloride and methyl 2-hydroxy-3-propionyl-benzoate was used instead of ethyl 2-hydroxy-3-propionyl-benzoate. The reaction lasted 4 hours at ambient temperature, yielding methyl 2-(4-methylbenzoyloxy)-3-propionyl-benzoate. This compound was used without purification for the second step which lasted 1½ hours at 100°C. In the third step 96% sulphuric acid was used instead of 37% hydrochloric acid. The title compound melted at 174-175°C after crystallization from ethanol.

8-ethoxycarbonyl-2-(4-biphenylyl)-3-methyl-4-oxo-4H-1-benzopyran (Intermediate CV)

This compound was prepared in three steps according to the methods described for Intermediate XC (first step) and Intermediate CIV (second and third steps). In the first step 4-phenylbenzoyl chloride was used instead of 2-furoyl chloride and the reaction lasted 20 hours at room temperature and 13 hours at reflux. Purification was performed by column chromatography on silica gel eluting with petroleum ether:ethyl acetate graduated 100:5 to 100:10, yielding ethyl 2-(4-biphenylyl)3-propionyl-benzoate. The title compound melted at 165-167°C after rinsing with 95% ethanol.

8-carboxy-2-(4-biphenylyl)-3-methyl-4-oxo-4H-1-benzopyran (Intermediate CVI)

A mixture of 4.3 g of Intermediate CV and 35 ml of 35% hydrochloric acid in 50 ml of 1,4-dioxane and 15 ml of water was stirred at reflux for 16 hours. After cooling, the mixture was poured into 200 ml of water and extracted with ethyl acetate.

The organic layer was separated and extracted with 20% aqueous sodium carbonate. The precipitate formed after acidifying the aqueous layer with dilute hydrochloric acid was collected by suction, washed with water and dried, yielding 2.5 g of the title compound, melting at 242.5-244°C.

8-carboxy-2-(4-hydroxyphenyl)-3-methyl-4-oxo-4H-1-benzopyran (Intermediate CVII)

A mixture of 3 g of 8-ethoxycarbonyl-2-(4-methoxyphenyl)-3-methyl-4-oxo-4H-1-benzopyran (prepared as described in JP 58-225083) and 60 ml of 48% hydrobromic acid in 80 ml of acetic acid was stirred at reflux for 8 hours. After cooling, the mixture was poured into 500 ml of water and the precipitate was collected by suction and washed with water. The crude product was purified by flash chromatography eluting with chloroform: isopropanol graduated 9:1 to 7:3 followed by methanol elution, yielding 1 g the title compound, melting at 300°C.

4-[4-(2-methoxyphenyl)-1-piperazinyl]-N-methyl-butylamine (Intermediate CVIII)

A solution of 3.8 ml of trifluoroacetic anhydride in 25 ml of anhydrous dichloromethane was added dropwise under stirring at 0°C to a solution of 2.53 g of 4-[4-(2-methoxyphenyl)-1-piperazinyl]-butylamine in 25 ml of anhydrous dichloromethane. After 2 hours stirring at room temperature, the reaction mixture was diluted with dichloromethane and washed with water. The organic layer was dried on anhydrous sodium sulphate and evaporated to dryness in vacuo, yielding 3.3 g of pure 4-[4-(2-methoxyphenyl)-1-piperazinyl]-N-trifluoroacetyl-butylamine

**NMR spectrum at 60 MHz ($CDCl_3$, δ)**

| | |
|---|---|
| 7.70-8.00 | (bs, 1H, NH) |
| 6.80-7.20 | (m, 4H, aromatic CHs) |
| 3.85 | (s, 3H, $CH_3O$) |
| 2.90-3.80 | (m, 12H, piperazine $CH_2$s, $CH_2N$ and $\underline{CH_2}NHCO$) |
| 1.50-2.05 | (m, 4H, C-$CH_2CH_2$-C) |

0.88 g of 50% sodium hydride was added portionwise under stirring at 0°C to solution of 3.3 g of the above intermediate in 46 ml of anhydrous dimethylformamide. After 1 hour stirring at the same temperature, 0.57 ml of methyl iodide was added. The reaction mixture was stirred at 0°C for additional 1½ hours; then it was poured into water and extracted with ethyl acetate. The organic layer was washed with water, dried on anhydrous sodium sulphate and evaporated to dryness in vacuo, yielding 1.13 g of crude 4-[4-(2-methoxyphenyl)-1-piperazinyl]-N-trifluoroacetyl-N-methyl-butylamine used in the following step without further purification. 0.18 g of sodium borohydride was added to a solution of 1.13 g of this intermediate in 30 ml of ethanol and the resulting mixture was stirred at 60°C for 1 hour. After cooling to room temperature, the reaction mixture was poured into water, and extracted with dichloromethane. The organic layer was washed with water, dried on anhydrous sodium sulphate and evaporated to dryness in vacuo, yielding 0.82 g of pure title compound.

**NMR spectrum at 60 MHz ($CDCl_3$, δ)**

| | |
|---|---|
| 6.80-7.20 | (m, 4H, aromatic CHs) |
| 3.85 | (s, 3H, $CH_3O$) |
| 2.90-3.20 | (m, 4H, piperazine $CH_2$s, positions 3 and 5) |
| 2.30-2.80 | (m, 8H, piperazine $CH_2$s, positions 2 and 6; 2 $CH_2N$) |
| 2.50 | (s, 3H, $CH_3N$) |
| 1.80 | (s, 1H, NH) |
| 1.40-1.80 | (m, 4H, C-$CH_2$-$CH_2$-C) |

<u>1-(3-amino-2,2-dimethylpropyl)-4-(2-methoxyphenyl)-piperazine</u>

The title compound may be prepared by treating 1-(2-methoxyphenyl)-piperazine with isobutyraldehyde, 37% formaldehyde in water and acetic acid at 90-150°C or with the same in ethanolic hydrogen chloride (Mannich reaction) to give 1-(2-formyl-2-methylpropyl)-4-(2-methoxyphenyl)-piperazine, which is then aminated by treatment with excess ammonia under reducing conditions. The latter may be hydrogen and a catalyst (for example, palladium-on-charcoal, Raney nickel or platinum dioxide) in a solvent (for example, ethanol, methanol, isopropanol, dichloromethane, chloroform or dimethylformamide) at between ambient temperature and 80°C, or alternatively a metal hydride (for example, sodium borohydride, sodium or potassium cyanoborohydride, or sodium triacetoxyborohydride) in a solvent (for example, methanol, ethanol, chloroform, benzene or 1,2-dichloroethane) in the presence of an acid (for example, gaseous hydrogen chloride or acetic acid). It may be reacted with 8-chlorocarbonyl-3-methyl-4-oxo-2-phenyl-4H-1-benzopyran in the manner described in Example 12 hereinbelow to give 8-(2,2-dimethyl-3-[4-(2-methoxyphenyl)-1-piperazinyl]-propylcarbamoyl)-3-methyl-4-oxo-2-phenyl-4H-1-benzopyran.

<u>8-trifluroacetamidomethyl-3-methyl-4-oxo-2-phenyl-4H-1-benzopyran</u>

This compound can be prepared according to the procedure described for Intermediate XXIII but using Intermediate XXIV instead of 8-amino-3-methyl-4-oxo-2-phenyl-4H-1-benzopyran. It can be used as starting material, instead of Intermediate XXIII, in the reaction described in Example 32 to yield 8-{2-[4-(2-methoxyphenyl)-1-piperazinyl]-ethylaminomethyl}-3-methyl-4-oxo-2-phenyl-4H-1-benzopyran.

<u>8-(2-chloroethylureidomethyl)-3-methyl-4-oxo-2-phenyl-4H-1-benzopyran</u>

This intermediate can be prepared by the method described for the preparation of Intermediate XLIV, but using Intermediate XXIV instead of 8-amino-3-methyl-4-oxo-2-phenyl-4H-1-benzopyran. It can be reacted with a compound of formula H-B, according to Path (a) to give the desired final compounds.

8-ethenylsulphonylaminomethyl-3-methyl-4-oxo-2-phenyl-4H-1-benzopyran

This compound can be prepared by reacting Intermediate XXIV with 2-chloroethylsulphonyl chloride in a halogenated solvent such as dichloromethane in the presence of triethylamine at 0-40°C, according to A.A. Goldberg, *J. Chem. Soc.*, 464, 1945. It can be reacted with the appropriate compounds H-B, according to Path (m) to yield the corresponding final compounds.

Operating as described above, but starting from 8-amino-3-methyl-4-oxo-2-phenyl-4H-1-benzopyran the final compounds F1'-Y36-$(CH_2)_2$-B can be obtained.

8-chlorosulphonylmethyl-3-methyl-4-oxo-2-phenyl-4H-1-benzopyran

This intermediate can be prepared by reacting 8-amidinothiomethyl-3-methyl-4-oxo-2-phenyl-4H-1-benzopyran (whose synthesis is described in Intermediate XXI) with chlorine gas in water at -10 to +10°C according to T.B. Johnson et al., *J. Chem. Soc.*, 61, 2548, 1939. By reaction of this intermediate with the appropriate compounds A-NH-Z-B according to Path n, the desired final compounds can be obtained.

## EXAMPLES

### Example 1

**8-{2-[4-(2-methoxyphenyl)-1-piperazinyl]-1-oxoethyl}-3-methyl-4-oxo-2-phenyl-4H-1-benzopyran hydrochloride**

A solution of 11.5 g of 1-(2-methoxyphenyl)-piperazine in 30 ml of methanol was added dropwise at 20-25°C to a stirred mixture consisting of 21.4 g of Intermediate VI and 4.1 g of potassium carbonate in 120 ml of methanol. After 4 hours stirring at the same temperature, the reaction mixture was stripped in vacuo. The residue was extracted with chloroform and the organic solution was washed with water, dried on anhydrous sodium sulphate/calcium chloride, filtered and stripped in vacuo. The obtained crude product was dissolved in acetone and a slight excess of ethanolic hydrogen chloride was added. After collection by suction filtration and recrystallization from 95% ethanol, 16.3 g of the title compound was obtained, m.p. (189) 195-199°C.

### Example 2

**8-{2-[4-(2-methylphenyl)-1-piperazinyl]-1-oxoethyl}-3-methyl-4-oxo-2-phenyl-4H-1-benzopyran hydrochloride**

This compound was prepared according to Example 1, but using 1-(2-methylphenyl)-piperazine instead of 1-(2-methoxyphenyl)-piperazine and carrying out the reaction in dimethylformamide for 1 hour instead of in methanol for 4 hours.

M.p. (194) 203-206°C (isopropanol).

### Example 3

**8-{2-[4-(2-ethoxyphenyl)-1-piperazinyl]-1-oxoethyl}-3-methyl-4-oxo-2-phenyl-4H-1-benzopyran hydrochloride**

This compound was prepared according to Example 1 but using 1-(2-ethoxyphenyl)-piperazine instead of 1-(2-methoxyphenyl)-piperazine and carrying out the reaction in dimethylformamide for 2 hours instead of in methanol for 4 hours.

M.p. 208-210°C (isopropanol).

### Exemple 4

**8-{3-[4-(2-methoxyphenyl)-1-piperazinyl]-1-oxopropyl}-3-methyl-4-oxo-2-phenyl-4H-1-benzopyran hydrochloride**

A solution of 10 ml of 37% formaldehyde in 15 ml of methanol was dropped, over a period of 3 minutes at 0°C, into a solution of 5.75 g of 1-(2-methoxyphenyl)-piperazine in 10 ml of methanol. After 12 hours at 0°C, the mixture was stripped in vacuo and redissolved in 15 ml of methanol. 20 ml of 3.6N hydrogen chloride in diethyl ether was added at 0°C. After stripping in vacuo, the residue was suspended in 15 ml of 1,4-dioxane. A solution of 8.3 g of Intermediate V in 100 ml of 1,4-dioxane was added under stirring at 20-25°C. After stirring for 8 hours at reflux the reaction mixture was cooled to 30-40°C. 50 ml of methanol was added and the mixture

was refluxed for a further 2 hours. After cooling to 20-25°C, the resultant solution was diluted with 300 ml of diethyl ether. Stirring was continued for a further 3 hours at the same temperature. The title compound was collected by suction filtration and recrystallized from ethanol. Yield 4 g, m.p. 209-210°C.

Example 5

**8-{3-[4-(2-methoxyphenyl)-1-piperazinyl]-propoxycarbonyl}-3-methyl-4-oxo-2-phenyl-4H-1-benzopyran dihydrochloride**

A mixture of 4.24 g of 8-carboxy-3-methyl-4-oxo-2-phenyl-4H-1-benzopyran and 6.3 g of anhydrous potassium carbonate in 60 ml of dimethylformamide was stirred at 80°C for 30 minutes. 5.23 g of 1-(3-chloropropyl)-4-(2-methoxyphenyl)-piperazine was then added and stirring was continued at 80°C for 3½ hours. The reaction mixture was cooled to ambient temperature, poured on to iced water and extracted with ethyl acetate. The organic extracts were washed with aqueous sodium chloride solution, dried on anhydrous sodium sulphate, and evaporated to dryness in vacuo. The residue was taken up in ethanol and excess ethanolic hydrogen chloride was added to the solution. Yield: 8.16 g of the title compound, m.p. 198-203°C.

Example 6

**8-{2-[4-(2-methoxyphenyl)-1-piperazinyl]-ethoxycarbonyl}-3-methyl-4-oxo-2-phenyl-4H-1-benzopyran dihydrochloride**

Operating as described in Example 5, but using 1-(2-chloroethyl)-4-(2-methoxyphenyl)-piperazine instead of 1-(3-chloropropyl)-4-(2-methoxyphenyl)-piperazine, the title compound was obtained, m.p. 200-203°C from ethanol.

Example 7

**8-{3-[4-(2-chlorophenyl)-1-piperazinyl]-propoxycarbonyl}-3-methyl-4-oxo-2-phenyl-4H-1-benzopyran dihydrochloride**

A mixture of 2.8 g of 1-(2-chlorophenyl)-piperazine hydrochloride and 4.2 g of anhydrous potassium carbonate in 25 ml of dimethylformamide was stirred at ambient temperature for 15 minutes. 4.81 g of Intermediate I was added, and stirring was continued for 2 days. The reaction mixture was then poured into 200 ml of cold water, and extracted with diethyl ether and ethyl acetate. The organic extracts were washed in turn with aqueous sodium chloride solution, 0.1N acetic acid, aqueous sodium chloride solution, aqueous 4% sodium carbonate solution and water, and were then dried on anhydrous sodium sulphate. After evaporation to dryness in vacuo, the residue was dissolved in 160 ml of acetonitrile and excess hydrogen chloride in diethyl ether was added. The insoluble title compound was recrystallized from acetonitrile. Yield 3.6 g, m.p. 138-143°C.

Example 8

**8-[3-(4-phenyl-1-piperazinyl)-propoxycarbonyl]-3-methyl-4-oxo-2-phenyl-4H-1-benzopyran dihydrochloride**

The title compound was prepared by the method described in Example 7, but using 1-phenyl-piperazine in place of 1-(2-chlorophenyl)-piperazine hydrochloride. Recrystallization was from methanol; the melting point was 229-231°C.

Example 9

**8-{3-[4-(2-methoxyphenyl)-1-piperazinyl]-propoxycarbonyl}-3-methyl-4-oxo-2-phenyl-4H-1-benzopyran dihydrochloride**

Operating as described in Example 7, but using 1-(2-methoxyphenyl)-piperazine hydrochloride instead of 1-(2-chlorophenyl)-piperazine hydrochloride, the title compound was obtained. This represents an alternative route to the product of Example 5.

Example 10

**8-{3-[4-(2-methoxyphenyl)-1-piperazinyl]-2-methyl-2-propoxycarbonyl}-3-methyl-4-oxo-2-phenyl-4H-1-benzopyran dihydrochloride**

5.29 g of Intermediate XXVIII in 25 ml of 1,2-dichloroethane was added dropwise at 60°C to a solution of

6 g of 8-chlorocarbonyl-3-methyl-4-oxo-2-phenyl-4H-1-benzopyran in 22 ml of 1,2-dichloroethane. The reaction mixture was refluxed for 16 hours, and then cooled to ambient temperature and poured into cold 0.5N sodium hydroxide solution. Water and dichloromethane were added. The organic phase was separated off, washed with aqueous sodium chloride solution and dried on anhydrous sodium sulphate. The solvents were evaporated off and the oily residue was purified by flash chromatography on silica gel, eluting with petroleum ether:ethyl acetate 85:15. The collected fractions were evaporated to dryness in vacuo and the residue was dissolved in ethanol. Excess ethanolic hydrogen chloride was added to give 6.71 g of the title compound, m.p. 203-204°C.

## Example 11

**8-{3-[4-(2-methoxyphenyl)-1-piperazinyl]-propylcarbamoyl}-3-methyl-4-oxo-2-phenyl-4H-1-benzopyran dihydrochloride hemihydrate**

A mixture of 6.28 g of 1-(2-methoxyphenyl)-piperazine and 5.34 g of Intermediate XXXVII was heated at 180°C for 5 hours. After cooling, the dark mass was purified by flash chromatography on silica gel, eluting with dichloromethane:methanol 100:3. The fractions containing the title compound were pooled. The solvents were removed in vacuo and the residue was dissolved in boiling ethanol. The solution was filtered, acidified with ethanolic hydrogen chloride, and stood overnight at 20-25°C. The crude product was collected by filtration and crystallized from ethanol to give 5 g of the title compound, m.p. (177) 182-186°C.

## Example 12

**8-{3-[4-(2-methoxyphenyl)-1-piperazinyl]-propylcarbamoyl}-3-methyl-4-oxo-2-phenyl-4H-1-benzopyran dihydrochloride hemihydrate**

A solution of 4.48 g of 8-chlorocarbonyl-3-methyl-4-oxo-2-phenyl-4H-1-benzopyran in 40 ml of chloroform was added dropwise over a period of 10 minutes at ambient temperature to a solution of 3.74 g of 3-[4-(2-methoxyphenyl)-1-piperazinyl]-propylamine, prepared as described in GB 2161807, and 1.97 g of triethylamine in 50 ml of chloroform. After stirring for 2 hours, the solution was washed first with 0.5N hydrochloric acid, secondly with a saturated aqueous sodium bicarbonate solution and finally with water. The chloroform solution was dried on anhydrous sodium sulphate and the solvent was evaporated off in vacuo. The residue was worked up as described in Example 11 to give 6.67 g of the title compound, m.p. (177) 182-186°C. This represents an alternative route to the product of Example 11.

The following salts were also prepared:

monohydrochloride hydrate, m.p. 151-154°C,
monomethanesulphonate, m.p. 162-164°C, and
(±)-hemimalate hydrate, m.p. 110-112°C.

This example has described the condensation of the amine, 3-[4-(2-methoxyphenyl)-1-piperazinyl]-propylamine, with the carbonyl chloride, 3-methyl-4-oxo-2-phenyl-4H-1-benzopyran-8-carbonyl chloride. It should be noted that the amine can be condensed with the corresponding free acid or the corresponding ethyl ester by heating equimolar amounts thereof with or without a solvent. If a solvent is used, a high boiling point hydrophilic or hydrophobic solvent is appropriate. The amine can also be condensed at ambient temperature with an equimolar amount of the corresponding free acid in the presence of N,N'-dicyclohexylcarbodiimide and 4-dimethylaminopyridine in a solvent such as dichloromethane, chloroform, tetrahydrofuran or dimethylformamide.

## Example 13

**8-{2-[4-(2-methoxyphenyl)-1-piperazinyl]-ethylcarbamoyl}-3-methyl-4-oxo-2-phenyl-4H-1-benzopyran monohydrochloride hemihydrate**

The title compound was prepared by the method described in Example 16, but using Intermediate XIV instead of Intermediate XV and heating at 55-60°C for 32 hours. Also, work up was varied as follows. After collecting the base by filtration, purification was by flash chromatography on silica gel, eluting with chloroform:methanol 100:0.5 and then 100:1. The fractions containing the title compound were pooled and the solvents were removed in vacuo. The residue was crystallized from ethanol. After filtration, the solids were taken up in boiling water and sufficient dilute hydrochloric acid was added to effect solution. The crystalline salt separated on cooling and was collected by suction filtration. M.p. 119-123°C.

**Example 14**

**8-{3-[2-(2-methoxyphenoxy)-ethylamino]-propylcarbamoyl}-3-methyl-4-oxo-2-phenyl-4H-1-benzopyran hydrochloride**

Operating as described in Example 11, but using 2-(2-methoxyphenoxy)-ethylamine (prepared according to Augstein, J. et al, *J. Med. Chem.*, 8, 356, 1965) instead of 1-(2-methoxyphenyl)-piperazine, heating for 2 hours instead of 5 hours, and using dichloromethane:methanol 100:5 as eluant, the title compound was obtained. M.p. 200-202°C (ethanol).

**Example 15**

**8-[3-(4-phenyl-1-piperazinyl)-propylcarbamoyl]-3-methyl-4-oxo-2-phenyl-4H-1-benzopyran monohydrochloride hemihydrate**

Operating as described in Example 11, but using 1-phenylpiperazine instead of 1-(2-methoxyphenyl)-piperazine, heating for 2 hours instead of 5 hours, and using dichloromethane:methanol 100:4 as eluant, the title compound was obtained. M.p. (251) 255-258°C with decomposition (87% ethanol).

**Example 16**

**8-{N-methyl-2-[4-(2-methoxyphenyl)-1-piperazinyl]-ethylcarbamoyl}-3-methyl-4-oxo-2-phenyl-4H-1-benzopyran monohydrochloride**

A mixture of 3.56 g of Intermediate XV, 2.35 g of 1-(2-methoxyphenyl)-piperazine, 2.76 g of anhydrous potassium carbonate and 1.66 g of potassium iodide in 25 ml of dimethylformamide was stirred at 100°C for 6 hours. After cooling, the solvent was removed in vacuo and the residue was taken up in 50 ml of water, stirred for 1 hour at ambient temperature, collected by filtration, washed with water and crystallized from 95% ethanol in the presence of a small amount of activated charcoal (for decolouring). The base was dissolved in 105 ml of boiling 0.086 N hydrochloric acid. After cooling, the crystallized salt was collected by filtration, giving 4.3 g of the title compound (m.p. 201-203°C).

**Example 17**

**8-{1-hydroxy-2-[4-(2-methoxyphenyl)-1-piperazinyl]-ethyl}-3-methyl-4-oxo-2-phenyl-4H-1-benzopyran hydrochloride**

1.36 g of sodium borohydride was added portionwise at 0 to 5°C to a solution of 15.5 g of the compound prepared in Example 1 in 1500 ml of methanol. After stirring for 90 minutes at 0 to 5°C, 3N hydrochloric acid was added in order slightly to acidify the reaction mixture, which was then stripped in vacuo. The residue was shaken with 2N aqueous sodium hydroxide solution and extracted with chloroform. The organic layer was dried on anhydrous sodium sulphate/calcium chloride, filtered, acidified with ethanolic hydrogen chloride and stripped in vacuo. After washing with diethyl ether, the crude product was crystallized from ethanol to give 9.5 g of title compound, m.p. 248-249°C.

**Example 18**

**8-{1-hydroxy-2-[4-(2-methylphenyl)-1-piperazinyl]-ethyl}-3-methyl-4-oxo-2-phenyl-4H-1-benzopyran hydrochloride**

This compound was prepared according to Example 17, but starting from the compound prepared in Example 2 rather than that prepared in Example 1. M.p. 257-258°C (ethanol).

**Example 19**

**8-{1-Hydroxy-2-[4-(2-ethoxyphenyl)-1-piperazinyl]-ethyl}-3-methyl-4-oxo-2-phenyl-4H-1-benzopyran hydrochloride**

This compound was prepared according to Example 17, but starting from the compound prepared in Example 3 rather than that prepared in Example 1. M.p. 241-242°C (methanol).

**Example 20**

**8-{1-Hydroxy-3-[4-(2-methoxyphenyl)-1-piperazinyl]-propyl}-3-methyl-4-oxo-2-phenyl-4H-1-benzopyran**

**hydrochloride**

This compound was prepared according to Example 17, but starting from the compound prepared in Example 4 rather than that prepared in Example 1. The crude base was purified by flash chromatography (silica gel, eluant - ethyl acetate:chloroform 4:1). The fractions containing the pure base were pooled, acidified with ethanolic hydrogen chloride and stripped in vacuo. The residue was crystallized from ethanol. M.p. (126) 156-160°C.

## Example 21

**8-{1-hydroxy-4-[4-(2-methoxyphenyl)-1-piperazinyl]-butyl}-3-methyl-4-oxo-2-phenyl-4H-1-benzopyran hydrochloride monohydrate**

A solution of 3.04 g of Intermediate XXXVIII and 2.45 g of 1-(2-methoxyphenyl)-piperazine in 21 ml of anhydrous dimethylformamide was stirred for 5 hours at ambient temperature. A further 1.22 g of 1-(2-methoxyphenyl)-piperazine was added, and the mixture was stirred for 4 hours, poured into 300 ml of water, and extracted with ethyl acetate. The combined organic extracts were washed with aqueous sodium bicarbonate solution and then with aqueous sodium chloride solution, and evaporated to dryness in vacuo. The residue was purified by flash chromatography on silica gel, eluting with ethyl acetate:methanol 95:5. The collected fractions were stripped on a rotary evaporator, and the residue was dissolved in 0.81M ethanolic hydrogen chloride and stripped again in vacuo. The solid residue was crystallized from water:ethanol 9:1 to give 2.43 g of the title compound, m.p. 144-146°C.

## Example 22

**8-{1-ethoxy-2-[4-(2-methoxyphenyl)-1-piperazinyl]-ethyl}-3-methyl-4-oxo-2-phenyl-4H-1-benzopyran hydrochloride**

6 ml of anhydrous dimethylsulphoxide was added to 6.55 g of sodium hydride (50% in mineral oil, repeatedly washed with hexane) under nitrogen. A solution of 3 g of the compound prepared in Example 17 in 50 ml of anhydrous dimethylsulphoxide was added to the mixture at 20-25°C. After stirring for 1 hour at 20°C, 0.66 g of ethyl bromide was added. The reaction mixture was stirred for an additional 20 minutes at the same temperature and then poured into iced water. The crude product obtained after suction filtration was purified by flash chromatography (silica gel, eluant - chloroform:ethyl acetate 8:2). The fractions containing the pure title compound were pooled, acidified with ethanolic hydrogen chloride, and stripped in vacuo. The residue was crystallized from chloroform:diethyl ether and dried in vacuo at 140°C to give 1.6 g of the title compound, m.p. (155) 209°C.

## Example 23

**8-{N-methyl-2-[4-(2-methoxyphenyl)-1-piperazinyl]-ethylaminomethyl}-3-methyl-4-oxo-2-phenyl-4H-1-benzopyran dihydrochloride hemihydrate**

A mixture of 5.2 g of Intermediate XXVII, 3.1 g of 1-(2-methoxyphenyl)-piperazine and 2.2 g of anhydrous potassium carbonate in 50 ml of dimethylformamide was stirred at 70°C for 7 hours. After cooling to 20-25°C, the reaction mixture was poured into 500 ml of water, and extracted with dichloromethane. The organic phase was washed with water and dried on anhydrous sodium sulphate. The solvent was removed in vacuo. The residue was purified by flash chromatography on silica gel, eluting with ethyl acetate:petroleum ether 98:2. The title compound was obtained by salification with ethanolic hydrogen chloride. M.p. 217-219°C.

## Example 24

**8-{N-acetyl-2-[4-(2-methoxyphenyl)-1-piperazinyl]-ethylaminomethyl}-3-methyl-4-oxo-2-phenyl-4H-1-benzopyran hydrochloride**

A mixture of 5 g of Intermediate XXXIII and 5.3 g of 1-(2-methoxyphenyl)-piperazine in 75 ml of dimethylformamide was stirred at 95°C for 2 hours. After cooling to 20-25°C, the reaction mixture was poured into 200 ml of water, made alkaline with potassium carbonate and extracted with ethyl acetate. The organic phase was washed with water and dried on anhydrous sodium sulphate. The solvent was removed in vacuo. The residue was purified by flash chromatography on silica gel, eluting with dichloromethane:methanol 100:0.2. Salification of the pure base with ethanolic hydrogen chloride and recrystallization from methanol gave 4.4 g of the title compound, melting at (200) 227-228°C and containing one equivalent of methanol.

**Example 25**

**8-[4-(2-methoxyphenyl)-1-piperazinylacetamidomethyl]-3-methyl-4-oxo-2-phenyl-4H-1-benzopyran hydrochloride**

A mixture of 3.42 g of Intermediate XXXII, 2.74 g of 1-(2-methoxyphenyl)-piperazine and 0.71 g of anhydrous potassium carbonate in 34 ml of anhydrous dimethylformamide was stirred at 0°C for 2 hours. The reaction mixture was poured into water and filtered under suction. The resultant solid was purified by flash chromatography on silica gel, eluting with ethyl acetate:petroleum ether 6:4. The collected fractions were evaporated to dryness in vacuo, and the residue was crystallized from ethyl methyl ketone. The base obtained was treated in ethanolic solution with a molar equivalent of aqueous 2.25N hydrochloric acid to give the title compound, m.p. 168-170°C.

**Example 26**

**8-{N-methyl-N-[4-(2-methoxyphenyl)-1-piperazinyl]-acetamidomethyl}-3-methyl-4-oxo-2-phenyl-4H-1-benzopyran hydrochloride hydrate**

A mixture of 5 g of Intermediate XXXI, 2.9 g of 1-(2-methoxyphenyl)-piperazine and 2 g of anhydrous potassium carbonate in 50 ml of dimethylformamide was stirred at 20-25°C for 3 hours. The reaction mixture was then poured into 500 ml of water and extracted with dichloromethane. The organic phase was washed with water and dried on anhydrous sodium sulphate. The solvent was removed in vacuo. The residue was purified by flash chromatography on silica gel, eluting with ethyl acetate:petroleum ether 6:4, and crystallized from acetone to give 3.6 g of the base of the title compound, melting at 144-145°C. The base was dissolved in ethanol and 8N hydrochloric acid and water were added, yielding the title compound, m.p. 218-220°C, after drying at 100°C in vacuo.

**Example 27**

**8-{2-[4-(2-methoxyphenyl)-1-piperazinyl]-ethoxymethyl}-3-methyl-4-oxo-2-phenyl-4H-1-benzopyran dihydrochloride**

A mixture of 4 g of Intermediate XVIII, 2.4 g of 1-(2-methoxyphenyl)-piperazine, 1.96 g of potassium iodide and 1.65 g of anhydrous potassium carbonate in 40 ml of anhydrous dimethylformamide was stirred at 90°C for 7 hours. After cooling to ambient temperature, the mixture was poured into water and extracted with dichloromethane. The combined extracts were washed with aqueous sodium chloride solution, dried on anhydrous sodium sulphate and evaporated to dryness in vacuo. The residue was crystallized from ethyl acetate and the collected crystals were dissolved in ethanol and treated with excess ethanolic hydrogen chloride to give 5.21 g of the title compound, m.p. 199-201°C.

**Example 28**

**8-{2-[2-(2-ethoxyphenoxy)-ethylamino]-ethoxymethyl}-3-methyl-4-oxo-2-phenyl-4H-1-benzopyran hydrochloride**

Operating as described in Example 27, but using 2-(2-ethoxyphenoxy)-ethylamine in place of 1-(2-methoxyphenyl)-piperazine and including a purification step of flash chromatography on silica gel eluted with ethyl acetate:methanol 97:3, 4.25 g of the title compound was obtained. M.p. 191-193°C.

**Example 29**

**8-{2-[4-(2-methoxyphenyl)-1-piperazinyl]-ethylthiomethyl}-3-methyl-4-oxo-2-phenyl-4H-1-benzopyran hydrochloride**

2.5 g of potassium carbonate, 2.13 g of potassium iodide and 3.15 g of 1-(2-methoxyphenyl)-piperazine were added to a solution of 5 g of Intermediate XXI in 50 ml of dimethylformamide, and the mixture was stirred at 90°C for 4½ hours. After cooling to ambient temperature, the reaction mixture was poured into 450 ml of water and extracted with ethyl acetate. The organic extracts were washed with water, dried on anhydrous sodium sulphate and evaporated to dryness in vacuo. A solution of the residue in acetone was treated with a molar equivalent of 3.8N hydrogen chloride in diethyl ether, filtered and recrystallized from ethanol to yield 6.15 g of the title compound, m.p. (218) 223-224°C.

**Example 30**

**8-{2-[4-(2-methoxyphenyl)-1-piperazinyl]-ethylsulphinylmethyl}-3-methyl-4-oxo-2-phenyl-4H-1-benzopyran dihydrochloride hemihydrate**

The title compound was prepared by the method described in Example 29, using Intermediate XXVI instead of Intermediate XXI and stirring for 2½ hours rather than for 4½ hours. After the usual work up, the residue was purified by flash chromatography on silica gel, eluting with ethyl acetate:methanol 97:3. The collected fractions were acidified with excess ethanolic hydrogen chloride, evaporated to dryness in vacuo. The residue was crystallized from ethanol, giving 5.2 g of the title compound, m.p. 170-172°C. This compound contains 1 equivalent of ethanol.

**Example 31**

**8-{2-[4-(2-methoxyphenyl)-1-piperazinyl]-ethylsulphonylmethyl}-3-methyl-4-oxo-2-phenyl-4H-1-benzopyran hydrochloride**

A mixture of 4.5 g of Intermediate XXV, 2.36 g of 1-(2-methoxyphenyl)-piperazine and 0.84 g of potassium carbonate in 45 ml of anhydrous dimethylformamide was stirred at ambient temperature for 2½ hours. The reaction mixture was poured into 300 ml of water and filtered under suction, washing with water. The solid base was crystallized from ethanol and had a melting point of 143-146°C. The crystallate was dissolved in 1,2-dichloroethane and acidified with ethanolic hydrogen chloride. 4.4 g of the title compound, m.p. 229-233°C, was obtained by recrystallization from methanol:water 1:3.5.

**Example 32**

**8-{2-[4-(2-methoxyphenyl)-1-piperazinyl]-ethylamino}-3-methyl-4-oxo-2-phenyl-4H-1-benzopyran dihydrochloride**

A solution of 3.7 g of Intermediate XXIII in 10 ml of dimethylformamide was added dropwise at 0°C to a suspension of 0.9 g of sodium hydride (50% in mineral oil) in 9 ml of dimethylformamide. The cooling bath was removed, and after 30 minutes at 20-25°C a solution of 4.1 g of 1-(2-chloroethyl)-4-(2-methoxyphenyl)-piperazine in 10 ml of dimethylformamide was added. The mixture was stirred at 90°C for 5 hours and then cooled to 20-25°C. A further addition of 0.25 g of sodium hydride (50% in mineral oil) followed by 1.36 g of 1-(2-chloroethyl)-4-(2-methoxyphenyl)-piperazine in 5 ml of dimethylformamide was made. The mixture was stirred at 90°C for 8 hours and again cooled to 20-25°C. 200 ml of water was cautiously added, and the mixture was extracted with ethyl acetate. The organic layer was washed with water and dried on anhydrous sodium sulphate. The solvent was evaporated off in vacuo and the residue was purified by flash chromatography on silica gel, eluting with n-hexane:ethyl acetate 3:2. This gave a mixture of the base of the title compound and the corresponding N-trifluoroacetyl compound.

3.8 g of this mixture was dissolved in 35 ml of ethanol and 35 ml of dimethylsulphoxide. To this solution, 0.55 g of sodium borohydride was added portionwise at 20-25°C. The mixture was stirred for 3 hours at this temperature, and then poured into 200 ml of water and extracted with ethyl acetate. The organic extracts were washed with water, dried on anhydrous sodium sulphate and evaporated to dryness in vacuo. The residue was dissolved in dichloromethane. 2 equivalents of ethanolic hydrogen chloride were added to give the title compound, which was recrystallized from ethanol. Yield 3.8 g, m.p. 231-234°C.

**Example 33**

**8-{3-[4-(2-methoxyphenyl)-1-piperazinyl]-propylamino}-3-methyl-4-oxo-2-phenyl-4H-1-benzopyran hydrochloride 2.75-hydrate**

Using 1-(3-chloropropyl)-4-(2-methoxyphenyl)-piperazine instead of 1-(2-chloroethyl)-4-(2-methoxyphenyl)-piperazine, but otherwise operating as described in Example 32, the title compound was obtained. M.p. 206-208°C (10% ethanol).

**Example 34**

**8-{4-[4-(2-methoxyphenyl)-1-piperazinyl]-butylamino}-3-methyl-4-oxo-2-phenyl-4H-1-benzopyran hydrochloride hemihydrate**

A mixture of 4.5 g of Intermediate XXXIX, 3.9 g of 8-amino-3-methyl-4-oxo-2-phenyl-4H-1-benzopyran, 8.3 g of sodium triacetoxyborohydride and 3.4 ml of acetic acid in 40 ml of 1,2-dichloroethane was stirred for

6 hours at 20-25°C. 15 ml of 5% aqueous sodium bicarbonate solution was then added, and the mixture was stirred for 10 minutes. The mixture was then made alkaline by addition of 0.5N sodium hydroxide solution and extracted with dichlormethane. The organic extracts were washed with water and dried on anhydrous sodium sulphate. The solvent was evaporated off in vacuo and the residue was purified by flash chromatography on silica gel, eluting with ethyl acetate:petroleum ether 9:1. The base obtained was dissolved in dichloromethane and 1 equivalent of ethanolic hydrogen chloride was added. After removing the solvent in vacuo, the residue was crystallized from 50% ethanol to give 1.6 g of the title compound, m.p. (140) 151-153°C.

## Example 35

**8-{N-methyl-3-[4-(2-methoxyphenyl)-1-piperazinyl]-propylamino}-3-methyl-4-oxo-2-phenyl-4H-1-benzopyran hydrochloride hemihydrate**

A mixture of 4 g of the compound prepared in Example 33, in the form of its base, 4.35 ml of 37% aqueous formaldehyde and 1.15 g of sodium cyanoborohydride in 25 ml of acetonitrile was stirred at 20-25°C, maintaining the pH in the range 5-6 by the addition of acetic acid during the reaction. After 4 hours the solvent was evaporated off in vacuo. 80 ml of ethyl acetate and 200 ml of ice cooled 1N sodium hydroxide solution were added to the residue. The organic phase was washed with water, dried on anhydrous sodium sulphate and evaporated to dryness in vacuo. The residue was purified by flash chromatography on silica gel, eluting with ethyl acetate:petroleum ether 3:1. The pure base which was obtained was dissolved in diethyl ether. 1 equivalent of ethanolic hydrogen chloride was added and the solvent was removed in vacuo. The residue was crystallized from water to give 2 g of the title compound, m.p. 186-187°C.

## Example 36

**8-{N-acetyl-3-[4-(2-methoxyphenyl)-1-piperazinyl]-propylamino}-3-methyl-4-oxo-2-phenyl-4H-1-benzopyran hydrochloride hydrate**

A mixture of 4.8 g of the compound prepared in Example 33, in the form of its base, 2.8 ml of acetic anhydride and 33 ml of pyridine was stirred at 80°C for 4 hours. After cooling to 20-25°C, the reaction mixture was poured into 200 g of iced water, acidified with 10N hydrochloric acid and extracted with dichloromethane. The organic extracts were washed with water, dried on anhydrous sodium sulphate and evaporated to dryness in vacuo. The residue was purified by flash chromatography on silica gel, eluting with ethyl acetate:methanol 95:5. The pure base which was obtained was dissolved in dichloromethane. 1 equivalent of ethanolic hydrogen chloride was added and the solvent was removed in vacuo. The residue was crystallized from acetonitrile to give 3 g of the title compound containing 0.33 equivalents of acetonitrile, m.p. 208.5-210.5°C.

## Example 37

**8-{3-[4-(2-methoxyphenyl)-1-piperazinyl]-propionamido}-3-methyl-4-oxo-2-phenyl-4H-1-benzopyran hydrochloride**

A mixture of 3.97 g of Intermediate X and 3.07 g of 1-(2-methoxyphenyl)-piperazine in 40 ml of dimethylformamide was stirred at 60°C for 6 hours. The reaction mixture was then cooled to ambient temperature and poured into water. Following extraction with dichloromethane, the organic phase was washed with water and dried on anhydrous sodium sulphate. The solvent was removed in vacuo. The crude residue was crystallized from ethanol to give the base of the title compound, which was then dissolved in hot ethanol. 1 molar equivalent of 0.81M ethanolic hydrogen chloride was added to the solution. 4 g of the title compound, m.p. 255-257°C, was obtained.

## Example 38

**8-{2-[4-(2-methoxyphenyl)-1-piperazinyl]-ethylureido}-3-methyl-4-oxo-2-phenyl-4H-1-benzopyran methanesulphonate**

A mixture of 3.34 g of Intermediate XLIV and 7.22 g of 1-(2-methoxyphenyl)-piperazine was stirred at 100°C for 5 hours. An additional 1.8 g of 1-(2-methoxyphenyl)-piperazine was then added and stirring was continued for a further 2 hours at 100°C. After cooling to ambient temperature, the reaction mixture was poured into water and extracted with ethyl acetate. The organic phase was washed with aqueous sodium hydroxide solution, dried on anhydrous sodium sulphate and evaporated to dryness in vacuo. The crude residue was purified by flash chromatography on silica gel, eluting with ethyl acetate:methanol 98:2. The collected fractions were evaporated to dryness in vacuo and crystallized from water:ethanol 4:6. The crystals were redissolved in dichloromethane and treated with 1 molar equivalent of methanesulphonic acid. The crude methanesulphonate obtained by evaporation in vacuo was crystallized from ethyl acetate:ethanol 1:1 to yield 2.35 g of the title compound, melting at 191-193°C.

## Example 39

**8-{2-[4-(2-methoxyphenyl)-1-piperazinyl]-ethoxy}-3-methyl-4-oxo-2-phenyl-4H-1-benzopyran hydrochloride hydrate**

A mixture of 6.61 g of the Intermediate XI, 8.34 g of 1-(2-methoxyphenyl)-piperazine and 1.26 g of sodium iodide in 70 ml of dimethylformamide was stirred at 80°C for 17 hours. After cooling to 20-25°C, the reaction mixture was poured into 600 ml of water, made alkaline with 5% aqueous sodium bicarbonate and extracted with dichloromethane. The organic extracts were washed with aqueous sodium chloride solution, dried on anhydrous sodium sulphate and evaporated to dryness in vacuo. The residue was purified by flash chromatography on silica gel, eluting with dichloromethane:methanol 99:1, then 98:2. The fractions containing the base of the title compound were pooled, and the solvent was removed in vacuo. The residue was dissolved in ethanol and ethanolic hydrogen chloride was added. The title compound crystallized and was collected by suction filtration. It was recrystallized from 95% ethanol. Yield 6.5 g, m.p. 224-225°C.

| **Elemental analysis:** | | | | | |
|---|---|---|---|---|---|
| Found % : | C=66.38, | H=6.34, | N=5.35, | Cl=6.76, | $H_2O$=3.35 |
| Calculated % : | 66.34, | 6.14, | 5.33, | 6.75, | 3.43 |

**NMR Spectrum at 60 MHz ($CDCl_3$-$CD_3OD$)**

| | |
|---|---|
| 7.8-7.1 | (m, 8H, aromatic protons of the benzopyran ring) |
| 7.1-6.6 | (m, 4H, aromatic protons of the 2-methoxyphenyl group) |
| 4.8-4.4 | (m, 2H, $OCH_2$) |
| 4.4-4.1 | (m, 3H, $H_2O$ and $N^+H$) |
| 3.9-3.0 | (m, 10H, 5 x $CH_2N$) |
| 3.8 | (s, 3H, $OCH_3$) |
| 2.1 | (s, 3H, $CH_3$) |

## Example 40

**8-{3-[4-(2-methoxyphenyl)-1-piperazinyl]-propoxy}-3-methyl-4-oxo-2-phenyl-4H-1-benzopyran dihydrochloride**

This compound was prepared by the method described in Example 39, but using Intermediate IX instead of Intermediate XI. Purification by flash chromatography was omitted as unnecessary in this case. M.p. 226-227°C.

## Example 41

**8-{4-[4-(2-methoxyphenyl)-1-piperazinyl]-butoxy}-3-methyl-4-oxo-2-phenyl-4H-1-benzopyran dihydrochloride**

A mixture of 7.75g of Intermediate XVI, 4.7 g of 1-(2-methoxyphenyl)-piperazine, 3.3 g of potassium iodide and 2.8 g of anhydrous potassium carbonate in 78 ml of dimethylformamide was stirred at 75°C for 2 hours. After cooling to 20-25°C, the reaction mixture was poured into 600 ml of water and extracted with dichloromethane. The organic extracts were washed with water and dried on anhydrous sodium sulphate, and the solvent was then evaporated off in vacuo. The residue was purified by column chromatography on silica gel, eluting with ethyl acetate. The pure title compound thus obtained as its base was transformed into its dihydrochloride by treatment with ethanolic hydrogen chloride. After crystallization from ethanol, 6.5 g of title compound was obtained. m.p. 217-219°C.

**Example 42**

**8-{5-[4-(2-methoxyphenyl)-1-piperazinyl]-pentyloxy}-3-methyl-4-oxo-2-phenyl-4H-1-benzopyran hydrochloride**

This compound was prepared by the method described in Example 41, but using Intermediate XVII instead of Intermediate XVI. M.p. 173°C (ethanol). The corresponding base melts at 117-118°C (ethanol).

**Example 43**

**8-{3-[4-(2-methoxyphenyl)-1-oxo-1-piperazinyl]-propoxy}-3-methyl-4-oxo-2-phenyl-4H-1-benzopyran 1.75-hydrate**

2.93 g of magnesium monoperoxyphthalate in 10 ml of water was added dropwise at -15°C to a solution of 4.34 g of the compound prepared in Example 40 and 0.1 g of benzyl(triethyl)ammonium chloride in 20 ml of dichloromethane and 20 ml of methanol. The mixture was stirred for 2 hours at 0°C and then warmed to ambient temperature. It was poured into water and made basic by addition of aqueous sodium hydroxide solution. Extraction with dichloromethane gave, after the usual work up, a solid which was purified by flash chromatography, eluting with dichloromethane:methanol 9:1. The collected fractions containing the pure compound were evaporated to dryness in vacuo and the residue was crystallized from acetonitrile to give 0.5 g of the title compound, m.p. 89-92°C.

**Example 44**

**8-{2-[2-(2,6-dimethoxyphenoxy)-ethylamino]-ethoxy}-3-methyl-4-oxo-2-phenyl-4H-1-benzopyran hydrochloride**

A mixture of 4.5 g of Intermediate XII, 3.7 g of triphenylphosphine and 2.85 g of 2,6-dimethoxyphenoxyacetaldehyde (prepared according to Nelson, W.L. et al., *J. Med. Chem.*, 22, 1125, 1979) in 45 ml of benzene was stirred at 20-25°C for 18 hours and at reflux for 5 hours. The solvent was evaporated off in vacuo and the residue was dissolved in 80 ml of anhydrous methanol. 3Å molecular sieve was added. Next, 0.61 g of sodium borohydride was added at 0°C. The mixture was stood for 1 hour at 0°C and for 1 hour at 20-25°C, and then poured into iced water and extracted with dichloromethane. The organic extracts were washed with water and dried on anhydrous sodium sulphate. The solvent was removed in vacuo and the residue was purified by flash chromatography on silica gel, eluting with dichloromethane: methanol 49:1. The base obtained was treated with ethanolic hydrogen chloride. After crystallization from ethanol, the title compound was obtained. Yield 40%, m.p. 200-202°C.

**Example 45**

**8-{2-hydroxy-3-[4-(2-methoxyphenyl)-1-piperazinyl]-propoxy}-3-methyl-4-oxo-2-phenyl-4H-1-benzopyran hydrochloride**

A solution of 3.7 g of Intermediate XL and 4.64 g of 1-(2-methoxyphenyl)-piperazine in 40 ml of dimethylformamide was stirred at 80°C for 3 hours. After cooling to 20-25°C, the reaction mixture was poured into 400 ml of water and extracted with dichloromethane. The aqueous phase was made alkaline with 1N sodium hydroxide solution and extracted with ethyl acetate. The combined organic extracts were washed with water and dried on anhydrous sodium sulphate. The solvents were evaporated off in vacuo. The residue was purified by flash chromatography on silica gel, eluting with ethyl acetate. The fractions containing the title compound in the form of its base were pooled, and stripped in vacuo. The residue was dissolved in dichloromethane and one equivalent of ethanolic hydrogen chloride was added. The solvents were removed in vacuo and the residue was crystallized from ethanol. 5 g of the title compound containing one molar equivalent of ethanol were obtained. M.p. (122) 126-128°C with decomposition.

**Example 46**

**8-{3-[4-(2-methoxyphenyl)-1-piperazinyl]-propylthio}-3-methyl-4-oxo-2-phenyl-4H-1-benzopyran**

A mixture of 4.4 g of Intermediate XXXIV, 2.5 g of 1-(2-methoxyphenyl)-piperazine, 1 g of potassium iodide and 1.8 g of anhydrous potassium carbonate in 40 ml of dimethylformamide was stirred at 100°C for 3 hours. After cooling to 20-25°C, the reaction mixture was poured into 350 ml of water and extracted with dichloromethane. The organic extracts were washed with water and dried on anhydrous sodium sulphate, and the solvent was then evaporated off in vacuo. The residue was purified by column chromatography on silica gel, eluting

with ethyl acetate: petroleum ether 3:2, and by crystallization from ethanol, yielding 3.9 g of the title compound, m.p. (70) 96-99°C.

**Example 47**

**8-{3-[4-(2-methoxyphenyl)-1-piperazinyl]-propylsulphonyl}-3-methyl-4-oxo-2-phenyl-4H-1-benzopyran hydrochloride**

A solution of 3.8 g of Intermediate XXXV and 4 g of 1-(2-methoxyphenyl)-piperazine in 40 ml of dimethylformamide was heated at 60°C for 7 hours. After cooling to 20-25°C, the reaction mixture was poured into 500 ml of water and extracted with dichloromethane. The organic extracts were washed with water and dried on anhydrous sodium sulphate, and the solvent was then evaporated off in vacuo. The residue was purified by flash chromatography on silica gel, eluting with ethyl acetate: petroleum ether 1:1. The base of the title compound was obtained. It was dissolved in ethanol and one equivalent of ethanolic hydrogen chloride was added to give 4.5 g of the title compound, m.p. (215) 226-228°C.

**Example 48**

**8-{2-[4-(2-methoxyphenyl)-1-piperazinyl]-ethylsulphamoyl}-3-methyl-4-oxo-2-phenyl-4H-1-benzopyran hydrochloride**

A solution of 4.5 g of Intermediate XLII and 3.8 g of 1-(2-methoxyphenyl)-piperazine in 40 ml of dimethylformamide was heated at 70°C for 7 hours. After cooling to 20-25°C, the reaction mixture was poured into 150 ml of water and extracted with dichloromethane. The organic solution was washed with water and dried on anhydrous sodium sulphate, and the solvent was then evaporated off in vacuo. The residue was purified by flash chromatography on silica gel, eluting with ethyl acetate: petroleum ether 3:7, and the title compound was obtained by salification with ethanolic hydrogen chloride. Yield 2.9 g, m.p. 236-238°C.

**Example 49**

**8-{N-methyl-2-[4-(2-methoxyphenyl)-1-piperazinyl]-ethylsulphamoyl}-3-methyl-4-oxo-2-phenyl-4H-1-benzopyran hydrochloride**

The title compound was obtained by the method described in Example 48, but using Intermediate XLI instead of Intermediate XLII. M.p. 194-198°C (ethanol).

**Example 50**

**8-{N-carbamoyl-3-[4-(2-methoxyphenyl)-1-piperazinyl]-propylamino}-3-methyl-4-oxo-2-phenyl-4H-1-benzopyran methanesulphonate hemihydrate**

A mixture of 4.06 g of the compound of Example 33 and 1.5 g of potassium cyanate in 42 ml of glacial acetic acid was stirred at 50°C for 4 hours. The rection mixture was poured into iced water and made alkaline. The precipitate was collected by suction filtration, dried and purified by flash chromatography using a silica gel column eluting with ethyl acetate:methanol 98:2. The fractions, containing the title product as a base, were evaporated to dryness in vacuo and one equivalent of methanesulphonic acid was added to the residue after dissolution in 30 ml of dichloromethane. The solvent was evaporated off in vacuo and the residue was crystallized from ethanol to give 3.1 g of the title compound (m.p. 157-160°C, with decomposition). This compound contained one molar equivalent of ethanol.

**Example 51**

**8-{4-[4-(2-methoxyphenyl)-1-piperazinyl]-1-oxobutyl}-3-methyl-4-oxo-2-phenyl-4H-1-benzopyran methanesulphonate**

A solution of 1.33 ml of anhydrous dimethylsulphoxide in 9 ml of dichloromethane was added at -70°C to a solution of 0.74 ml of oxalyl chloride in 6 ml of dichloromethane. After stirring at -70°C for 15 minutes, a solution of 2.8 g of the compound of Example 21 (as a base) in 14 ml of dichloromethane was added. After 15 minutes at the same temperature, 4.7 ml of anhydrous triethylamine was added and the temperature was raised to -30°C over a period of 30 minutes. Stirring was continued at -30°C for another 30 minutes. After letting the temperature rise to 0°C, the mixture was diluted with 120 ml of water and extracted with dichloromethane. The organic phase was washed with water, dried on anydrous sodium sulphate and evaporated to dryness in vacuo. The residue was purified by flash chromatography in a silica gel column, eluting with ethyl acetate:dichloromethane 9:1. The fractions, containing the title product as a base, were evaporated to dryness in vacuo and one equivalent of methanesulphonic acid was added to the residue after dissolution in 30 ml of dichloromethane. The solvent was evaporated off in vacuo and the residue was crystallized from ethanol to give 2.9 g of the title compound, m.p. 194-195°C.

## Example 52

**8-{3-[2-(1,4-benzodioxanyl)methylamino]propylcarbamoyl}-3-methyl-4-oxo-2-phenyl-4H-1-benzopyran methansulphonate**

A mixture of 5.56 g of Intermediate XLIII, as a base, 4.58 g of 2-(p-toluenesulphonyloxymethyl)-1,4-benzodioxane and 1.9 g of anhydrous potassium carbonate in 80 ml of anhydrous dimethylformamide was stirred at 110°C for 5 hours. The reaction mixture was cooled to ambient temperature, poured into water and extracted with dichloromethane. The organic phase was washed with water, dried on anhydrous sodium sulphate, filtered and evaporated to dryness in vacuo. The residue was purified by flash chromatography using a silica gel column, eluting with ethyl acetate:methanol 95:5. The fractions containing the title compound as a base were evaporated to dryness in vacuo. The residue was dissolved in ethanol and one equivalent of methanesulphonic acid dissolved in ethyl acetate was added. The crystallized product was filtered off and recrystallized from ethanol to give 2.4 g of the title compound, m.p. 172-174°C.

## Example 53

**8-{4-[4-(2-methoxyphenyl)-1-piperazinyl]-butyl}-3-methyl-4-oxo-2-phenyl-4H-1-benzopyran methanesulphonate**

A solution of 2.8 g of Intermediate XLVI and 0.13 g of p-toluenesulphonic acid in 150 ml of methanol was refluxed for 5 hours. After cooling to 20-25°C, 0.8 g of anhydrous potassium carbonate was added and stirring was continued for 3 hours. After filtration, the reaction mixture was evaporated to dryness in vacuo to give 2.5 g of 8-(4,4-dimethoxybutyl)-3-methyl-4-oxo-2-phenyl-4H-1-benzopyran.

**NMR ($CDCl_3$, δ)**

| | |
|---|---|
| 1.6-1.9 | (4H, m, $CHC\underline{H}_2C\underline{H}_2CH$) |
| 2.2 | (3H, s, flavone $CH_3$) |
| 2.9 | (2H, t, Fl-$CH_2$) |
| 3.3 | (6H, s, 2 x $OCH_3$) |
| 4.4 | (1H, t, $C\underline{H}(OCH_3)_2$) |
| 7.3 | (1H, dd, flavone CH in 6) |
| 7.5-7.8 | (6H, m, flavone CH in 7, and 5 x phenyl CH) |
| 8.1 | (1H, dd, flavone CH in 5) |

A solution of 2.5 g of the compound thus prepared in 10 ml of water and 30 ml of acetic acid was heated at 50°C for 2½ hours. The reaction mixture was cooled to ambient temperature, diluted with iced water, basified with aqueous sodium carbonate, and extracted with chloroform. The organic phase was dried on anhydrous sodium sulphate, filtered and evaporated to dryness in vacuo. The residue was purified by flash chromatography on silica gel, eluting with petroleum ether:ethyl acetate 3:1. 2.1 g of 8-(4-oxobutyl)-3-methyl-4-oxo-2-phenyl-4H-1-benzopyran was obtained (>75% yield) and was used with no further purification in the next step.

**NMR ($CDCl_3$, δ)**

| | |
|---|---|
| 1.9-2.1 | (2H, dd, $CH_2C\underline{H}_2CH_2CHO$) |
| 2.2 | (3H, s, flavone $CH_3$) |
| 2.5 | (2H, t, $C\underline{H}_2CHO$) |
| 2.9 | (2H, t, Fl-$CH_2$) |
| 7.3 | (1H, dd, flavone CH in 6) |
| 7.5-7.7 | (6H, m, flavone CH in 7, and 5 x phenyl CH) |
| 8.1 | (1H, dd, flavone CH in 5) |
| 9.7 | (1H, s, CHO) |

2.3 ml of 6N hydrochloric acid in ethanol, a solution of 2.1 g of the compound thus prepared in 40 ml of methanol and 0.45 g of sodium cyanoborohydride were added in succession to a solution of 8 g of 1-(2-methoxyphenyl)-piperazine in 30 ml of methanol. After stirring the reaction mixture at ambient temperature for

24 hours, it was poured into 500 ml of iced water and extracted with dichloromethane. The organic phase was washed with water, dried on anhydrous sodium sulphate and evaporated to dryness in vacuo. The residue was purified by flash chromatography using a silica gel column, eluting with ethyl acetate:petroleum ether 9:1. The fractions, containing the title product as a base, were evaporated to dryness in vacuo. The residue was dissolved in 30 ml of dichloromethane and one equivalent of methanesulphonic acid was added. The solvent was evaporated off in vacuo and the residue was crystallized from acetone to give 2.35 g of the title compound (m.p. 141-143°C).

## Example 54

**8-[3-(4-phenyl-1-piperidinyl)-propylcarbamoyl]-3-methyl-4-oxo-2-phenyl-4H-1-benzopyran methansulphonate**

This compound was prepared by the method described in Example 11, using 4-phenylpiperidine instead of 1-(2-methoxyphenyl)-piperazine and conducting the reaction for 1 hour instead of 5 hours. Purification was carried out by flash chromatography using a silica gel column, eluting with dichloromethane:methanol 100:5. M.p. 157-159°C (ethyl acetate). The respective base melted at (127) 147-149°C (ethanol).

## Example 55

**8-[3-(4,4-diphenyl-1-piperidinyl)-propylcarbamoyl]-3-methyl-4-oxo-2-phenyl-4H-1-benzopyran methansulphonate**

This compound was prepared by the method described in Example 11, using 4,4-diphenylpiperidine instead of 1-(2-methoxyphenyl)-piperazine and conducting the reaction for 2 hours instead of 5 hours. M.p. 221-223°C (ethyl acetate).

## Example 56

**8-{3-[4-(4-fluorobenzoyl)-1-piperidinyl]-propyl-carbamoyl}-3-methyl-4-oxo-2-phenyl-4H-1-benzopyran**

This compound was prepared by the method described in Example 11, using 4-(4-fluorobenzoyl)-piperidine instead of 1-(2-methoxyphenyl)-piperazine and conducting the reaction for 30 minutes instead of 5 hours. Purification was carried out by flash chromatography using a silica gel column, eluting with dichloromethane:5N methanolic ammonia graduated 100:1 to 100:20. M.p. 181-183°C (ethanol).

## Example 57

**8-{3-[4-(2-oxo-1-benzimidazolinyl)-1-piperidinyl]-propylcarbamoyl-}-3-methyl-4-oxo-2-phenyl-4H-1-benzopyran**

This compound was prepared by the method described in Example 11, using 4-(2-oxo-1-benzimidazolinyl)-piperidine instead of 1-(2-methoxyphenyl)-piperazine. Purification was carried out by flash chromatography using a silica gel column, eluting with chloroform:5N methanolic ammonia 100:3. M.p. 238-241°C (ethanol).

## Example 58

**8-{3-[4-(2-pyrimidinyl)-1-piperazinyl]-propylcarbamoyl}-3-methyl-4-oxo-2-phenyl-4H-1-benzopyran methanesulphonate**

The title compound was prepared by the method described in Example 11, using 1-(2-pyrimidinyl)-piperazine instead of 1-(2-methoxyphenyl)-piperazine and conducting the reaction for 2 hours. The product was purified by flash chromatography using a silica gel column, eluting with chloroform:methanol 100:3. The desired fractions were dissolved in dichloromethane and one equivalent of methanesulphonic acid was added to the solution. After evaporation of the solvent in vacuo, the residue was boiled for 1 hour with ethyl acetate and then collected by filtration. M.p. 209-210°C. The product contained 0.2 equivalents of ethyl acetate and 0.1 equivalents of water. The respective base melted at 178-180°C (ethanol).

## Example 59

**8-{3-[4-(2-hydroxyphenyl)-1-piperazinyl]-propylcarbamoyl}-3-methyl-4-oxo-2-phenyl-4H-1-benzopyran**

Operating as described in Example 11, but using 1-(2-hydroxyphenyl)-piperazine instead of 1-(2-methoxyphenyl)-piperazine, heating for 1½ hours instead of 5 hours, and using dichloromethane:methanol 100:3 to

100:10 as eluant for column chromatography, the title compound was obtained. M.p. 118-120°C (ethanol 95%).

**Example 60**

**8-{4-[4-(2-methoxyphenyl)-1-piperazinyl]-butylcarbamoyl}-3-methyl-4-oxo-2-phenyl-4H-1-benzopyran methansulphonate**

This compound was prepared by the method used in Example 12, but using 4-[4-(2-methoxyphenyl)-1-piperazinyl]-butylamine instead of 3-[4-(2-methoxyphenyl)-1-piperazinyl]-propylamine. The reaction mixture was stirred at room temperature for 22 hours, diluted with water and filtered by suction, washing the insoluble solids with water. The crude residue was dried and purified by column chromatography on silica gel, eluting with ethyl acetate:methanol 9:1. The fractions contining the pure product as a base were collected, evaporated to dryness in vacuo and dissolved in dichloromethane. Methanesulphonic acid was added to the solution and the salt was precipitated by adding 2 volumes of ethyl acetate, filtered and recrystallized from ethanol to give the title compound, m.p. 230-232°C. This product contained 0.3 molar equivalent of ethanol.

**Example 61**

**8-{3-[4-(2-methoxyphenyl)-1-piperazinyl]-propylsulphamoyl}-3-methyl-4-oxo-2-phenyl-4H-1-benzopyran methanesulphonate**

This compound was obtained operating as described in Example 12 but using Intermediate VIII instead of 8-chlorocarbonyl-3-methyl-4-oxo-2-phenyl-4H-1-benzopyran and stirring for 24 hours instead of 2½ hours. The crude product was purified by column chromatography on silica gel, eluting with ethyl acetate: methanol 98.5:1.5. The collected fractions containing the pure product as a base were evaporated to dryness in vacuo and dissolved in dichloromethane. Methanesulphonic acid was added to the solution and the solvent was removed by evaporation in vacuo. The crude salt was crystallized from ethanol to give the title compound, m.p. (196) 198-200°C.

**Example 62**

**8-{3-[N-methyl-2-(2-methoxyphenoxy)-ethylamino]-propylcarbamoyl}-3-methyl-4-oxo-2-phenyl-4H-1-benzopyran hydrochloride**

A solution of 10.5 ml of 40% formaldehyde in water was added to a suspension of 6.66 g of the compound prepared in Example 14 in 55 ml of acetonitrile and 20 ml of water. After stirring for 15 minutes at room temperature, 2.70 g of sodium cyanoborohydride 95% was added to the red solution, and after an additional 15 minutes in the same conditions 1.38 ml of acetic acid was added. After stirring for 3 hours, the solvents were removed in vacuo and the residue was rinsed with 250 ml of water and 250 ml of chloroform. After addition of 3N sodium hydroxide, the organic phase was separated off and the aqueous phase was extracted twice with chloroform. The solvent was removed from the collected organic phases by evaporation in vacuo and the residue was purified by flash chromatography on silica gel, eluting with chloroform:5.2N methanolic ammonia 100:0.5 to 100:2. The collected fractions containing the pure title compound as a base were evaporated to dryness in vacuo and the residue was dissolved in hot ethanol. The solution was acidified with ethanolic hydrogen chloride and, after evaporation of the solvent in vacuo, the residue was rinsed with diethyl ether and stirred at room temperature. The crude product was collected by filtration and crystallized from acetonitrile to give 3.1 g of the title compound, m.p. 146-148°C.

**Example 63**

**8-{N-methyl-3-[4-(2-methoxyphenyl)-1-piperazinyl]-propionamido}-3-methyl-4-oxo-2-phenyl-4H-1-benzopyran methanesulphonate**

Operating as described in Example 37, but using Intermediate L instead of Intermediate X and stirring at 90°C for 4 hours instead of 60°C for 6 hours, the title compound was obtained as a crude base. After purification by column chromatography on silica gel, eluting with ethyl acetate:methanol 95:5, a crude methanesulphonate was obtained as described in Example 61 and crystallized from acetone to give the title compound, m.p. 200-202°C.

**Example 64**

**8-{3-[4-(2-methoxyphenyl)-1-piperazinyl]-propylcarbamoyl}-3-phenyl-4-oxo-4H-1-benzopyran dime-**

**thanesulphonate**

The title compound was prepared operating as described in Example 12, but using Intermediate LVI instead of 8-chlorocarbonyl-3-methyl-4-oxo-2-phenyl-4H-1-benzopyran and stirring for 24 hours instead of 2½ hours. The crude product was purified by column chromatography, eluting with ethyl acetate:methanol 92:8, and the pure base, obtained by evaporation in vacuo of the collected fractions, was dissolved in dichloromethane. Two equivalents of methanesulphonic acid were added. The crude dimethanesulphonate, obtained after evaporating off the solvent, was recrystallized from acetone, m.p. 153-156 (200)°C.

## Example 65

**8-{3-[(3,4-dihydro-1-oxo-2H-naphthyl)-methylamino]-propylcarbamoyl}-3-methyl-4-oxo-2-phenyl-4H-1-benzopyran methanesulphonate**

A mixture of 6 g of Intermediate XLIII, 2.4 g of 2-methylene-α-tetralone (prepared as described in *Org. Synth.*, 60, 88, 1981) and 3.14 ml of triethylamine in 48 ml of anhydrous dimethylformamide was stirred at room temperature for 6 hours, and then at 50°C for 1 hour. The reaction mixture was diluted with water and extracted with dichloromethane. The organic layers were washed with water, dried on anhydrous sodium sulphate and evaporated to dryness in vacuo. The crude residue was purified twice by column chromatography eluting first with dichloromethane:methanol 95:5 and then with dichloromethane: methanol:5.8N methanolic ammonia 98:2:0.2, to give 1.74 g of the title compound as a base. The base was converted into the methanesulphonate by the procedure described in Example 61. The salt was recrystallized first from acetone and then from acetonitrile to give the title compound, m.p. (69) 157-159°C.

## Example 66

**8-{2-[4-(2-methoxyphenyl)-1-piperazinyl]-ethoxycarbonylmethyl}-3-methyl-4-oxo-2-phenyl-4H-1-benzopyran dihydrochloride**

The title compound was prepared by the method described in Example 5, but using Intermediate XLVII in place of 8-carboxy-3-methyl-4-oxo-2-phenyl-4H-1-benzopyran and 1-(2-chloroethyl)-4-(2-methoxyphenyl)-piperazine instead of 1-(3-chloropropyl)-4-(2-methoxyphenyl)-piperazine. M.p. 193-196°C from ethanol: diethyl ether.

## Example 67

**8-{4-[4-(2-methoxyphenyl)-1-piperazinyl]-butylsulphamoyl}-3-methyl-4-oxo-2-phenyl-4H-1-benzopyran dimethanesulphonate**

The title compound was prepared as described in Example 61 but using 4-[4-(2-methoxyphenyl)-1-piperazinyl]-butylamine instead of 3-[4-(2-methoxyphenyl)-1-piperazinyl]-propylamine. The crude dimethanesulphonate was crystallized first from acetonitrile and then from ethanol. M.p. 172-174°C.

## Example 68

**8-{N-(2-tetrahydropyranyloxy)-3-[4-(2-methoxyphenyl)-1-piperazinyl]-propylcarbamoyl}-3-methyl-4-oxo-2-phenyl-4H-1-benzopyran methanesulphonate hemihydrate**

A solution of 3.6 g of 1-(3-chloropropyl)-4-(2-methoxyphenyl)-piperazine in 30 ml of anhydrous dimethylformamide was added dropwise, under stirring at 0°C, to a mixture of 3.92 g of O-(2-tetrahydropyranyl)-hydroxylamine (prepared as described by R.N. Watrener et al., *Angewandte Chem. Int. Ed.*, 5, 511, 1966). Stirring was continued for 2 hours at 0°C, and then for 12 hours at 110°C. The reaction mixture was cooled to room temperature and dimethylformamide was removed by distillation in vacuo. The residue was rinsed with water and extracted with ethyl acetate. The combined organic layers were washed with water and dried on anhydrous sodium sulphate. The solvent was evaporated off in vacuo to give 4.39 g of 1-[3-(2-tetrahydropyranyloxyamino)-propyl]-4-(2-methoxyphenyl)-piperazine.

**$^1$H-NMR ($CDCl_3$, δ)**

| | |
|---|---|
| 6.50-6.75 | (m, 4H, aromatic protons) |
| 5.20 | (bs, 1H, NH) |
| 4.60 | (m, 1H, O-CH-O) |
| 3.30-4.00 | (m, 5H, $OCH_3$ and tetrahydropyran $CH_20$) |

| | |
|---|---|
| 2.80-3.20 | (m, 6H, piperazine 2 x $CH_2$, alkyl chain $CH_2N$) |
| 2.20-2.80 | (m, 6H, piperazine 2 x $CH_2$, alkyl chain $CH_2N$) |
| 1.30-2.00 | (m, 8H, tetrahydropyran 3 x $CH_2$, alkyl chain C-$CH_2$-C) |

A solution of 2.79 g of 8-chlorocarbonyl-3-methyl-4-oxo-2-phenyl-4H-1-benzopyran in 47 ml of chloroform was added dropwise at room temperature to a mixture of 3.26 g of the compound thus prepared and 1.42 g of potassium carbonate in 47 ml of chloroform. The reaction mixture was stirred for 3 hours, and then diluted with 75 ml of chloroform and washed three times with 1M sodium hydroxide. The organic layer was washed with water, dried on anhydrous sodium sulphate and evaporated to dryness in vacuo. The crude residue was purified by column chromatography on silica gel eluting with ethyl acetate: methanol 98:2. The collected fractions were evaporated to dryness in vacuo to give 2.99 g of pure title compound as a base. The base was dissolved in dichloromethane, and methanesulphonic acid was added to the solution. The solvent was removed by evaporation in vacuo and the crude salt was crystallized from ethyl acetate to yield the title compound, m.p. 159-160°C.

## Example 69

**8-{4-[4-(2-methoxyphenyl)-1-piperazinyl]-butyramido}-3-methyl-4-oxo-2-phenyl-4H-1-benzopyran methanesulphonate hemihydrate**

The title compound was prepared by the method described in Example 38, but using Intermediate XLVIII instead of Intermediate XLIV and stirring for 1 hour at 70°C and 2 hours at 130°C instead of 7 hours at 100°C. After the usual workup, the crude residue was purified by column chromatography on silica gel eluting with ethyl acetate:methanol 95:5. The fractions containing the pure title compound as a base were collected and evaporated to dryness in vacuo. The residue was dissolved in dichloromethane and one equivalent of methanesulphonic acid was added to the solution. After evaporation of the solvent to dryness in vacuo, the crude salt was crystallized from acetone, m.p. 175-176°C.

## Example 70

**E-8-{2-[4-(2-methoxyphenyl)-1-piperazinyl]-ethoxyimino-methyl}-3-methyl-4-oxo-2-phenyl-4H-1-ben zopyran**

A solution of 5.4 g of 8-formyl-3-methyl-4-oxo-2-phenyl-4H-1-benzopyran and 5.13 g of Intermediate LII in 10 ml of chloroform, containing 3Å molecular sieve, was stirred at reflux for 6 hours. The molecular sieve was removed by filtration and the solution was evaporated to dryness in vacuo. The crude product was purified by column chromatography on silica gel, eluting with ethyl acetate:petroleum ether 7:3. Two groups of fractions were collected and evaporated to dryness in vacuo.

The first eluted group of fractions (less polar) contained almost pure title compound; the second group (more polar) was a 1:1 mixture of the E and Z diastereomers, as determined by NMR.

**$^1$H-NMR ($CDCl_3$, $\delta$)**

| | |
|---|---|
| 8.75 | (dd, 0.5H, benzopyran CH in 7, Z) |
| 8.65 | (s, 0.5H, iminic CH, E) |
| 8.30 | (dd, 1H, benzopyran CH in 5, E+Z) |
| 8.15 | (dd, 0.5H, benzopyran CH in 7, E) |
| 8.00 | (s, 0.5H, iminic CH, Z) |
| 7.60-7.75 | (m, 2H, phenyl CH in 2' and 6', E+Z) |
| 7.50-7.60 | (m, 3H, phenyl CH in 3', 4' and 5', E+Z) |
| 7.45 | (dd, 0.5H, benzopyran CH in 6, Z) |
| 7.41 | (dd, 0.5H, benzopyran CH in 6, E) |
| 6.70-7.10 | (m, 4H, phenyl protons, E+Z) |
| 4.41 | (t, 2H, $CH_20$, E+Z) |
| 3.86 | (s, 3H, $CH_30$, E+Z) |
| 3.05-3.20 | (m, 4H, piperazine 2 x $CH_2$, E+Z) |
| 2.70-2.90 | (m, 6H, piperazine 2 x $CH_2$ and $CH_2N$, E+Z) |
| 2.20 | (s, 1.5H, benzopyran $CH_3$ in 3, Z) |
| 2.18 | (s, 1.5H, benzopyran $CH_3$ in 3, E) |

The E diasteromer was crystallized from ethanol:water 2:1 to give 2.5 g of pure title compound, m.p. 107-109°C.

**Example 71**

**8-{N-hydroxy-3-[4-(2-methoxyphenyl)-1-piperazinyl]-propylcarbamoyl}-3-methyl-4-oxo-2-phenyl-4H-1-benzopyran methanesulphonate 0.25 H20**

A solution of 2.04 g of the compound of Example 68 as a base in 104 ml of 1.6N ethanolic hydrogen chloride was stirred for 12 hours at ambient temperature. Ethanol was removed by evaporation in vacuo and the residue was rinsed with 1N sodium hydroxide and dichloromethane. The organic layer was collected, washed with water, dried on anhydrous sodium sulphate and evaporated to dryness in vacuo. One molar equivalent of methanesulphonic acid was added to a solution of the residue in dichloromethane. The solvent was removed and the crude methanesulphonate crystallized from acetone to give 1.02 g of the title compound, m.p. 211-213°C. The product contained 0.25 mole of water.

**Example 72**

**E-8-<2-{2-[4-(2-methoxyphenyl)-1-piperazinyl]-ethylcarbamoyl}-ethenyl>-3-methyl-4-oxo-2-phenyl-4H-1-benzopyran methanesulphonate 1.2 $H_20$**

The title compound was obtained by the method described in Example 61 but using Intermediate IV instead of Intermediate VIII and 2-[4-(2-methoxyphenyl)-1-piperazinyl]-ethylamine instead of the corresponding propylamine, in 1,1,2,2-tetrachloroethylene as solvent. At the end, the reaction mixture was diluted with water and chloroform and washed with 1N aqueous sodium hydroxide, then with water. To the organic layer, after drying on anhydrous sodium sulphate, was added methanesulphonic acid and the solvents were evaporated off in vacuo. The crude product was crystallized twice from isopropanol to give the title compound containing 1.2 molar equivalents of water. M.p. 124-127°C.

**Example 73**

**8-{4-[4-(2-methoxyphenyl)-1-piperazinyl]-butylsulphinyl}-3-methyl-4-oxo-2-phenyl-4H-1-benzopyran methanesulphonate**

The title compound was prepared according to Example 38, but using Intermediate LIV instead of Intermediate XLIV, stirring at 70°C for 3 hours and again at 90°C for 3 hours after adding a catalytic quantity (0.01 equivalents) of potassium iodide. Purification by column chromatography on silica gel, eluting with ethyl acetate:methanol 9:1, gave the title compound as a base. To the crude base, dissolved in dichloromethane, was added one molar equivalent of methanesulphonic acid. After removal of the solvent by evaporation in vacuo, the resulting salt was crystallized from acetone to give the title compound, m.p. 183-184°C.

**Example 74**

**8-{3-[3-(2-methoxyphenoxy)-propylamino]-propylcarbamoyl}-3-methyl-4-oxo-2-phenyl-4H-1-benzopyran methanesulphonate hemihydrate**

The title compound was prepared according to the method described in Example 76 but using 3-(2-methoxyphenoxy)-propyl chloride (prepared as described in B. Willhalm, *Tetrahedron*, 20, 1185, 1964) instead of 2-(2,6-dimethoxyphenoxy)-ethyl bromide. The residue from dichloromethane extraction was purified by column chromatography on silica gel eluting with dichloromethane:methanol:5N methanolic ammonia 9:1:0.3; the pure base was converted into the methanesulphonate, which was crystallized twice from ethyl acetate:acetonitrile 9:1 to give the title compound, melting at (60) 87-90°C.

**Example 75**

**8-{3-[2-(2-methylthiophenoxy)-ethylamino]-propylcarbamoyl}-3-methyl-4-oxo-2-phenyl-4H-1-benzopyran methanesulphonate**

1.85 g of 95% sodium borohydride was added to a solution of 7 g of Intermediate LIX in 70 ml of methanol stirred at 0°C. After stirring for 1 hour at the same temperature, the solvent was removed by evaporation in vacuo. The residue was diluted with water and 2N hydrochloric acid and extracted with ethyl acetate. The organic layer was washed with water, dried on anhydrous sodium sulphate and evaporated to dryness in vacuo, giving 6.6 g of pure 2-(2-methylthiophenoxy)-ethanol as on oil. 8.57 g of p-toluenesulphonyl chloride was added portionwise to a solution of the compound thus prepared in 35 ml of pyridine stirred at 0°C. After stirring for 14 hours at ambient temperature, the reaction mixture was poured into cold 2N hydrochloric acid and extracted with dichloromethane. The organic layer was washed twice with water, dried on anhydrous sodium sulphate

and evaporated to dryness in vacuo yielding 7.8 g of a 3:1 mixture of 2-(2-methylthiophenoxy)ethyl p-toluenesulphonate and 2-(2-methylthiophenoxy)-ethyl chloride (assessed by NMR) as a low melting solid which was used without further purification.

A homogeneous mixture of 3.3 g of the above mixture and 8 g of Intermediate XLIII was kept in an oil bath at 140°C for 20 minutes. After this period the molten mass was cooled to ambient temperature and solidified. The solid residue was rinsed with dichloromethane and 4N sodium hydroxide. The organic layer was washed with water, dried on anhydrous sodium sulphate and evaporated to dryness in vacuo.

The crude product was purified by column chromatography on silica gel eluting with dichloromethane:methanol 9:1 giving 2.07 g of the title compound as a base. This was converted by the usual method into a crude methanesulphonate, which was crystallized first from acetone and then from acetonitrile. M.p. 143-146°C.

## Example 76

**8-{3-[2-(2,6-dimethoxyphenoxy)-ethylamino]-propylcarbamoyl}-3-methyl-4-oxo-2-phenyl-4H-1-benzopyran hydrochloride**

A homogeneous mixture of 3.3 g of 2-(2,6-dimethoxyphenoxy)-ethyl bromide (prepared as described in J. Augstein et al., *J. Med. Chem.*, 8, 356, 1965) and 8.4 g of Intermediate XLIII was heated in an oil bath at 150°C for 10 minutes. The molten mass was cooled to ambient temperature and solidified. The solid residue was rinsed with ethyl acetate and 2N sodium hydroxide. The organic layer was washed with water, dried on anhydrous sodium sulphate and evaporated to dryness in vacuo. The oily residue was purified twice by column chromatography on silica gel eluting firstly with ethyl acetate:methanol:5N methanolic ammonia 97:3:0.3 and then with dichloromethane:methanol: triethylamine 90:10:0.3. This gave 3.3 g of the pure title compound as a base. The crude hydrochloride, obtained by the usual method, was crystallized from acetone followed by acetonitrile. M.p. 179-181°C.

## Example 77

**8-{3-[4-(5-chloro-2-methoxyphenyl)-1-piperazinyl]-propylcarbamoyl}-3-methyl-4-oxo-2-phenyl-4H-1-benzopyran.**

This compound was prepared by the method described in Example 11, but using 1-(5-chloro-2-methoxyphenyl)-piperazine instead of 1-(2-methoxyphenyl)-piperazine and carrying out the reaction for 6 hours instead of 5 hours. Purification was carried out by flash chromatography on silica gel, eluting with chloroform : 5N methanolic ammonia 100:1. The title compound melted at 163-166°C after crystallization from 95% ethanol.

## Example 78

**(E)-8-{4-[4-(2-methoxyphenyl)-1-piperazinyl]-1-butenyl}-3-methyl-4-oxo-2-phenyl-4H-1-benzopyran**

33.4 ml of a 1 molar solution of lithium bis(trimethylsilyl)amide in anhydrous tetrahydrofuran was added dropwise over a period of 15 minutes to a suspension of 6.4 g of 3-hydroxypropyltriphenylphosphonium bromide in 60 ml of anhydrous tetrahydrofuran cooled to -15°C. Then a solution of 4 g of 8-formyl-3-methyl-4-oxo-2-phenyl-4H-1-benzopyran in 40 ml of tetrahydrofuran was added dropwise. The reaction mixture was stirred at 0°C for 30 minutes, then at ambient temperature for 1½ hours.

Quenching with methanol, followed by evaporation to dryness in vacuo, gave a residue which was purified by column chromatography on silica gel eluting with ethyl acetate:petroleum ether 6:4. The collected fractions were evaporated in vacuo giving 4.17 g of 8-(4-hydroxy-1-butenyl)-3-methyl-4-oxo-2-phenyl-4H-1-benzopyran as a E-Z diastereoisomer mixture having a 3.5:1 ratio determined by NMR.

**$^1$H-NMR, 200MHz ($CDCl_3$, $\delta$)**

| | |
|---|---|
| 8.10-8.20 | (d, 1H, benzopyran CH in 5, E+Z) |
| 7.30-7.80 | (m, 7H, other aromatics, E+Z) |
| 6.80;7.00 | (2d, 1H, aryl-CH=, E+Z) |
| 6.41 | (dt, 0.78H, $\underline{CH}$-$CH_2$, E) |
| 5.90 | (dt, 0.22H, $\underline{CH}$-$CH_2$, Z) |
| 3.60-3.80 | (m, 2H, $\underline{CH_2O}$, E+Z) |
| 2.45-2.60 | (m, 2H, CH-$\underline{CH_2}$, E+Z) |
| 2.18 | (s, 3H, benzopyran $CH_3$ in 3, E+Z) |

| | |
|---|---|
| 1.60-1.90 | (sa, 1H, OH, E+Z) |

1.65 g of p-toluenesulphonyl chloride was added to a solution of 2.2 g of the above mixture in 24 ml of anhydrous pyridine was stirred at 0°C. Stirring was continued for 48 hours at the same temperature, and the reaction mixture was then poured into cold 1N hydrochloric acid and filtered by suction. The gummy solid was washed with water and rinsed with dichloromethane. The solution was dried on anhydrous sodium sulphate and evaporated to dryness in vacuo to give 2.30 g of (E,Z)-4-{8-[3-methyl-4-oxo-2-phenyl-4H-1-benzopyranyl}-3-butenyl p-toluenesulphonate having the same diastereoisomer composition as the above Intermediate.

A solution of 2.85 g of the p-toluenesulphonic acid ester above and of 2.98 g of 1-(2-methoxyphenyl)-piperazine in anhydrous dimethylformamide was stirred at ambient temperature for 48 hours. After this period, the mixture was poured into 250 ml of water and extracted with ethyl acetate. The organic layer was washed with water, dried on anhydrous sodium sulphate and evaporated to dryness in vacuo giving a residue which was purified by column chromatography on silica gel eluting with ethyl acetate:petroleum ether 6:4. The collected fractions were evaporated to dryness in vacuo and the crude product was crystallized from 70% ethanol yielding 1.48 g of the title compound that melted at 119-121°C.

**$^1$H-NMR, 200MHz ($CDCl_3$, $\delta$)**

| | |
|---|---|
| 8.14 | (dd, 1H, benzopyran CH in 5) |
| 7.85 | (dd, 1H, benzopyran CH in 7) |
| 7.41-7.70 | (m, 5H, phenyl CHs) |
| 7.34 | (dd, 1H, benzopyran CH in 6) |
| 6.70-7.10 | (m, 5H, aryl-CH= and methoxyphenyl CHs) |
| 6.30-6.50 | (dt, 1H, $J_{trans}$=16.5Hz, $C\underline{H}$-$CH_2$) |
| 3.86 | (s, 3H, $CH_3O$) |
| 3.00-3.15 | (m, 4H, 2 piperazine $CH_2$) |
| 2.50-2.80 | (m, 8H, 2 piperazine $CH_2$, $CHC\underline{H}_2C\underline{H}_2N$) |
| 2.18 | (s, 3H, benzopyran $CH_3$ in 3) |

**Example 79**

**(E)-8-<2-{2-[4-(2-methoxyphenyl)-1-piperazinyl]-ethoxycarbonyl}-ethenyl>-3-methyl-4-oxo-2-phenyl-4H-1-benzopyran methanesulphonate**

The title compound was prepared according to Example 6, but using Intermediate III instead of 8-carboxy-3-methyl-4-oxo-2-phenyl-4H-1-benzopyran. After the usual workup, the residue was crystallized twice from ethanol; the solid obtained was purified by column chromatography on silica gel eluting with chloroform:ethyl acetate 8:2 to give the pure base, which was dissolved in chloroform:ethanol 1:1. Methanesulphonic acid was added to the solution and the solvents were removed by evaporation in vacuo. The crude salt was crystallized from isopropanol yielding the title compound, melting at 193-195°C. This compound contained 0.33 equivalent of isopropanol and 0.25 equivalent of water.

**Example 80**

**8-{2-[4-(2-methoxyphenyl)-1-piperazinyl]-ethylcarbamoylmethyl}-4-oxo-2-phenyl-4H-1-benzopyran methanesulphonate hydrate**

A mixture of 2.8 g of Intermediate XLVII and 1.28 g of 1-hydroxybenzotriazole in 20 ml of anhydrous dimethylformamide was stirred at 0-5°C for 15 minutes. A solution of 1.96 g of dicyclohexylcarbodiimide in 20 ml of anhydrous dimethylformamide was added dropwise to the mixture over a period of about 40 minutes. After stirring for 8 hours at ambient temperature, a solution of 2.24 g of 1-(2-aminoethyl)-4-(2-methoxyphenyl)-piperazine in 15 ml of anhydrous dimethylformamide was added. After 5 hours stirring and overnight standing at the same temperature, the insoluble matter was filtered off and the filtrate was poured into about 300 ml of water and made alkaline by addition of 1N sodium hydroxide. The mixture was extracted with dichloromethane and the organic layer was separated, dried on anhydrous sodium sulphate and evaporated in vacuo. The crude product was purified by flash chromatography on silica gel eluting with chloroform:methanol 95:5. One molar equivalent of methanesulphonic acid was added to the solution of the crude base in ethanol. Diethyl ether was added until the salt crystallized. The salt was then filtered off and recrystallized from ethanol:diethyl ether 1:2 to give 1.15 g of the title compound, m.p. 160-162°C.

**Example 81**

**8-{N-acetyl-3-[4-(2-methoxyphenyl)-1-piperazinyl]-propylcarbamoyl}-3-methyl-4-oxo-2-phenyl-4H-1-benzopyran**

A solution of 2.86 g of Intermediate LVII, 5.04 g of 1-(3-chloropropyl)-4-(2-methoxyphenyl)-piperazine and 2.58 g of anhydrous potassium carbonate in 50 ml of dimethylformamide was stirred at 90°C for 7 hours. After cooling to ambient temperature, the reaction mixture was poured into 500 ml of water and extracted with dichloromethane. The organic layer was washed with water, dried on anhydrous sodium sulphate and evaporated to dryness in vacuo. The residue was purified by column chromatography on silica gel eluting with ethyl acetate:petroleum ether 7:3 to give 1.89 g of the title compound melting at (55) 62-63°C.

**Example 82**

**8-{2-[4-(2-methoxyphenyl)-1-piperazinyl]-ethylsulphonylamino}-3-methyl-4-oxo-2-phenyl-4H-1-benzopyran methanesulphonate**

1.05 ml of 2-chloroethanesulphonyl chloride was added dropwise to a solution of 5 g of 8-amino-3-methyl-4-oxo-2-phenyl-4H-1-benzopyran and 1.4 ml of triethylamine stirred at 0°C. The reaction mixture was stirred at ambient temperature for 2 days. After filtering off the precipitated solids, the solution was evaporated to dryness in vacuo to give a crude residue containing 8-(ethenylsulphonylamino)-3-methyl-4-oxo-2-phenyl-4H-1-benzopyran used without further purification. A mixture of 7.54 g of this residue and 5.8 g of 1-(2-methoxyphenyl)-piperazine and 4.15 g of potassium carbonate in 100 ml of dimethylformamide was stirred at ambient temperature for 4 hours, poured into 600 ml of water and extracted with ethyl acetate. The organic layer was evaporated to dryness in vacuo and the residue was purified by column chromatography on silica gel eluting with petroleum ether:acetone 8:2. The collected fractions were evaporated to dryness in vacuo and crystallized from 70% ethanol to give 0.75 g of the title compound as a base. This solid was dissolved in dichloromethane and one equivalent of methanesulphonic acid was added to the solution. The crude methanesulphonate, obtained by evaporation in vacuo, was crystallized from acetone, yielding 0.6 g of the title compound melting at 202-203°C.

**Example 83**

**8-{3-[4-(2-methoxyphenyl)-1-piperazinyl]-propylthiocarbamoyl}-3-methyl-4-oxo-2-phenyl-4H-1-benzopyran methanesulphonate**

A stirred mixture of 0.8 g 8-formyl-3-methyl-4-oxo-2-phenyl-4H-1-benzopyran, 0.75 g of 1-(3-aminopropyl)-4-(2-methoxyphenyl)-piperazine and 0.14 g of sulphur in 5 ml of pyridine was refluxed for 6 hours. After solvent evaporation in vacuo, the residue was purified by flash chromatography on silica gel, eluting with chloroform. The obtained title compound (as the base) was dissolved in dichloromethane and one equivalent of methanesulphonic acid was added. The title compound was obtained by evaporation in vacuo and crystallization of the residue from acetonitrile. Yield 0.7 g, m.p. 189-190°C.

**Example 84**

**8-{4-[4-(2-methoxyphenyl)-1-piperazinyl]-butylsulphonyl}-3-methyl-4-oxo-2-phenyl-4H-1-benzopyran methanesulphonate . ¼ $H_2O$**

The title compound was prepared according to Example 73, but using Intermediate LX instead of Intermediate LIV. Its purification was performed by column chromatography on silica gel, eluting with ethyl acetate:petroleum ether 7:3. One molar equivalent of methanesulphonic acid was added to a solution of the crude base in dichloromethane. After removal of the solvent by evaporation in vacuo, the resulting salt was crystallized from acetone to give the title compound, m.p. 212-214°C.

**Example 85**

**8-{3-[4-(2-methoxyphenyl)-1-piperazinyl]-propylcarbamoyl}-3-hydroxymethyl-4-oxo-2-phenyl-4H-1-benzopyran. 0.4 ethanol**

A mixture of 3.6 g of Intermediate XCV and 1.65 g of 1-hydroxybenzotriazole in 35 ml of anhydrous dimethylformamide was stirred at 0-5°C for 15 minutes. A solution of 2.5 g of dicyclohexylcarbodiimide in 35 ml of anhydrous dimethylformamide was added dropwise. After stirring for 1 hour at the same temperature, a solution of 1-(3-aminopropyl)-4-(2-methoxyphenyl)-piperazine was added. After stirring for 2 hours at the same

temperature and standing overnight at ambient temperature, the reaction mixture was evaporated to dryness in vacuo. The residue was purified by flash chromatography eluting with a mixture of dichloromethane:methanol 100:3, followed by crystallization from ethanol. 2.5 g of the title compound was obtained, m.p. 152-154°C.

## Example 86

**8-{3-[4-(2-methoxyphenyl)-1-piperazinyl]-propylcarbamoyl}-4-oxo-2-phenyl-4H-1-benzopyran methanesulphonate . ¼ $H_2O$**

3.6 ml of diethyl cyanophosphonate were added dropwise at 0-5°C into a stirred solution of 4 g of 8-carboxy-4-oxo-2-phenyl-4H-1-benzopyran (prepared as described in Da Re et al., *Ber.*, 99, 1962, 1966) and 3.75 g of 1-(3-aminopropyl)-4-(2-methoxyphenyl)-piperazine in 35 ml of anhydrous dimethylformamide. Immediately afterwards, 2.5 ml of triethylamine was added dropwise at the same temperature. After 30 minutes stirring at 0-5°C and 1 hour at ambient temperature, the reaction mixture was poured into 350 ml of 2.5% aqueous sodium carbonate. The precipitate formed was stirred for 1 hour at ambient temperature, collected by suction filtration and crystallized from ethanol. The base of the title compound, thus obtained, was dissolved in dichloromethane and one equivalent of methanesulphonic acid was added. After evaporation in vacuo, a glassy solid was obtained, which was crushed and refluxed for 1 hour in acetone, yielding 5 g of the title compound melting at 191-194°C.

## Example 87

**8-{3-[4-(2-methoxyphenyl)-1-piperazinyl]-propylcarbamoyl}-2,3-dihydro-4-oxo-4H-1-benzopyran methanesulphonate**

A solution of 0.84 ml of thionyl chloride in 17 ml of anhydrous dichloromethane was added dropwise to a solution of 2.0 g of 8-carboxy-2,3-dihydro-4-oxo-4H-1-benzopyran (prepared according to Lichtenberger et al., *Bull.Chem.Soc.Fr.*, 275, 1963) and 1.75 ml of triethylamine in 17 ml of dichloromethane stirred at ambient temperature. Stirring was continued for 1½ hour at the same temperature; after this period the reaction mixture was evaporated to dryness in vacuo to give crude 8-chlorocarbonyl-2,3-dihydro-4-oxo-4H-1-benzopyran. This was used in the method of Example 10 instead of 8-chlorocarbonyl-3-methyl-4-oxo-2-phenyl-4H-1-benzopyran to prepare the base of the title compound, which was purified by column chromatography eluting with ethyl acetate:methanol 85:15. The yield was 1.91 g of the pure base. After dissolution in dichloromethane, acidification with methanesulphonic acid, and evaporation to dryness in vacuo, the resultant crude salt was crystallized from acetonitrile giving 1.57 g of the title compound, melting at 175-177°C.

## Example 88

**8-{3-[4-(2-methoxyphenyl)-1-piperazinyl]-propylcarbamoyl}-4-oxo-4H-1-benzopyran methanesulphonate . 1.25 $H_2O$**

The title compound was prepared by the method described in Example 87 but using Intermediate LXII instead of 8-carboxy-2,3-dihydro-4-oxo-4H-1-benzopyran. The crude methanesulphonate was rinsed with diethyl ether, filtered and repeatedly crystallized from acetonitrile. M.p. 155-157°C.

## Example 89

**8-{3-[4-(2-methoxyphenyl)-1-piperazinyl]-propylcarbamoyl}-6-bromo-3-methyl-4-oxo-2-phenyl-4H-1-benzopyran**

This compound was prepared according to Example 86 but using 8-carboxy-6-bromo-3-methyl-4-oxo-2-phenyl-4H-1-benzopyran (prepared as described in EP 107804) instead of 8-carboxy-4-oxo-2-phenyl-4H-1-benzopyran. It was purified as a base by flash chromatography on silica gel eluting with dichloromethane:methanol 100:3, and crystallized from 95% ethanol. M.p. (150) 154-159°C.

## Example 90

**8-{3-[4-(2-methoxyphenyl)-1-piperazinyl]-propylcarbamoyl}-6-methoxy-3-methyl-4-oxo-2-phenyl-4H-1-benzopyran methanesulphonate**

1.01 ml of diethyl cyanophosphonate and 0.85 ml of triethylamine were added to a solution of 1.7 g of 8-carboxy-6-methoxy-3-methyl-4-oxo-2-phenyl-4H-1-benzopyran (prepared as described in JP 61-15880) and 1.51 g 1-(2-methoxyphenyl)-4-(3-aminopropyl)-piperazine in 20 ml of anhydrous dimethylformamide stirred at

0°C. After stirring for 1 hour at 0°C to ambient temperature, the reaction mixture was poured into a mixture of 100 ml of water and 10 ml of 1N sodium hydroxide. The base of the title compound precipitated; it was filtered off and washed with water. After desiccation it was converted in the usual way to the methanesulphonate, which was crystallized from acetonitrile. Yield 1.7 g; m.p. 185-186°C.

## Example 91

**8-{3-[4-(2-methoxyphenyl)-1-piperazinyl]-propylcarbamoyl}-6-hydroxy-3-methyl-4-oxo-2-phenyl-4H-1-benzopyran methanesulphonate**

0.8 g of the compound prepared in Example 114 and 5.8 ml of 1N sodium hydroxide in 10 ml of methanol were stirred at ambient temperature for 4 hours. After standing overnight, 15 ml of 1N sodium hydroxide and 15 ml of methanol were added and the mixture was stirred at ambient temperature for 1 hour. Methanol was evaporated off in vacuo and water was added to the residue. The suspension was filtered by suction to give 0.48 g of the base of the title compound. This was converted by the usual procedure into its methanesulphonate salt, recrystallized from acetonitrile. M.p. 200-202°C.

## Example 92

**8-{3-[4-(2-methoxyphenyl)-1-piperazinyl]-propylcarbamoyl}-3,6-dimethyl-4-oxo-2-phenyl-4H-1-benzopyran methanesulphonate**

The title compound was prepared according to Example 90 but using 8-carboxy-3,6-dimethyl-4-oxo-2-phenyl-4H-1-benzopyran (prepared as described in Da Re et al., *Arch.Pharm.*, 296, 714, 1963) instead of 8-carboxy-6-methoxy-3-methyl-4-oxo-2-phenyl-4H-1-benzopyran. The crude methanesulphonate was crystallized from acetonitrile and melted at 196-197°C.

## Example 93

**8-{3-[4-(2-methoxyphenyl)-1-piperazinyl]-propylcarbamoyl}-3-methyl-6-nitro-4-oxo-2-phenyl-4H-1-benzopyran**

This product was obtained operating as described in Example 12 but using Intermediate LXVIII instead of 8-chlorocarbonyl-3-methyl-4-oxo-2-phenyl-4H-1-benzopyran and 1,1,2-trichloroethane instead of chloroform. After the usual workup, the crude product was purified by column chromatography eluting with dichloromethane:methanol 98:2. Evaporation in vacuo to dryness of the collected fractions and crystallization from ethanol yielded the title compound, m.p. 159.5-161°C.

## Example 94

**8-{3-[4-(2-methoxyphenyl)-1-piperazinyl]-propylcarbamoyl}-6-amino-3-methyl-4-oxo-2-phenyl-4H-1-benzopyran**

A mixture of 33 g of the compound prepared in Example 93, 109 ml of 1N hydrochloric acid, 105 ml of water and 8.78 g of Raney-Nickel in 950 ml of ethanol was hydrogenated in a Parr apparatus, hydrogen pressure 2 atmospheres, stirring at 40°C for 12 hours. After this period, the catalyst was filtered off and washed with 80% ethanol. The mother liquors were evaporated in vacuo to a volume of 80 ml and filtered. The crude product was washed with water, and suspended in water; 37% hydrochloric acid was added until the pH was 1. The insoluble matter was filtered off by suction and the filtrate was alkalinized by adding 35% sodium hydroxide. The title compound precipitated. It was recovered by filtration and washed with water. Dessiccation yielded 26 g melting at (108) 215-217.5°C for use in Example 95 without further purification. For characterization, 4.7 g of the product were crystallized first from ethanol and then from 85% ethanol giving 3 g of the title compound, m.p. 218-219°C.

## Example 95

**8-{3-[4-(2-methoxyphenyl)-1-piperazinyl]-propylcarbamoyl}-6-acetamido-3-methyl-4-oxo-2-phenyl-4H-1-benzopyran**

The title compound was obtained according to the procedure described in Example 36, but using the compound prepared in Example 94 instead of that prepared in Example 33. The reaction mixture was diluted with water and filtered by suction, washing the solid with water. After desiccation at 80°C, this solid was purified by column chromatography on silica gel eluting with chloroform:methanol 95:5. Evaporation in vacuo of the

collected fractions and crystallization from 95% ethanol of the residue gave the title compound, m.p. (150) 218-220°C.

**Example 96**

**8-{3-[4-(2-methoxyphenyl)-1-piperazinyl]-propylcarbamoyl}-6-ethylamino-3-methyl-4-oxo-2-phenyl-4H-1-benzopyran**

A mixture of 8.42 g of the compound of Example 94, 0.45 ml of acetaldehyde, 0.59 g of 85% sodium cyanoborohydride and 3.3 ml of 4.85N ethanolic hydrogen chloride in 73 ml of methanol was stirred at ambient temperature for 5 days. After this period, the reaction mixture was poured into cold 1.5N sodium hydroxide; the suspension was diluted with water and filtered by suction. After desiccation, the residue was purified by column chromatography on silica gel eluting with chloroform:methanol 100:3. Evaporation in vacuo of the collected fractions yielded 6 g of the compound of Example 94 and 2.67 g of the title compound, which melted at 198-201°C after recrystallization from ethanol.

**Example 97**

**8-{3-[4-(2-methoxyphenyl)-1-piperazinyl]-propylcarbamoyl}-6-dimethylamino-3-methyl-4-oxo-2-phenyl-4H-1-benzopyran**

The title compound was prepared according to Example 35 but using the compound prepared in Example 94 instead of that prepared in Example 33, reacting 10 molar equivalents of 40% formaldehyde instead of 7 molar equivalents and 3 moles of sodium cyanoborohydride instead of 2 moles and stirring at ambient temperature for 18 hours instead of 4½ hours. After the usual workup and purification by column chromatography on silica gel eluting with chloroform:methanol 97:3, evaporation in vacuo of the collected fractions and crystallization of the residue from ethanol yielded the title compound which melted at 183-186°C.

**Example 98**

**8-{3-[4-(2-methoxyphenyl)-1-piperazinyl]-propylcarbamoyl}-7-methoxy-3-methyl-4-oxo-2-phenyl-4H-1-benzopyran**

The title compound was prepared according to the procedure described in Example 87 but using Intermediate LXIX instead of 8-carboxy-2,3-dihydro-4-oxo-4H-1-benzopyran. After the usual workup, the solid residue was purified by column chromatography on silica gel eluting with ethyl acetate:methanol 8:2. The collected fractions were evaporated to dryness in vacuo and the residue was crystallized from acetonitrile to give the title compound, melting at 151-152°C.

**Example 99**

**8-{3-[4-(2-methoxyphenyl)-1-piperazinyl]-propylcarbamoyl}-3-methyl-4-oxo-2-(4-trifluoromethylphenyl)-4H-1-benzopyran methanesulphonate sesquihydrate**

The title compound was prepared following the procedure described in Example 90, but starting from Intermediate LXXII instead of 8-carboxy-6-methoxy-3-methyl-4-oxo-2-phenyl-4H-1-benzopyran. The methanesulphonate melted at (85) 90-120°C (dec), after crystallization from acetonitrile.

**Example 100**

**8-{3-[4-(2-methoxyphenyl)-1-piperazinyl]-propylcarbamoyl}-2-(4-benzoylphenyl)-3-methyl-4-oxo-4H-1-benzopyran methanesulphonate hemihydrate**

The title compound was prepared following the procedure described in Example 90, but starting from Intermediate LXXIV instead of 8-carboxy-6-methoxy-3-methyl-4-oxo-2-phenyl-4H-1-benzopyran. The crude methanesulphonate was crystallized from acetonitrile. M.p. 208-210°C.

**Example 101**

**8-{3-[4-(2-methoxyphenyl)-1-piperazinyl]-propylcarbamoyl}-3-methyl-4-oxo-2-(4-phenoxyphenyl)-4H-1-benzopyran methanesulphonate . 0.25 $H_2O$**

The title compound was prepared following the procedure described in Example 90, but starting from Intermediate LXXVII instead of 8-carboxy-6-methoxy-3-methyl-4-oxo-2-phenyl-4H-1-benzopyran. The crude

methanesulphonate was crystallized from acetonitrile. M.p. 200-202°C.

Example 102

**8-{3-[4-(2-methoxyphenyl)-1-piperazinyl]-propylcarbamoyl}-2,3-dimethyl-4-oxo-4H-1-benzopyran**

This compound was prepared according to Example 86, but using 8-carboxy-2,3-dimethyl-4-oxo-4H-1-benzopyran (prepared according to Da Re, *Farmaco Ed. Sci.*, 11, 678, 1956) instead of 8-carboxy-4-oxo-2-phenyl-4H-1-benzopyran and carrying out the reaction at ambient temperature for 5 hours. It was purified by flash chromatography on silica gel eluting with chloroform: methanol 98:2, and crystallized from acetone. M.p. 155-158.5°C.

Example 103

**8-{3-[4-(2-methoxyphenyl)-1-piperazinyl]-propylcarbamoyl}-2-(t-butyl)-3-methyl-4-oxo-4H-1-benzopyran dihydrochloride dihydrate**

This compound was prepared according to Example 86, but using Intermediate LXXVIII instead of 8-carboxy-4-oxo-2-phenyl-4H-1benzopyran. The base was purified by flash chromatography on silica gel eluting with chloroform:methanol 49:1 and converted into the dihydrochloride in methanol:diethyl ether. It melted at 226-229.5°C after recrystallization from methanol:diethyl ether 1:1.

Example 104

**8-{3-[4-(2-methoxyphenyl)-1-piperazinyl]-propylcarbamoyl}-2-cyclohexyl-3-methyl-4-oxo-4H-1-benzopyran**

This compound was prepared according to Example 86, but using Intermediate LXXIX instead of 8-carboxy-4-oxo-2-phenyl-4H-1-benzopyran, and carrying out the reaction for 5 hours at ambient temperature. It was purified by flash chromatography on silica gel, eluting with chloroform:methanol 49:1, and crystallized from acetonitrile (m.p. 155-157°C).

Example 105

**8-{3-[4-(2-methoxyphenyl)-1-piperazinyl]-propylcarbamoyl}-2-(2-furyl)-3-methyl-4-oxo-4H-1-benzopyran**

This compound was prepared according to Example 86, but using Intermediate LXXXI instead of 8-carboxy-4-oxo-2-phenyl-4H-1-benzopyran, and completing the reaction at ambient temperature for 5 hours. After quenching, the title compound was isolated by extraction with chloroform and purified by flash chromatography on silica gel eluting with chloroform:methanol 49:1, followed by crystallization from acetonitrile. M.p. 151-153°C.

Example 106

**8-{3-[4-(2-methoxyphenyl)-1-piperazinyl]-propylcarbamoyl}-3-methyl-4-oxo-2-thienyl-4H-1-benzopyran**

This compound was obtained according to Example 86, but using Intermediate LXXXIII instead of 8-carboxy-4-oxo-2-phenyl-4H-1-benzopyran. It was purified by stirring in water (completely to remove dimethylformamide), followed by column chromatography on silica gel eluting with chloroform:methanol 49:1, and crystallized from acetonitrile. M.p. 174-175°C.

Example 107

**8-{3-[4-(2-methoxyphenyl)-1-piperazinyl]-propylcarbamoyl}-4-oxo-2-phenyl-4H-1-benzothiopyran**

A mixture of 2.8 g of Intermediate LXXXIV and 3.4 g of 1,1' -carbonyldiimidazole in 60 ml of anhydrous dimethylformamide was stirred under nitrogen at ambient temperature for 1½ hours. 2.7 g of 1-(3-aminopropyl)-4-(2-methoxyphenyl)-piperazine were then added. After further 2 hours stirring at ambient temperature, the reaction mixture was poured into 300 ml of water and extracted with chloroform. The organic layer was dried on anhydrous sodium sulphate and evaporated in vacuo. The residue was purified by flash chromatography on silica gel eluting with chloroform:methanol 49:1, washed with water and crystallized from acetonitrile, yielding 2 g of the title compound melting at 144-146°C.

**Example 108**

**(E)-8-{3-[4-(2-methoxyphenyl)-1-piperazinyl]-propylcarbamoyl}-3-methyl-4-oxo-2-(2-styryl)-4H-1-benzopyran**

This compound was prepared according to Example 86, but using Intermediate LXXXVI instead of 8-carboxy-4-oxo-2-phenyl-4H-1-benzopyran. It was purified by crystallization from acetonitrile. M.p. 191-194°C.

**Example 109**

**8-{3-[4-(2-methoxyphenyl)-1-piperazinyl]-propylcarbamoyl}-3-methyl-2-(4-methylphenyl)-4-oxo-4H-1-benzopyran**

This compound was prepared according to Example 86, but using Intermediate LXXXVII instead of 8-carboxy-4-oxo-2-phenyl-4H-1-benzopyran and completing the reaction at ambient temperature for 4 hours. After quenching, the title compound was isolated by extraction with ethyl acetate, drying on anhydrous sodium sulphate and evaporation in vacuo, followed by rinsing with diethyl ether and crystallization from acetonitrile. M.p. 161-163°C.

**Example 110**

**8-{3-[4-(2-methoxyphenyl)-1-piperazinyl]-propylcarbamoyl}-2-(4-methoxyphenyl)-3-methyl-4-oxo-4H-1-benzopyran**

This compound was prepared according to Example 86, but using 8-carboxy-2-(4-methoxyphenyl)-3-methyl-4-oxo-4H-1-benzopyran (prepared as described in EP 108986) instead of 8-carboxy-4-oxo-2-phenyl-4H-1-benzopyran, and completing the reaction at ambient temperature for 3½ hours. It was purified by flash chromatography on silica gel eluting with chloroform:methanol 49:1, followed by crystallization from acetonitrile. M.p. 158-161°C.

**Example 111**

**8-{3-[4-(2-methoxyphenyl)-1-piperazinyl]-propylcarbamoyl}-2-(4-fluorophenyl)-3-methyl-4-oxo-4H-1-benzopyran**

This compound was prepared according to Example 86, but using Intermediate LXXXIX instead of 8-carboxy-4-oxo-2-phenyl-4H-1-benzopyran. It was purified by flash chromatography on silica gel eluting with chloroform:methanol 100:2 to 100:6 and crystallized from 95% ethanol. M.p. 166-168°C.

**Example 112**

**8-{3-[4-(2-methoxyphenyl)-1-piperazinyl]-propylcarbamoyl}-6-methanesulphonylamino-3-methyl-4-oxo-2-phenyl-4H-1-benzopyran hydrochloride**

0.032 ml of methanesulphonyl chloride in 1 ml of dimethylformamide was added dropwise over a period of 10 minutes to a solution of 0.21 g of the compound of Example 94 and 0.062 ml of triethylamine in 4 ml of dimethylformamide, stirred at -20°C. Stirring was continued at the same temperature for 3½ hours. After this period, the reaction mixture was poured into water and the suspension was filtered by suction, yielding 0.1 g of the title compound which was recrystallized from 80% ethanol. M.p. 272-275°C.

**Example 113**

**8-{3-[4-(2-methoxyphenyl)-1-piperazinyl]-propylcarbamoyl}-3-methyl-2-(4-nitrophenyl)-4-oxo-4H-1-benzopyran**

The title compound was prepared following the procedure described in Example 90 but starting from Intermediate XCVIII instead of 8-carboxy-6-methoxy-3-methyl-4-oxo-2-phenyl-4H-1-benzopyran. After stirring for 1 hour at ambient temperature, the reaction mixture was poured into cold 2% sodium carbonate solution and the precipitated solid was collected by suction filtration. After desiccation and crystallization from ethanol, the title compound melted at (60) 185-187°C.

**Example 114**

**8-{3-[4-(2-methoxyphenyl)-1-piperazinyl]-propylcarbamoyl}-6-diethoxyphosphonyloxy-3-methyl-4-oxo-**

**2-phenyl-4H-1-benzopyran**

The title compound was prepared following the procedure described in Example 90, but starting from Intermediate LXIII instead of 8-carboxy-6-methoxy-3-methyl-4-oxo-2-phenyl-4H-1-benzopyran and using 2 equivalents of diethyl cyanophosphate instead of 1.1 equivalents. Filtration from water yielded the title compound melting at 48-50°C. The title compound may be regarded as a prodrug of 8-{3-[4-(2-methoxyphenyl)-1-piperazinyl]-propylcarbamoyl}-6-hydroxy-3-methyl-4-oxo-2-phenyl-4H-1-benzopyran

## Example 115

**8-{3-[4-(2-methoxyphenyl)-1-piperazinyl]-propylcarbamoyl}-3-methyl-4-oxo-2-trifluoromethyl-4H-1-benzopyran methanesulphonate**

Operating as described in Example 90, but starting from Intermediate C instead of 8-carboxy-6-methoxy-3-methyl-4-oxo-2-phenyl-4H-1-benzopyran, the crude base of the title compound was obtained. After the usual workup by extraction with ethyl acetate, the residue was purified by column chromatography on silica gel eluting with ethyl acetate:methanol 9:1. It was converted into its methanesulphonate by the usual procedure and recrystallized from ethyl acetate. M.p. 145-148°C.

## Example 116

**8-{N-methyl-3-[4-(2-methoxyphenyl)-1-piperazinyl]-propylcarbamoyl}-3-methyl-4-oxo-2-phenyl-4H-1-benzopyran hydrochloride**

This compound was prepared according to Example 12, but using Intermediate CI instead of 3-[4-(2-methoxyphenyl)-1-piperazinyl]-propylamine. The crude base was purified by flash chromatography on silica gel, eluting with chloroform:5N methanolic ammonia 100:1, and transformed into the hydrochloride in the usual way. M.p. 195-198°C, after crystallization from acetone.

## Example 117

**7-{3-[4-(2-methoxyphenyl)-1-piperazinyl]-propylcarbamoyl}-2-benzoyl-3-ethyl-benzo[b]furan**

This compound was prepared according to Example 86 but using Intermediate CIII instead of 8-carboxy-4-oxo-2-phenyl-4H-1-benzopyran-8-carboxylic acid, and completing the reaction at ambient temperature for 4 hours. It was purified by crystallization from ethanol. M.p. 165-166°C.

## Example 118

**8-{3-[4-(2-methoxyphenyl)-1-piperazinyl]-propylcarbamoyl}-2-(4-biphenylyl)-3-methyl-4-oxo-4H-1-benzopyran.**

The title compound was prepared according to Example 86 but using Intermediate CVI instead of 8-carboxy-4-oxo-2-phenyl-4H-1-benzopyran. The reaction lasted 20 hours at room temperature. The base was purified by crystallization from ethanol (m.p. 164-166°C).

## Example 119

**8-{3-[4-(2-methoxyphenyl)-1-piperazinyl]-propylcarbamoyl}-3-methyl-4-oxo-2-(3-pyridyl)-4H-1-benzopyran.**

A mixture of 6.2 g of methyl 3-propionyl-salicylate and 5.8 g of nicotinoyl chloride hydrochloride in 18 ml of anhydrous pyridine was stirred and heated at 100°C for 2 hours under nitrogen. 16 ml of triethylamine was then added and heating was continued for 1 hour at the same temperature. The reaction mixture was cooled to room temperature and poured into 600 ml of water. The precipitate was collected by suction and washed with water, yielding 5.4 g of methyl 2-hydroxy-3-(2-nicotinoylpropionyl)-benzoate, which was used without purification in the next step. 3.4 g of the compound thus prepared was heated at 100°C for 1½ hours after dissolution in a mixture containing 15 ml of acetic acid and 1 ml of 37% hydrochloric acid. After cooling to room temperature, the mixture was poured into 150 ml of water and extracted with ethyl acetate. The organic phase was washed with 5% aqueous sodium hydrogen carbonate followed by water, dried on anhydrous sodium sulphate and evaporated in vacuo, yielding 1.3 g of crude 8-methoxycarbonyl-3-methyl-4-oxo-2-(3-pyridyl)-4H-1-benzopyran.

1 g of this ester was dissolved in 9 ml of methanol and 15 ml of 1,4-dioxane and slowly added with 1.7 ml of 10N sodium hydroxide, maintaining the temperature between 20 and 25°C. After 1 hour at 50°C, the reaction

mixture was poured into 150 ml of water and extracted with ethyl acetate. The aqueous layer was acidified with 1N hydrochloric acid. The precipitate was collected by suction, yielding 0.6 g of 8-carboxy-3-methyl-4-oxo-2-(3-pyridyl)-4H-1-benzopyran, which was used without purification in the next step.

The title compound was prepared according to Example 86 but using the acid thus prepared instead of 8-carboxy-4-oxo-2-phenyl-4H-1-benzopyran and carrying out the reaction for 2 hours at room temperature. The base was purified by flash chromatography on silica gel eluting with chloroform:methanol 98:2, followed by crystallization from acetone. Yield: 0.15 g, m.p. 134.5-137°C.

**Example 120**

**8-{3-[4-(2-acetoxyphenyl)-1-piperazinyl]-propylcarbamoyl}-3-methyl-4-oxo-2-phenyl-4H-1-benzopyran.**

1 g of compound of Example 59 and 0.32 g of 4-dimethylaminopyridine were dissolved in 10 ml of dichloromethane. 0.15 ml of acetyl chloride, was slowly added, maintaining the temperature between 8 and 10°C. After 2 hours at ambient temperature, the reaction mixture was poured into 70 ml of water and extracted with dichloromethane. The organic layer was washed with 5% aqueous sodium hydrogen carbonate followed by water, dried on anhydrous sodium sulphate and evaporated to dryness in vacuo. The crude base was purified by flash chromatography on silica gel eluting with ethyl acetate:methanol 9:1, followed by crystallization from ethanol. Yield: 0.74 g of the title compound, m.p. 120-123°C.

**Example 121**

**8-{3-[4-(2-methylaminocarbonyloxyphenyl)-1-piperazinyl]-propylcarbamoyl}-3-methyl-4-oxo-2-phenyl-4H-1-benzopyran.**

3 g of compound of Example 59 and 1.8 ml of methyl isocyanate were dissolved in 30 ml of dry dimethylformamide and stirred at room temperature for 24 hours. The mixture was diluted with water, stirred for 2 hours, and then filtered under suction. The crude base was purified by flash chromatography on silica gel eluting with chloroform:5N methanolic ammonia 100:3. The title compound melted at 132-135°C after crystallization from ethanol.

**Example 122**

**8-{3-[4-(2-methoxyphenyl)-1-piperazinyl]-propylcarbamoyl}-6-acetoxy-3-methyl-4-oxo-2-phenyl-4H-1-benzopyran.**

0.17 ml of acetyl chloride was added dropwise over a period of 5 minutes, under stirring at 0°C, to a solution of 1 g of the compound of Example 91 and 0.32 ml of triethylamine in 36 ml of chloroform. After 2 hours stirring at the same temperature, the reaction mixture was diluted with dichloromethane and water. The organic layer was separated, washed with water, dried on anhydrous sodium sulphate and evaporated to dryness in vacuo. Crystallization of the residue from acetonitrile yielded 0.8 g of the title compound, melting at 148-149°C.

**Example 123**

**(R,S)-8-{3-[4-(2-methoxyphenyl)-1-piperazinyl]-propylcarbamoyl}-2,3-dihydro-4-hydroxy-4H-1-benzo--pyran methanesulphonate.**

The title compound was prepared by the method described in Example 17 but starting from compound of Example 87 instead of the compound of Example 1. The reaction mixture was diluted with water and stirred for 15 minutes; it was then extracted with ethyl acetate. Usual work up gave a crude product, which was purified by flash chromatography on silica gel eluting with dichloromethane:methanol 95:5. Evaporation in vacuo of the collected fraction yielded the pure base, which was converted into the methanesulphonate and crystallized from acetonitrile. M.p. 172-175°C.

**Example 124**

**8-{3-[4-(2-methoxyphenyl)-1-piperazinyl]-propylcarbamoyl}-2-(4-aminophenyl)-3-methyl-4-oxo-4H-1-benzopyran**

2.22 g of the compound of Example 113 and 0.56 g of Raney-Nickel in 96 ml of ethanol and 4.8 ml of acetic acid was hydrogenated in a Parr apparatus (Hydrogen pressure = 1 atm) at room temperature. After shaking for 6 hours, the catalyst was filtered off. The filtrate was made alkaline with 3N sodium hydroxide and diluted with water. After standing for 2 days, the precipitated title compound was collected by suction filtration, washed

with water, desiccated and recrystallized first from ethyl acetate and then from ethanol. Yield: 1.5 g, m.p. 192-194°C).

**Example 125**

**8-{3-[4-(2-methoxyphenyl)-1-piperazinyl]-propylcarbamoyl}-2-(4-acetylaminophenyl)-3-methyl-4H-1-benzopyran.**

The title compound was prepared by the method described in Example 36, but using the compound prepared in Example 124 instead of that prepared in Example 33. It was purified by crystallization from 95% ethanol. M.p. 207-209°C.

**Example 126**

**8-{3-[4-(2-methoxyphenyl)-1-piperazinyl]-propylcarbamoyl}-2-(4-hydroxyphenyl)-3-methyl-4H-1-benzopyran dihydrochloride monohydrate.**

The title compound was prepared by the method described in Example 86 but using Intermediate CVII instead of 8-carboxy-4-oxo-2-phenyl-4H-1-benzopyran, carrying out the reaction for 14 hours at ambient temperature and using hexamethylphosphoramide as co-solvent. The isolated diethylphosphonyl ester of the title compound was hydrolized by alkaline treatment followed by neutralization with dilute hydrochloric acid. The crude base was extracted with chloroform. The organic layer was washed with water and evaporated in vacuo. The salt was prepared by addition of ethanolic hydrogen chloride to an acetone solution of the base, evaporating to dryness and rinsing with acetone. M.p. 193-205°C.

**Example 127**

**8-{3-[4-(2-methoxyphenyl)-1-piperazinyl]-propylcarbamoyl}-2-phenyl-4,$N^1$,$N^4$-trioxo-4H-1-benzothiopyran monohydrate.**

0. 32 ml of 30% hydrogen peroxide was added to 0. 8 g of the compound of Example 107 in 15 ml of acetic acid. The mixture was stirred at 50°C for 3 hours. 0.48 ml of 30% hydrogen peroxide was added to the mixture in three equal portions at 2 hour intervals. After cooling, the mixture was poured into 240 ml of water, neutralized (pH 7) with 5% aqueous sodium hydrogen carbonate and extracted with chloroform. The organic layer was washed with water, dried on anhydrous sodium sulphate and evaporated in vacuo, yielding 0.18 g of the title compound, melting at 172-175°C after crystallization from acetonitrile.

**Example 128**

**7-{3-[4-(2-methoxyphenyl)-1-piperazinyl]-propylcarbamoyl}-2-phenyl-benzo[b]furan.**

The title compound was prepared by the method described in Example 86 but using 7-carboxy-2-phenylbenzo[b]furan (prepared as described in EP 0306226) instead of 8-carboxy-4-oxo-2-phenyl-4H-1-benzopyran, and carrying out the reaction for 1½ hours at ambient temperature. It was purified by crystallization from carbon tetrachloride. M.p. 132-136°C.

**Example 129**

**8-{N-methyl-3-[4-(2-methoxyphenyl)-1-piperazinyl]-propylsulfamoyl}-3-methyl-4-oxo-2-phenyl-4H-1-benzopyran methanesulphonate.**

2.29 g of Intermediate VIII was added portionwise under stirring at 0°C to a solution of 1.5 g of Intermediate CI and 0.95 of triethylamine in 30 ml of chloroform. After stirring for 2 hours at ambient temperature, the reaction mixture was diluted with dichloromethane, water and 0.5 N sodium hydroxide. The organic layer was washed with water, dried on anhydrous sodium sulphate and evaporated to dryness in vacuo. The residue was purified by flash chromatography on silica gel eluting with ethyl acetate:methanol 96:4. Evaporation in vacuo of the collected fractions yielded the pure title compound as a base. This was converted by the usual method into its methanesulphonate salt, which was crystallized from ethyl acetate yielding 2.75 g, melting at 135-141°C (dec.).

**Example 130**

**8-{N-methyl-4-[4-(2-methoxyphenyl)-1-piperazinyl]-butylsulfamoyl}-3-methyl-4-oxo-2-phenyl-4H-1-ben-**

**zopyran methanesulphonate.**

The title compound was obtained by the method described in Example 129 but using Intermediate CVIII instead of Intermediate CI. It was crystallized from acetonitrile. M.p. 173-175°C.

## PHARMACOLOGICAL DATA

### Methodology

Male Sprague Dawley rats [Crl: CD' BR] of 200-300 g b.w., female Albino Swiss mice [Crl: CD-1 (ICR) BR] 20-30 g b.w., and male Beagle dogs (10-12 kg b.w.) were obtained from Charles River, Italy and Nossan (Correzzana, Milan, Italy), respectively. Animals were housed with free access to food and water and maintained on forced light-dark cycle at 22-24°C until the day of experiments.

Acute toxicity

The acute toxicity of synthesized compounds was evaluated in female albino Swiss mice after intraperitoneal and oral administration. Four logarithmic scaled doses of the compounds were dissolved or suspended in 0.5% Methocel and administered in a volume of 10 ml/kg to groups of 4 mice/dose. Mortality was recorded 7 days after the administration. Data analysis: the $LD_{50}$ values and their fiducial limits were calculated according to the method of Weil (*Biometrics*, 8, 249, 1952).

Receptor Binding studies:

- [$^3$H]prazosin binding ($\alpha_1$ receptors)

Rat cerebral cortices were homogenized in 50 volumes of original wet weight of ice-cold 50 mM Tris-HCl buffer pH 7.4. The homogenates were centrifuged at 48,000 x g for 10 minutes, and the pellets were resuspended in the same volume of ice-cold buffer, centrifuged and resuspended two more times. The final pellets obtained were resuspended in the same volume of buffer and incubated according to the conditions reported in the table below.

The foregoing receptor binding studies, as well as the experimental data on dogs reported below, establish compounds of the invention as $\alpha_1$-blockers, i.e. to be within a class of substances widely used as antihypertensive and anti-BPH agents. See, e.g., Frishman, W.H. et al., *Medical Clinics of N. America*, 72, 427, 1988 and references cited therein.

- [$^3$H]8-OH-DPAT binding ($5HT_{1A}$ receptors)

Rat hippocampi were homogenized in 50 volumes of original wet weight of ice-cold 50 mM Tris-HCl buffer pH 7.4. The homogenates were centrifuged at 48,000 x g for 10 minutes, and the pellets were resuspended in the same volume of ice-cold buffer, incubated for 10 minutes at 37°C, centrifuged and resuspended two more times. The final pellets obtained were resuspended in the same volume of buffer and incubated according to the conditions reported in the table below. These receptor binding studies serve to establish compounds of the present invention as ligands for the $5HT_{1A}$ receptor. As previously reported, compounds that are $5HT_{1A}$ ligands exert anxiolytic and antidepressant effects in animals and humans (Hamon, M. et al., *Ann. N.Y. Acad. Sci.*, 600, 114, 1990; Traber J. et al., *T.I.P.S.*, 8, 437, 1987)

| RECEPTOR/LIGAND | Receptor binding studies $\alpha_1$ adrenergic [$^3$H]prazosin | 5-$HT_{1A}$ serotonergic [$^3$H]8-OH-DPAT |
|---|---|---|
| Conditions | | |
| [nM] ligand | 0.35 | 1.0 |
| preparation (c.m.p) | 1 ml | 1 ml |
| | 10 mg/ml | 10 mg/ml |
| incubation buffer* | Tris HCl | Tris HCl |
| | 50 mM | 50 mM |
| | pH 7.4 | pH 7.4 |
| non-specific binding | prazosin | 5-HT |
| | 2 μM | 10 μM |
| incubation | 25°C | 25°C |
| | 30 min | 30 min |

c.m.p. = crude membranes preparation;
* = containing ascorbic acid 1% and pargyline 10 μM

The incubations were terminated after the appropriate time (see table) by rapid filtration through Whatman GF/B filters using a Brandel cell harvester. The filters were washed twice with 15 ml of ice-cold buffer (see table). The radioactivity retained on the filters was determined by liquid scintillation counting. Nonspecific binding (which amounted generally to 10-30%) was evaluated by adding high concentrations of the specific displacers (see table). All the compounds were initially tested at $1x10^{-6}$ M concentration, and in the presence of significant displacing activity, a complete competition curve was generated (down to a concentration of $10^{-11}$ M). All the samples were run in triplicate.

The competition curves were always analyzed (to evaluate the $IC_{50}$ values) by non linear curve fitting of the logistic equation according to the method reported by De Lean et al. (*Am. J. Physiol.*, 235, E97, 1978), utilizing the ALLFIT program [publically available from the National Institutes of Health (N.I.H.) Bethesda, Maryland, USA] written for the IBM PC.

$K^+$-induced contractions of rat bladder strips:

The whole bladder of the rat was removed and immediately placed in Krebs solution warmed at 37°C. Strips of detrusor muscle (20-30 mm long, 1-2 mm wide), were cut from the dome of the bladder. Each strip was placed in a 10 ml organ bath and connected, under a constant load of 1 g, to an isometric strain gauge (DY-1 Basile, Comerio, Varese, Italy). Contractions were recorded by means of a Basile 7070 polygraph. After a 60 minutes equilibration period the strips were exposed to 80 mM KCl (final concentration). This produced a rapid phasic contraction followed by a slow ensuing and sustained tonic component. When the tonic contraction was stable, the strips were washed and 30 minutes later a new contraction was induced. After having recorded two or more reproducible responses, one concentration of the tested drugs was added to the bath and 30 minutes later a new contraction was induced. The experimental groups consisted of at least two preparations taken from different animals for each concentration of drug tested. The $IC_{50}$ values of inhibition of agonist-induced contractions were evaluated by linear regression analysis.

Effects on Urethral Contractions and Blood Pressure in Dogs

The experiments were performed according to the method of Imagawa et al. (*J. Pharmacol. Methods*, 22, 103-111, 1989), with substantial modifications, as follows: Adult male beagle dogs, weighing 8-10 Kg, were

anaesthetized with pentobarbital sodium (30 mg/Kg i.v. and 2 mg/Kg/h i.v.), intubated and spontaneously ventilated with air room. In order to monitor systemic blood pressure (BP), a PE catheter was introduced into the aortic arch through the right common carotid artery.

A collateral of the left femoral vein was cannulated for infusion of anaesthetic, and the right femoral vein was cannulated for administration of drugs. For intrarterial (i.a.) injection of noradrenaline (NA), a PE catheter was introduced into the lower portion of abdominal aorta via the right external iliac artery. Through such procedure, NA was selectively distributed to the lower urinary tract. Via a midline laparotomy, the urinary bladder and proximal urethra were exposed. In order to prevent the filling of the bladder, the two ureters were cannulated and the urine led outside. In order to record the prostatic urethral pressure, a Mikro-tip catheter (6 F) was introduced into the bladder via the external urethral meatus, and withdrawn until the pressure transducer was positioned in the prostatic urethra. A ligature was secured between the neck of the bladder and urethra to isolate the response of the latter and avoid any interaction with the bladder. Another ligature was put around the Mikro-tip catheter at the external urethral meatus, to secure the catheter itself. After a stabilizing period following surgical procedure (30 min), in which arterial and prostatic urethral pressure were continuously monitored as basal values, i.a. administration of NA was made at intervals of 10 min. The dose of NA chosen was such to produce an increase of at least 100% in urethral pressure. The test compounds were administered i.v. in a cumulative manner with intervals of 15-20 min between administrations. I.a. injections of NA were repeated approximately 5 min. after every dosing of test compound. Dose response curves were constructed computing the percentage inhibition to the increase in urethral pressure (NA induced), and the percentage drop in blood pressure produced by the test compound. $ED_{25}$ for diastolic blood pressure (dose inducing a 25% decrease) and $ID_{50}$ (dose inducing a 50% inhibition of NA-induced increase in urethral pressure) were computed by means of linear regression analysis.

## Results

Compounds as prepared in the Examples were tested according to the methods reported above, and the results are given in the Tables below, together with comparative results for the reference standards used. Compounds having receptor affinity ($IC_{50}$ values) lower than about 500 nM are generally considered to have good affinity. Compounds with $IC_{50}$ values less than 100 nM are generally preferred.

**TABLE I**

| Compound Example No. | Receptor Binding $IC_{50}$ (nM) $\alpha_1$ | 5-$HT_{1a}$ | Acute Toxicity in Mice $LD_{50}$ (mg/kg) i.p. | p.o. | K+ Stimulation of Rat Bladder $IC_{50}$ (μM) Contractions Phasic | Tonic |
|---|---|---|---|---|---|---|
| 4 | 550 | 55 | 346 | 1732 | | |
| 5 | 20 | 19 | 621 | >3000 | | |
| 6 | 107 | 1000 | 233 | | | |
| 7 | 86 | 155 | 384 | 1915 | | |
| 8 | 66 | 111 | >500 | 1915 | | |
| 11 | 29 | 9 | 247 | 297 | 2.9 | 3.0 |
| 13 | 68 | 229 | >1000 | >3000 | 10.0 | 10.0 |
| 14 | 61 | 6 | 140 | 559 | | |
| 15 | 8 | 131 | 306 | 496 | | |
| 16 | 220 | 1050 | 345 | 778 | 1.6 | 2.2 |
| 17 | 59 | 910 | 299 | 608 | 8.8 | 3.8 |
| 18 | 270 | >1000 | 457 | 3000 | | |
| 19 | 165 | 340 | >1000 | >3000 | | |
| 20 | 169 | 85 | 297 | 594 | 2.7 | 2.5 |
| 21 | 17 | 33 | 297 | 566 | | |
| 22 | 117 | 48 | >500 | >2000 | | |
| 24 | 690 | 212 | >1000 | >3000 | | |
| 26 | 270 | >1000 | >500 | >2000 | | |
| 27 | 23 | 124 | 399 | >3000 | 1.0 | 0.8 |
| 28 | 120 | 96 | 203 | 1127 | | |
| 29 | 86 | 45 | 730 | >3000 | 10.0 | 10.0 |
| 32 | 119 | 46 | 301 | >2000 | 10.0 | >10 |
| 33 | 17 | 38 | 399 | >2000 | 10.0 | 10.0 |
| 34 | 30 | 34 | >500 | | 2.8 | 3.6 |
| 35 | 15 | 8 | 329 | 959 | | |
| 36 | 18 | 54 | >500 | >2000 | | |
| 37 | 32 | 77 | >500 | >2000 | | |
| 38 | 20 | 344 | >500 | >2000 | | |
| 39 | 90 | 170 | >1000 | >3000 | | |
| 40 | 75 | 83 | 140 | 349 | 0.5 | 0.9 |
| 41 | 43 | 53 | 399 | 2241 | 0.7 | 1.4 |
| 42 | 111 | 39 | 459 | 2163 | 10.0 | 10.0 |
| 43 | 166 | >1000 | | | | |
| 44 | 685 | 201 | 84 | 399 | 0.6 | 0.5 |
| 45 | 15 | 106 | 329 | 1727 | | |
| 46 | 86 | 23 | | | | |
| 47 | 36 | 23 | 330 | 1047 | 2.7 | 5.2 |
| 48 | 104 | 5 | 500 | 1914 | | |
| 49 | 152 | 9 | 432 | >2000 | | |
| 50 | 39 | 300 | 211 | 299 | | |
| 51 | 22 | 84 | >500 | >2000 | | |
| 52 | 89 | 2 | 127 | 224 | | |
| 53 | 7 | 41 | 127 | 1020 | | |
| 54 | 35 | 143 | 106 | 421 | | |
| 55 | 291 | >1000 | 128 | ≥2000 | | |
| 56 | 25 | 748 | >500 | | | |

[N.B. Table 1 is continued on the next page]

**TABLE I (continued)**

| Compound Example No. | Receptor Binding $IC_{50}$ (nM) $\alpha_1$ | 5-$HT_{1a}$ | Acute Toxicity in Mice $LD_{50}$ (mg/kg) i.p. | p.o. | K+ Stimulation of Rat Bladder $IC_{50}$ ($\mu$M) Contractions Phasic | Tonic |
|---|---|---|---|---|---|---|
| 57 | 69 | >1000 | 500 | >2000 | | |
| 58 | ≥1000 | 126 | 258 | 508 | | |
| 59 | 5 | 9 | >500 | 278 | | |
| 60 | 25 | 45 | 203 | 329 | | |
| 61 | 36 | 11 | 315 | 592 | | |
| 62 | 252 | 278 | 128 | 344 | | |
| 63 | 194 | 99 | 309 | 479 | | |
| 64 | 40 | 11 | | | | |
| 65 | 113 | 26 | | | | |
| 66 | 57 | 56 | | 601 | | |
| 67 | 4 | 4 | 508 | 1868 | | |
| 68 | 29 | 13 | | | | |
| 69 | 16 | 87 | 344 | | | |
| 70 | 53 | 85 | >500 | 1868 | | |
| 71 | 108 | 69 | | | | |
| 72 | 145 | 111 | 479 | >2000 | | |
| 73 | 6 | 89 | 202 | 462 | | |
| 74 | 178 | 482 | | | | |
| 75 | 147 | 15 | | | | |
| 78 | 18 | 116 | | | | |
| 79 | 77 | 141 | >500 | >2000 | | |
| 80 | 28 | 162 | | | | |
| 81 | 13 | 81 | | | | |
| 83 | 30 | 5 | | | | |
| 84 | 5 | 2 | | | | |
| 85 | 16 | 14 | | | | |
| 86 | 48 | 20 | | | | |
| 87 | 119 | 107 | 237 | >500 | | |
| 88 | 216 | 11 | | | | |
| 93 | 47 | 38 | | | | |
| 94 | 39 | 66 | | | | |
| 98 | 103 | 12 | | | | |
| 99 | 44 | 5 | | | | |
| 102 | 56 | 18 | | | | |
| 104 | 9 | 15 | | | | |
| 107 | 37 | 16 | | | | |
| 110 | 26 | 15 | | | | |
| Flavoxate | >>1000 | >>1000 | 385 | 808 | 13 | 13 |

**TABLE II**

Effects on Urethral Contractility and Blood Pressure in Dogs

| Compound<br>Example No. | Urethra<br>$ED_{50}$ (μg/kg) | DBP<br>$ED_{25}$ (μg/kg) | DBP/Urethra<br>ratio |
|---|---|---|---|
| 5 | 37.0 | 1074 | 29.0 |
| 11 | 1.4 | 390 | 278.6 |
| 13 | 16.0 | 215 | 13.4 |
| 17 | 10.0 | 6.2 | 0.6* |
| 21 | 6.6 | 127 | 19.2 |
| 27 | 3.2 | 9.8 | 3.1* |
| 40 | 11.0 | 152 | 13.8 |
| 41 | 57.0 | 745 | 13.1 |
| 42 | 31.0 | 404 | 13.0 |
| 45 | 16.4 | 186 | 11.3 |
| 47 | 35.0 | 530 | 15.1 |
| Prazosin | 3.6 | 6.6 | 1.8* |
| Terflavoxate | >10000 | 6060 | - |

Urethra: active dose in inhibiting by 50% the noradrenaline induced contraction of urethra

DBP: active dose in lowering diastolic blood pressure by 25%

DBP/urethra: ratio between the active doses (selectivity index)
* non-selective: substantial effect on both urethra and DBP

Effective Amounts

The following represent guidelines to effective oral, parenteral or intravenous dose ranges expressed in mg/kg of body weight per day for the following uses:

(a) In obstructive disorders of the lower urinary tract:

| | |
|---|---|
| General | 0.001 - 20 |
| Preferred | 0.05 - 1 |
| Most Preferred** | 0.3 |

(b) As antihypertensives:

| | |
|---|---|
| General | 0.01 - 20 |
| Preferred | 0.1 - 5 |
| Most Preferred** | 1 |

(c) As anxiolytics - antidepressants:

| | |
|---|---|
| General | 0.01 - 20 |
| Preferred | 0.05 - 5 |
| Most Preferred** | 0.5 |

(d) As bladder spasmolytics:

| | |
|---|---|
| General | 0.01 - 20 |
| Preferred | 0.02 - 10 |
| Most Preferred** | 2 |

** Most preferred values refer to oral dosing. Intravenous dosages should be 10 to 100 fold lower.

Patients in need of treatment bu the present compounds and compositions also include humans that have one or more depression symptoms (as defined, e.g. in Harrison's Principles of Internal Medicine, XII Ed., McGraw-Hill, Inc., p.2124) or humans that display anxiety symptoms (Harrison's, *supra*, pp.2131-2134).

Selective use dosages, i.e. dosages that are active in the lower urinary tract without a substantial effect on the blood pressure depend on the particular compound employed but, generally, up to four times the $ED_{50}$ of a selective compound can be administered without substantial effect on blood pressure. Further refinements and optimization of dosages are possible using no more than routine experiments.

The active compounds of the invention may be orally administered, for example, with an inert diluent or with an edible carrier, or they may be enclosed in gelatin capsules, or they may be compressed into tablets. For the purpose of oral therapeutic administration, the active compounds of the invention may be incorporated with excipients and used in the form of tablets, troches, capsules, elixirs, suspensions, syrups, wafers, chewing gum and the like. These preparations should contain at least 0.5% of active compounds, but the amount of active ingredient may be varied depending upon the particular form and may conveniently be between 5% to about 70% of the weight of the unit. The amount of active compound in such compositions is such that a suitable dosage will be obtained although the desired dosage can be obtained by administering a plurality of dosage forms. Preferred compositions and preparations according to the invention are prepared so that an oral dosage unit form contains between 1.0-300 milligrams of active compound.

The tablets, pills, capsules, troches and the like may also contain for example the following ingredients: a binder such as micro-crystalline cellulose, gum tragacanth or gelatin; an excipient such as starch or lactose, a disintegrating agent such as alginic acid, Primogel, cornstarch and the like; a lubricant such as magnesium stearate or Sterotex; a glidant such as colloidal silicon dioxide; and a sweetening agent such as sucrose or saccharin may be added or a flavouring agent such as peppermint, methyl salicylate, or orange flavouring. When the dosage unit form is a capsule, it may contain, in addition to materials of the above type, a liquid carrier such as a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the active compounds, sucrose as a sweetening agent and certain preservatives, dyes, colouring and flavours. Materials used in preparing these various compositions should be pharmaceutically pure and non-toxic in the amounts used.

For the purpose of parenteral therapeutic administration, the active compounds of the invention may be incorporated into a solution or suspension. These preparations should contain at least 0.1% of active compound, but may be varied between 0.5 and about 30% of the weight thereof. The amount of active compound in such compositions is such that a suitable dosage will be obtained. Preferred compositions and preparations according to the present inventions are prepared so that a parenteral dosage unit contains between 0.2 to 100 milligrams of active compound. The solutions or suspensions may also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulphite; chelating agents such as ethylenediaminetetraacetic acid; buffers such as acetates; citrates or phosphates and agents for the adjustment of tonicity such as sodium chloride or dextrose. The parenteral multiple dose vials may be of glass or plastics material.

## Claims

1. A compound having the general formula I

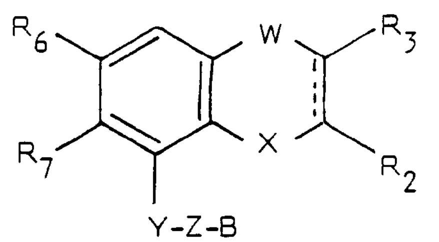

wherein

- ---- represents a single or double bond;
- X represents an oxygen or sulphur atom or an imino, alkylimino, sulphinyl or sulphonyl group;
- W represents a valence bond or a carbonyl, thiocarbonyl, methylene or hydroxymethylene group;
- $R_2$ represents a hydrogen atom or an alkyl, substituted alkyl, alkenyl, substituted alkenyl, alkynyl, substituted alkynyl, carbocyclyl, substituted carbocyclyl, heterocyclyl, substituted heterocyclyl or aroyl group; the substituents for the aforesaid substituted groups being one or more halogen atoms and/or one or more alkyl, cyano, hydroxy, alkoxy, phenyl, phenoxy, trifluoromethyl, nitro, amino, alkylamino, dialkylamino, acylamino, alkylsulphonylamino or benzoyl groups;
- $R_3$ represents a hydrogen atom or an alkyl, hydroxyalkyl, alkoxyalkyl, aralkoxyalkyl, phenyl, hydroxy, alkoxy or aralkoxy group;
- $R_6$ represents a hydrogen or halogen atom or a nitro, amino, alkylamino, dialkylamino, acylamino, alkylsulphonylamino, cyano, hydroxy, alkoxy or alkyl group;
- $R_7$ represents a hydrogen atom or an alkoxy group;
- Y represents one of the following groups, each of which is depicted with its left hand end being the end which attaches to the heterobicyclic ring and its right hand end being the end which attaches to the group Z:

(Y1) -CO-,
(Y2) -COO-,
(Y3) -CONH-,
(Y4) $-CON(CH_3)-$,
(Y5) -CON(OH)-,
(Y6) -CH(OH)-,
(Y7) -CH(OAlkyl)-,
(Y8) -CH=CH-,
(Y9) -CH=CH-COO-,
(Y10) -CH=CH-CONH-,
(Y11) -CH=NO-,
(Y12) $-CH_2-$,
(Y13) $-CH_2COO-$,
(Y14) $-CH_2CONH-$,
(Y15) $-CH_2NH-$,

(Y16) $-CH_2N(CH_3)-$,
(Y17) $-CH_2N(COCH_3)-$,
(Y18) $-CH_2N(CONH_2)-$,
(Y19) $-CH_2NHCO-$,
(Y20) $-CH_2N(CH_3)CO-$,
(Y21) $-CH_2NH-CONH-$,
(Y22) $-CH_2NHSO_2-$,
(Y23) $-CH_2O-$,
(Y24) $-CH_2S-$,
(Y25) $-CH_2SO-$,
(Y26) $-CH_2SO_2-$,
(Y27) $-CH_2SO_2NH-$,
(Y28) $-CH_2SO_2N(CH_3)-$,
(Y29) -NH-,
(Y30) $-N(CH_3)-$,
(Y31) $-N(COCH_3)-$,
(Y32) $-N(CONH_2)-$,
(Y33) -NHCO-,
(Y34) $-N(CH_3)CO-$,
(Y35) -NH-CONH-,
(Y36) $-NHSO_2-$,
(Y37) -O-,
(Y38) -S-,
(Y39) -SO-,
(Y40) $-SO_2-$,
(Y41) $-SO_2NH-$,
(Y42) $-SO_2N(CH_3)-$,
(Y43) -CONHO-,
(Y44) $-CON(COCH_3)-$,
(Y45) -CSNH-,
(Y46) $-CSN(CH_3)-$,
(Y47)

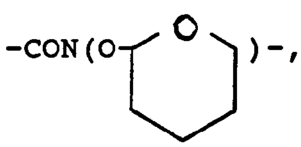

(Y48) -NHCOO-, and
(Y49) -COS-;

Z represents a linear or branched chain alkylene group having from 1 to 6 carbon atoms and optionally having one hydroxy substituent; and

B represents one of the following groups:

(B1)

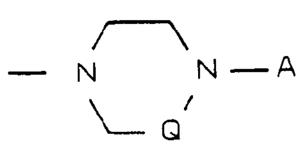

wherein Q represents a methylene or ethylene group and A represents one of the following groups:

(A1) a phenyl group substituted by one or more halogen atoms and/or one or more alkyl, alkoxy or hydroxy groups,
(A2) a 2-pyrimidinyl group, and
(A3) a group of the general formula

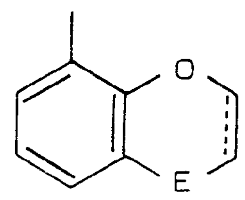

wherein ---- is as above defined and E represents an oxygen atom or a valence bond,

(B2)

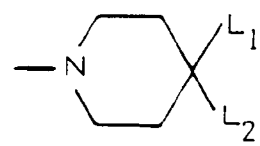

wherein each of $L_1$ and $L_2$ independently represents a hydrogen atom, a phenyl, 4-fluorobenzoyl or 2-oxo-1-benzimidazolinyl group, or a group of the general formula $(CH_2)_n$-O-A wherein n is 0, 1 or 2 and A is as defined in this claim, with the proviso that $L_1$ and $L_2$ do not both represent hydrogen atoms,

(B3)

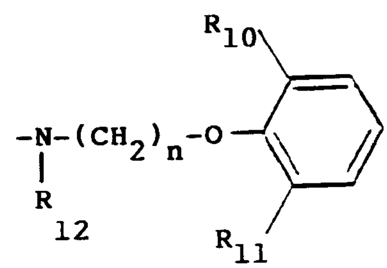

wherein each of $R_{10}$ and $R_{11}$ independently represents a hydrogen atom or an alkoxy or alkylthio group, $R_{12}$ represents a hydrogen atom or an alkyl group, and n is 2 or 3,

(B4)

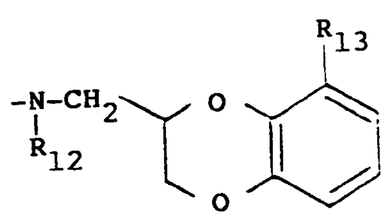

wherein $R_{12}$ is as defined in this claim and $R_{13}$ represents a hydrogen atom or an alkoxy group, and

(B5)

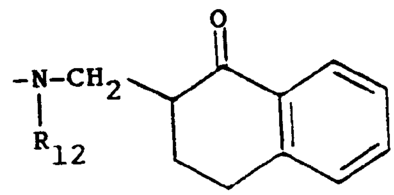

wherein $R_{12}$ is as defined in this claim.

or a prodrug, enantiomer, diastereoisomer, N-oxide or pharmaceutically acceptable salt of such a compound.

2. A compound according to claim 1, wherein

| | |
|---|---|
| ---- | represents a double bond, |
| X | represents an oxygen atom, |
| W | represents a carbonyl group, |
| $R_2$ | represents a phenyl group, |
| $R_3$ | represents a methyl group, |
| $R_6$ | represents a hydrogen atom, and |
| $R_7$ | represents a hydrogen atom. |

3. A compound according to claim 1 or claim 2 wherein Y represents one of the groups Y2, Y3, Y37, Y40 or Y41.

4. A compound according to any preceding claim wherein Z represents a trimethylene or tetramethylene group.

5. A compound according to any preceding claim wherein B represents one of the groups B1 or B3.

6. A compound according to any preceding claim wherein B represents a 4-(2-methoxyphenyl)-1-piperazinyl group.

7. A compound according to any preceding claim wherein Y, Z and B together represent a 3-[4-(2-methoxyphenyl)-1-piperazinyl]-propylcarbamoyl group.

8. Any one of the following compounds:
8-{2-[4-(2-methoxyphenyl)-1-piperazinyl]-1-oxoethyl}-3-methyl-4-oxo-2-phenyl-4H-1-benzopyran,
8-{2-[4-(2-methylphenyl)-1-piperazinyl]-1-oxoethyl}-3-methyl-4-oxo-2-phenyl-4H-1-benzopyran,
8-{2-[4-(2-ethoxyphenyl)-1-piperazinyl]-1-oxoethyl}-3-methyl-4-oxo-2-phenyl-4H-1-benzopyran,
8-{3-[4-(2-methoxyphenyl)-1-piperazinyl]-1-oxopropyl}-3-methyl-4-oxo-2-phenyl-4H-1-benzopyran,
8-{3-[4-(2-methoxyphenyl)-1-piperazinyl]-propoxycarbonyl}-3-methyl-4-oxo-2-phenyl-4H-1-benzopyran,
8-{2-[4-(2-methoxyphenyl)-1-piperazinyl]-ethoxycarbonyl}-3-methyl-4-oxo-2-phenyl-4H-1-benzopyran,
8-{3-[4-(2-chlorophenyl)-1-piperazinyl]-propoxycarbonyl}-3-methyl-4-oxo-2-phenyl-4H-1-benzopyran,
8-[3-(4-phenyl-1-piperazinyl)-propoxycarbonyl]-3-methyl-4-oxo-2-phenyl-4H-1-benzopyran,
8-{3-[4-(2-methoxyphenyl)-1-piperazinyl]-propoxycarbonyl}-3-methyl-4-oxo-2-phenyl-4H-1-benzopyran,
8-{3-[4-(2-methoxyphenyl)-1-piperazinyl]-2-methyl-2-propoxycarbonyl}-3-methyl-4-oxo-2-phenyl-4H-1-benzopyran,
8-{3-[4-(2-methoxyphenyl)-1-piperazinyl]-propylcarbamoyl}-3-methyl-4-oxo-2-phenyl-4H-1-benzopyran,
8-{2-[4-(2-methoxyphenyl)-1-piperazinyl]-ethylcarbamoyl}-3-methyl-4-oxo-2-phenyl-4H-1-benzopyran,
8-{3-[2-(2-methoxyphenoxy)-ethylamino]-propylcarbamoyl}-3-methyl-4-oxo-2-phenyl-4H-1-benzopyran,
8-[3-(4-phenyl-1-piperazinyl)-propylcarbamoyl]-3-methyl-4-oxo-2-phenyl-4H-1-benzopyran,
8-{N-methyl-2-[4-(2-methoxyphenyl)-1-piperazinyl]-ethylcarbamoyl}-3-methyl-4-oxo-2-phenyl-4H-1-benzopyran,
8-{1-hydroxy-2-[4-(2-methoxyphenyl)-1-piperazinyl]-ethyl}-3-methyl-4-oxo-2-phenyl-4H-1-benzopyran,
8-{1-hydroxy-2-[4-(2-methylphenyl)-1-piperazinyl]-ethyl}-3-methyl-4-oxo-2-phenyl-4H-1-benzopyran,
8-{1-hydroxy-2-[4-(2-ethoxyphenyl)-1-piperazinyl]-ethyl}-3-methyl-4-oxo-2-phenyl-4H-1-benzopyran,
8-{1-hydroxy-3-[4-(2-methoxyphenyl)-1-piperazinyl]-propyl}-3-methyl-4-oxo-2-phenyl-4H-1-benzopyran,
8-{1-hydroxy-4-[4-(2-methoxyphenyl)-1-piperazinyl]-butyl}-3-methyl-4-oxo-2-phenyl-4H-1-benzopyran,

8-{1-ethoxy-2-[4-(2-methoxyphenyl)-1-piperazinyl]-ethyl}-3-methyl-4-oxo-2-phenyl-4H-1-benzopyran,
8-{N-methyl-2-[4-(2-methoxyphenyl)-1-piperazinyl]-ethylaminomethyl}-3-methyl-4-oxo-2-phenyl-4H-1-benzopyran,
8-{N-acetyl-2-[4-(2-methoxyphenyl)-1-piperazinyl]-ethylaminomethyl}-3-methyl-4-oxo-2-phenyl-4H-1-benzopyran,
8-[4-(2-methoxyphenyl)-1-piperazinylacetamidomethyl]-3-methyl-4-oxo-2-phenyl-4H-1-benzopyran,
8-{N-methyl-N-[4-(2-methoxyphenyl)-1-piperazinyl]-acetamidomethyl}-3-methyl-4-oxo-2-phenyl-4H-1-benzopyran,
8-{2-[4-(2-methoxyphenyl)-1-piperazinyl]-ethoxymethyl}-3-methyl-4-oxo-2-phenyl-4H-1-benzopyran,
8-{2-[2-(2-ethoxyphenoxy)-ethylamino]-ethoxymethyl}-3-methyl-4-oxo-2-phenyl-4H-1-benzopyran, hydrochloride
8-{2-[4-(2-methoxyphenyl)-1-piperazinyl]-ethylthiomethyl}-3-methyl-4-oxo-2-phenyl-4H-1-benzopyran,
8-{2-[4-(2-methoxyphenyl)-1-piperazinyl]-ethylsulphinylmethyl}-3-methyl-4-oxo-2-phenyl-4H-1-benzopyran,
8-{2-[4-(2-methoxyphenyl)-1-piperazinyl]-ethylsulphonylmethyl}-3-methyl-4-oxo-2-phenyl-4H-1-benzopyran,
8-{2-[4-(2-methoxyphenyl)-1-piperazinyl]-ethylamino}-3-methyl-4-oxo-2-phenyl-4H-1-benzopyran,
8-{3-[4-(2-methoxyphenyl)-1-piperazinyl]-propylamino}-3-methyl-4-oxo-2-phenyl-4H-1-benzopyran,
8-{4-[4-(2-methoxyphenyl)-1-piperazinyl]-butylamino}-3-methyl-4-oxo-2-phenyl-4H-1-benzopyran,
8-{N-methyl-3-[4-(2-methoxyphenyl)-1-piperazinyl]-propylamino}-3-methyl-4-oxo-2-phenyl-4H-1-benzopyran,
8-{N-acetyl-3-[4-(2-methoxyphenyl)-1-piperazinyl]-propylamino}-3-methyl-4-oxo-2-phenyl-4H-1-benzopyran,
8-{3-[4-(2-methoxyphenyl)-1-piperazinyl]-propionamido}-3-methyl-4-oxo-2-phenyl-4H-1-benzopyran,
8-{2-[4-(2-methoxyphenyl)-1-piperazinyl]-ethylureido}-3-methyl-4-oxo-2-phenyl-4H-1-benzopyran,
8-{2-[4-(2-methoxyphenyl)-1-piperazinyl]-ethoxy}-3-methyl-4-oxo-2-phenyl-4H-1-benzopyran,
8-{3-[4-(2-methoxyphenyl)-1-piperazinyl]-propoxy}-3-methyl-4-oxo-2-phenyl-4H-1-benzopyran,
8-{4-[4-(2-methoxyphenyl)-1-piperazinyl]-butoxy}-3-methyl-4-oxo-2-phenyl-4H-1-benzopyran,
8-{5-[4-(2-methoxyphenyl)-1-piperazinyl]-pentyloxy}-3-methyl-4-oxo-2-phenyl-4H-1-benzopyran,
8-{3-[4-(2-methoxyphenyl)-1-oxo-1-piperazinyl]-propoxy}-3-methyl-4-oxo-2-phenyl-4H-1-benzopyran,
8-{2-[2-(2,6-dimethoxyphenoxy)-ethylamino]-ethoxy}-3-methyl-4-oxo-2-phenyl-4H-1-benzopyran,
8-{2-hydroxy-3-[4-(2-methoxyphenyl)-1-piperazinyl]-propoxy}-3-methyl-4-oxo-2-phenyl-4H-1-benzopyran,
8-{3-[4-(2-methoxyphenyl)-1-piperazinyl]-propylthio}-3-methyl-4-oxo-2-phenyl-4H-1-benzopyran,
8-{3-[4-(2-methoxyphenyl)-1-piperazinyl]-propylsulphonyl}-3-methyl-4-oxo-2-phenyl-4H-1-benzopyran,
8-{2-[4-(2-methoxyphenyl)-1-piperazinyl]-ethylsulphamoyl}-3-methyl-4-oxo-2-phenyl-4H-1-benzopyran,
8-{N-methyl-2-[4-(2-methoxyphenyl)-1-piperazinyl]-ethylsulphamoyl}-3-methyl-4-oxo-2-phenyl-4H-1-benzopyran,
8-{N-carbamoyl-3-[4-(2-methoxyphenyl)-1-piperazinyl]-propylamino}-3-methyl-4-oxo-2-phenyl-4H-1-benzopyran,
8-{4-[4-(2-methoxyphenyl)-1-piperazinyl]-1-oxobutyl}-3-methyl-4-oxo-2-phenyl-4H-1-benzopyran,
8-{3-[2-(1,4-benzodioxanyl)methylamino]propylcarbamoyl}-3-methyl-4-oxo-2-phenyl-4H-1-benzopyran,
8-{4-[4-(2-methoxyphenyl)-1-piperazinyl]-butyl}-3-methyl-4-oxo-2-phenyl-4H-1-benzopyran,
8-[3-(4-phenyl-1-piperidinyl)-propylcarbamoyl]-3-methyl-4-oxo-2-phenyl-4H-1-benzopyran,
8-[3-(4,4-diphenyl-1-piperidinyl)-propylcarbamoyl]-3-methyl-4-oxo-2-phenyl-4H-1-benzopyran,
8-{3-[4-(4-fluorobenzoyl)-1-piperidinyl]-propyl-carbamoyl}-3-methyl-4-oxo-2-phenyl-4H-1-benzopyran,
8-{3-[4-(2-oxo-1-benzimidazolinyl)-1-piperidinyl]-propyl-carbamoyl}-3-methyl-4-oxo-2-phenyl-4H-1-benzopyran,
8-{3-[4-(2-pyrimidinyl)-1-piperazinyl]-propylcarbamoyl}-3-methyl-4-oxo-2-phenyl-4H-1-benzopyran,
8-{3-[4-(2-hydroxyphenyl)-1-piperazinyl]-propylcarbamoyl}-3-methyl-4-oxo-2-phenyl-4H-1-benzopyran,
8-{4-[4-(2-methoxyphenyl)-1-piperazinyl]-butylcarbamoyl}-3-methyl-4-oxo-2-phenyl-4H-1-benzopyran,
8-{3-[4-(2-methoxyphenyl)-1-piperazinyl]-propylsulphamoyl}-3-methyl-4-oxo-2-phenyl-4H-1-benzopyran,
8-{3-[N-methyl-2-(2-methoxyphenoxy)-ethylamino]-propylcarbamoyl}-3-methyl-4-oxo-2-phenyl-4H-1-benzopyran,
8-{N-methyl-3-[4-(2-methoxyphenyl)-1-piperazinyl]-propionamido}-3-methyl-4-oxo-2-phenyl-4H-1-benzopyran,
8-{3-[4-(2-methoxyphenyl)-1-piperazinyl]-propylcarbamoyl}-3-phenyl-4-oxo-4H-1-benzopyran,
8-{3-[(3,4-dihydro-1-oxo-2H-naphthyl)-methylamino]-propylcarbamoyl}-3-methyl-4-oxo-2-phenyl-4H-1-benzopyran,

8-{2-[4-(2-methoxyphenyl)-1-piperazinyl]-ethoxycarbonylmethyl}-3-methyl-4-oxo-2-phenyl-4H-1-benzopyran,
8-{4-[4-(2-methoxyphenyl)-1-piperazinyl]-butylsulphamoyl}-3-methyl-4-oxo-2-phenyl-4H-1-benzopyran,
8-{N-(2-tetrahydropyranyloxy)-3-[4-(2-methoxyphenyl)-1-piperazinyl]-propylcarbamoyl}-3-methyl-4-oxo-2-phenyl-4H-1-benzopyran,
8-{4-[4-(2-methoxyphenyl)-1-piperazinyl]-butyramido}-3-methyl-4-oxo-2-phenyl-4H-1-benzopyran,
E-8-{2-[4-(2-methoxyphenyl)-1-piperazinyl]-ethoxyimino-methyl}-3-methyl-4-oxo-2-phenyl-4H-1-benzopyran,
8-{N-hydroxy-3-[4-(2-methoxyphenyl)-1-piperazinyl]-propylcarbamoyl}-3-methyl-4-oxo-2-phenyl-4H-1-benzopyran,
E-8-<2-{2-[4-(2-methoxyphenyl)-1-piperazinyl]-ethylcarbamoyl}-ethenyl>-3-methyl-4-oxo-2-phenyl-4H-1-benzopyran,
8-{4-[4-(2-methoxyphenyl)-1-piperazinyl]-butylsulphinyl}-3-methyl-4-oxo-2-phenyl-4H-1-benzopyran,
8-{3-[3-(2-methoxyphenoxy)-propylamino]-propylcarbamoyl}-3-methyl-4-oxo-2-phenyl-4H-1-benzopyran,
8-{3-[2-(2-methylthiophenoxy)-ethylamino]-propylcarbamoyl}-3-methyl-4-oxo-2-phenyl-4H-1-benzopyran,
8-{3-[2-(2,6-dimethoxyphenoxy)-ethylamino]-propylcarbamoyl}-3-methyl-4-oxo-2-phenyl-4H-1-benzopyran,
8-{3-[4-(5-chloro-2-methoxyphenyl)-1-piperazinyl]-propylcarbamoyl}-3-methyl-4-oxo-2-phenyl-4H-1-benzopyran,
(E)-8-{4-[4-(2-methoxyphenyl)-1-piperazinyl]-1-butenyl}-3-methyl-4-oxo-2-phenyl-4H-1-benzopyran,
(E)-8-<2-{2-[4-(2-methoxyphenyl)-1-piperazinyl]-ethoxycarbonyl}-ethenyl>-3-methyl-4-oxo-2-phenyl-4H-1-benzopyran
8-{2-[4-(2-methoxyphenyl)-1-piperazinyl]-ethylcarbamoylmethyl}-4-oxo-2-phenyl-4H-1-benzopyran,
8-{N-acetyl-3-[4-(2-methoxyphenyl)-1-piperazinyl]-propylcarbamoyl}-3-methyl-4-oxo-2-phenyl-4H-1-benzopyran,
8-{2-[4-(2-methoxyphenyl)-1-piperazinyl]-ethylsulphonylamino}-3-methyl-4-oxo-2-phenyl-4H-1-benzopyran,
8-{3-[4-(2-methoxyphenyl)-1-piperazinyl]-propylthiocarbamoyl}-3-methyl-4-oxo-2-phenyl-4H-1-benzopyran,
8-{4-[4-(2-methoxyphenyl)-1-piperazinyl]-butylsulphonyl}-3-methyl-4-oxo-2-phenyl-4H-1-benzopyran,
8-{3-[4-(2-methoxyphenyl)-1-piperazinyl]-propylcarbamoyl}-3-hydroxypethyl-4-oxo-2-phenyl-4H-1-benzopyran,
8-{3-[4-(2-methoxyphenyl)-1-piperazinyl]-propylcarbamoyl}-4-oxo-2-phenyl-4H-1-benzopyran,
8-{3-[4-(2-methoxyphenyl)-1-piperazinyl]-propylcarbamoyl}-2,3-dihydro-4-oxo-4H-1-benzopyran,
8-{3-[4-(2-methoxyphenyl)-1-piperazinyl]-propylcarbamoyl}-4-oxo-4H-1-benzopyran,
8-{3-[4-(2-methoxyphenyl)-1-piperazinyl]-propylcarbamoyl}-6-bromo-3-methyl-4-oxo-2-phenyl-4H-1-benzopyran,
8-{3-[4-(2-methoxyphenyl)-1-piperazinyl]-propylcarbamoyl}-6-methoxy-3-methyl-4-oxo-2-phenyl-4H-1-benzopyran,
8-{3-[4-(2-methoxyphenyl)-1-piperazinyl]-propylcarbamoyl}-6-hydroxy-3-methyl-4-oxo-2-phenyl-4H-1-benzopyran,
8-{3-[4-(2-methoxyphenyl)-1-piperazinyl]-propylcarbamoyl}-3,6-dimethyl-4-oxo-2-phenyl-4H-1-benzopyran,
8-{3-[4-(2-methoxyphenyl)-1-piperazinyl]-propylcarbamoyl}-3-methyl-6-nitro-4-oxo-2-phenyl-4H-1-benzopyran,
8-{3-[4-(2-methoxyphenyl)-1-piperazinyl]-propylcarbamoyl}-6-amino-3-methyl-4-oxo-2-phenyl-4H-1-benzopyran,
8-{3-[4-(2-methoxyphenyl)-1-piperazinyl]-propylcarbamoyl}-6-acetamido-3-methyl-4-oxo-2-phenyl-4H-1-benzopyran,
8-{3-[4-(2-methoxyphenyl)-1-piperazinyl]-propylcarbamoyl}-6-ethylamino-3-methyl-4-oxo-2-phenyl-4H-1-benzopyran,
8-{3-[4-(2-methoxyphenyl)-1-piperazinyl]-propylcarbamoyl}-6-dimethylamino-3-methyl-4-oxo-2-phenyl-4H-1-benzopyran,
8-{3-[4-(2-methoxyphenyl)-1-piperazinyl]-propylcarbamoyl}-7-methoxy-3-methyl-4-oxo-2-phenyl-4H-1-benzopyran,
8-{3-[4-(2-methoxyphenyl)-1-piperazinyl]-propylcarbamoyl}-3-methyl-4-oxo-2-(4-trifluoromethylphenyl)-4H-1-benzopyran,
8-{3-[4-(2-methoxyphenyl)-1-piperazinyl]-propylcarbamoyl}-2-(4-benzoylphenyl)-3-methyl-4-oxo-4H-1-benzopyran,

8-{3-[4-(2-methoxyphenyl)-1-piperazinyl]-propylcarbamoyl}-3-methyl-4-oxo-2-(4-phenoxyphenyl)-4H-1-benzopyran,
8-{3-[4-(2-methoxyphenyl)-1-piperazinyl]-propylcarbamoyl}-2,3-dimethyl-4-oxo-4H-1-benzopyran,
8-{3-[4-(2-methoxyphenyl)-1-piperazinyl]-propylcarbamoyl}-2-(t-butyl)-3-methyl-4-oxo-4H-1-benzopyran,
8-{3-[4-(2-methoxyphenyl)-1-piperazinyl]-propylcarbamoyl}-2-cyclohexyl-3-methyl-4-oxo-4H-1-benzopyran,
8-{3-[4-(2-methoxyphenyl)-1-piperazinyl]-propylcarbamoyl}-2-(2-furyl)-3-methyl-4-oxo-4H-1-benzopyran,
8-{3-[4-(2-methoxyphenyl)-1-piperazinyl]-propylcarbamoyl}-3-methyl-4-oxo-2-thienyl-4H-1-benzopyran,
8-{3-[4-(2-methoxyphenyl)-1-piperazinyl]-propylcarbamoyl}-4-oxo-2-phenyl-4H-1-benzothiopyran,
(E)-8-{3-[4-(2-methoxyphenyl)-1-piperazinyl]-propylcarbamoyl}-3-methyl-4-oxo-2-(2-styryl)-4H-1-benzopyran,
8-{3-[4-(2-methoxyphenyl)-1-piperazinyl]-propylcarbamoyl}-3-methyl-2-(4-methylphenyl)-4-oxo-4H-1-benzopyran,
8-{3-[4-(2-methoxyphenyl)-1-piperazinyl]-propylcarbamoyl}-2-(4-methoxyphenyl)-3-methyl-4-oxo-4H-1-benzopyran,
8-{3-[4-(2-methoxyphenyl)-1-piperazinyl]-propylcarbamoyl}-2-(4-fluorophenyl)-3-methyl-4-oxo-4H-1-benzopyran,
8-{3-[4-(2-methoxyphenyl)-1-piperazinyl]-propylcarbamoyl}-6-methanesulphonylamino-3-methyl-4-oxo-2-phenyl-4H-1-benzopyran,
8-{3-[4-(2-methoxyphenyl)-1-piperazinyl]-propylcarbamoyl}-3-methyl-2-(4-nitrophenyl)-4-oxo-4H-1-benzopyran,
8-{3-[4-(2-methoxyphenyl)-1-piperazinyl]-propylcarbamoyl}-6-diethoxyphosphonyloxy-3-methyl-4-oxo-2-phenyl-4H-1-benzopyran,
8-{3-[4-(2-methoxyphenyl)-1-piperazinyl]-propylcarbamoyl}-3-methyl-4-oxo-2-trifluoromethyl-4H-1-benzopyran,
8-{N-methyl-3-[4-(2-methoxyphenyl)-1-piperazinyl]-propylcarbamoyl}-3-methyl-4-oxo-2-phenyl-4H-1-benzopyran,
7-{3-[4-(2-methoxyphenyl)-1-piperazinyl]-propylcarbamoyl}-2-benzoyl-3-ethyl-benzo[b]furan,
8-{3-[4-(2-methoxyphenyl)-1-piperazinyl]-propylcarbamoyl}-2-(4-biphenylyl)-3-methyl-4-oxo-4H-1-benzopyran,
8-{3-[4-(2-methoxyphenyl)-1-piperazinyl]-propylcarbamoyl}-2-(4-biphenylyl)-3-methyl-4-oxo-4H-1-benzopyran,
8-{3-[4-(2-methoxyphenyl)-1-piperazinyl]-propylcarbamoyl}-3-methyl-4-oxo-2-(3-pyridyl)-4H-1-benzopyran,
8-{3-[4-(2-acetoxyphenyl)-1-piperazinyl]-propylcarbamoyl}-3-methyl-4-oxo-2-phenyl-4H-1-benzopyran,
8-{3-[4-(2-methylaminocarbonyloxyphenyl)-1-piperazinyl]-propylcarbamoyl}-3-methyl-4-oxo-2-phenyl-4H-1-benzopyran,
8-{3-[4-(2-methoxyphenyl)-1-piperazinyl]-propylcarbamoyl}-6-acetoxy-3-methyl-4-oxo-2-phenyl-4H-1-benzopyran,
(R,S)-8-{3-[4-(2-methoxyphenyl)-1-piperazinyl]-propylcarbamoyl}-2,3-dihydro-4-hydroxy-4H-1-benzopyran,
8-{3-[4-(2-methoxyphenyl)-1-piperazinyl]-propylcarbamoyl}-2-(4-aminophenyl)-3-methyl-4-oxo-4H-1-benzopyran,
8-{3-[4-(2-methoxyphenyl)-1-piperazinyl]-propylcarbamoyl}-2-(4-acetylaminophenyl)-3-methyl-4H-1-benzopyran,
8-{3-[4-(2-methoxyphenyl)-1-piperazinyl]-propylcarbamoyl}-2-(4-hydroxyphenyl)-3-methyl-4H-1-benzopyran,
8-{3-[4-(2-methoxyphenyl)-1-piperazinyl]-propylcarbamoyl}-2-phenyl-4,$N^1$,$N^4$-trioxo-4H-1-benzothiopyran,
7-{3-[4-(2-methoxyphenyl)-1-piperazinyl]-propylcarbamoyl}-2-phenyl-benzo[b]furan, and
8-{N-methyl-4-[4-(2-methoxyphenyl)-1-piperazinyl]-butylsulfamoyl}-3-methyl-4-oxo-2-phenyl-4H-1-benzopyran;
or a prodrug, enantiomer, diastereoisomer, N-oxide or pharmaceutically acceptable salt of such a compound.

9. A pharmaceutical composition comprising a compound according to any preceding claim, or a prodrug, enantiomer, diastereoisomer, N-oxide or pharmaceutically acceptable salt of such a compound, in admixture with a pharmaceutically acceptable diluent or carrier.

European Patent Office

# PARTIAL EUROPEAN SEARCH REPORT

which under Rule 45 of the European Patent Convention shall be considered, for the purposes of subsequent proceedings, as the European search report

Application Number
EP 93 30 1264 .3.
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| X | WO-A-9 118 597 (R.H. ERICKSON)<br>* page 12, line 16 - page 14, line 4; claims 1,11 *<br>--- | 1,2,9 | C07D311/30<br>C07D407/12<br>C07D405/12<br>C07D405/14<br>C07D311/22<br>C07D407/04<br>C07D409/04<br>C07D335/06<br>C07D405/04<br>C07D307/79<br>C07F9/655<br>A61K31/35 |
| A | US-A-2 921 070 (P. DA RE)<br>* column 1, line 21 - line 40 *<br>--- | 1,2,9 | |
| A | PATENT ABSTRACTS OF JAPAN<br>vol. 11, no. 85 (C-410)(2532) 14 March 1987<br>& JP-A-61 238 783 ( HOKURIKU SEIYAKU ) 24 October 1986<br>* abstract *<br>--- | 1,2,9 | |
| A | US-A-3 810 896 (E.-C. WITTE)<br>* column 1, line 41 - line 62 *<br>--- | 1,2,9 | |
| X | EP-A-0 190 015 (WARNER-LAMBERT)<br>* column 1; claims 1,10,11 *<br>--- | 1,3-5,9 | |
| X | EP-A-0 156 331 (KAKEN PHARMACEUTICAL)<br>* page 1 - page 2; examples 16,22,23 *<br>---<br>-/-- | 1,3-5,9 | TECHNICAL FIELDS SEARCHED (Int. Cl. 5)<br>C07D |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 08 JUNE 1993 | RUSSELL F. ENGLISH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)

European Patent Office

# PARTIAL EUROPEAN SEARCH REPORT

Application Number
EP 93 30 1264 ·3
Page 2

| DOCUMENTS CONSIDERED TO BE RELEVANT | | | CLASSIFICATION OF THE APPLICATION (Int. Cl. 5) |
|---|---|---|---|
| Category | Citation of document with indication, where appropriate, of relevant passages | Relevant to claim | |
| A | EP-A-0 306 226 (TANABE SEIYAKU)<br>* page 2 - page 3 *<br>--- | 1,9 | |
| A | EP-A-0 270 342 (TANABE SEIYAKU)<br>* page 2 *<br>----- | 1,9 | |
| | | | TECHNICAL FIELDS SEARCHED (Int. Cl. 5) |

EPO FORM 1503 03.82 (P04E10)

EP 93 30 1264 -C-

INCOMPLETE SEARCH

Claims searched completely : 8
Claims searched incompletely : 1-7,9

Reason : The term "prodrug" is not precise and it is not possible to determine which compounds are meant by it. The search has been carried out on the basis of the well-defined parts of the claims and the examples (including examples 114 and 120-122) (Art. 84 EPC).

Claim 2 : In claim 1, the variable $R_2$ does not have "phenyl group" as a possible value. However, since in the majority of the examples and in claim 2, $R_2$ is phenyl group, this value has been included in the search.